# EUROPEAN PATENT APPLICATION

(11) **EP 4 696 686 A1**
(43) Date of publication of application: **18.02.2026**
(21) Application number: 24194789.4
(22) Date of filing: 15.08.2024
(51) Int. Cl.: C07D 403/14, C07F 5/00, C07B 59/00, A61P 35/00, A61K 51/04

(54) **FAPI THERANOSTIC COMPOUNDS ASSEMBLED WITH TETRAZINE LIGATION**

(71) Applicant: Tetrakit Technologies ApS, 2200 Copenhagen N (DK)
(72) Inventor: HERTH, Matthias Manfred, 2200 Copenhagen N (DK); POULIE, Christian Bernard Matthijs, 2200 Copenhagen N (DK); MARTIN, Marcel, 2200 Copenhagen N (DK); BATTISTI, Umberto Maria, 2200 Copenhagen (DK); VÁZQUEZ, Rocío García, 2200 Copenhagen N (DK)
(74) Representative: Budde Schou A/S

(57) **Abstract**

The present invention relates to fibroblast activation protein (FAP) - based ligands assembled through tetrazine ligation and their use in radiotherapy and imaging. The FAP inhibitor (FAPi) ligand compounds provided herein comprises a FAP targeting moiety, a linker, an IsoF-TCO and a radiolabeled tetrazine. The FAPi targeting compounds are possessing advantageous pharmacokinetic properties and enable quick and efficient modifications of the radiopharmaceuticals allowing a fast renal excretion and reduced liver accumulation.

## Description

### Field of the Invention

The present invention relates to fibroblast activation protein (FAP) - based ligands assembled through tetrazine ligation and their use in radiotherapy and imaging.

### Background

Fibroblast activation protein alpha (FAP) is a S9b family serine protease and an integral type-II trans-membrane glycoprotein. FAP is mainly expressed on activated fibroblasts such as cancer-associated fibroblasts (CAFs), which are a major part of the tumor microenvironment (TME). The TME plays an essential role in tumor genesis, tumor growth, metastasis and angiogenesis and can account for up to 90% of the total tumor mass. Each epithelial tumor of a certain size (ca. ≥ 1 mm) is surrounded by an extensive TME. More than 90% of these epithelial tumors strongly express FAP including breast, lung, colorectal, prostate, ovarian among other cancers as well as sarcomas. In contrast, FAP is not expressed by resting fibroblasts in adult, healthy tissue. The selective expression makes FAP an interesting pan-tumor target in medicinal use, as FAP-based ligands can be used in the diagnosis and/or treatment of a wide range of cancers, including breast, lung, colorectal, prostate, ovarian, pancreatic, and hepatocellular cancers, as well as sarcomas and non-small cell lung cancer (NSCLC).

Beyond cancer, FAP expression is significant in several non-cancerous conditions such as fibrosis, arthritis, atherosclerosis, and inflammatory diseases like spondyloarthritis. This broad range of expression underscores FAP's potential as a target for diagnostic and therapeutic applications in various pathological conditions.

In 2018, the high-affinity and selective FAP inhibitor (FAPi) UAMC-1110 was used as a targeting vector in the first small-molecule radiotracers successfully visualizing various epithelial tumors via positron emission tomography (PET). Within a few years, molecular imaging with these ⁶⁸Ga-labeled radiotracers such as FAPI-04, FAPI-46, DOTA.SA.FAPi and OncoFAP showed highly valuable diagnostic results outperforming or at least being on par with ¹⁸F-FDG. Other diagnostic tracers using different radionuclides such as ¹⁸F-FAPI-42/74 and ^{99m}Tc-FAPI-34 among others have also been developed and have shown diagnostic value.

For diagnostic purposes, the amount of radioactivity administered can vary based on the specific radiotracer and imaging modality used. Typically, for PET imaging with radiolabeled FAP inhibitors such as 68Ga-FAPI tracers, a dose in the range of 100-200 MBq is commonly used to achieve high-quality images while minimizing radiation exposure to patients. For SPECT imaging using 99mTc-FAPI tracers, the administered dose might range from 300 to 740 MBq, depending on the specific clinical protocol and the patient's condition. The dosage can vary significantly depending on factors such as the type of radionuclide used, the sensitivity of the imaging equipment, and the specific clinical scenario. For instance, whole-body PET scans, which are highly sensitive, may require lower doses of radiotracer to achieve effective imaging results.

However, translation into effective therapeutic radiopharmaceuticals for radioligand therapy (RLT) has proven to be challenging. The main problem is the short tumor residence time of the above-mentioned FAPi monomers (one targeting vector) resulting in a low radiation tumor-dose when labeled with therapeutic radiometals such as ¹⁷⁷Lu, ⁹⁰Y or ²²⁵Ac. Substantial progress regarding the tumor-dose could be achieved with FAPi dimers ¹⁷⁷Lu-DOTAGA.(SA.FAPi)₂ and ¹⁷⁷Lu-DOTAGA.Glu.(FAPi)₂ containing two FAPi targeting vectors. They can be administered safely, and the therapeutic efficacy has been investigated mostly in thyroid cancer, with a response rate of >50% in 15 heavily pre-treated RR-DTC patients and an overall survival after a 3-year follow-up of >50%. Other groups adapted the dimer approach and found prolonged tumor retention and improved therapeutic efficacy. Simultaneously, FAP-targeting peptides FAP-2286 and 3BP-3940 labeled with various therapeutic radiometals were developed and performed promisingly in preclinical xenograft models, but the translation into clinical studies could not confirm the therapeutic efficacy (Zboralski et al., Eur. J. Nucl. Med. Mol. Imaging, 49, 3651-3667, 2022; Baum et al., J. Nucl. Med., 63, 415-423, 2022; Baum et al., J. Nucl. Med., 63, 2269, 2022).

Radiohalogens have also been tested with FAP-directed therapy. For instance, radioiodine, especially ¹³¹I, since it is available in large quantities and relatively cheap as it formed as a fission product in nuclear reactors would be beneficial to apply since the potentially high demand for FAP therapeutics can be met with ¹³¹I as the therapeutic radionuclide. The only published radioiodine-FAP compound ¹³¹I-FAPI-04 thus far showed suboptimal properties in preclinical models including short tumor retention time and high doses to liver and GI tract due to hepatobiliary excretion (Ma et al., Mol. Pharm., 18, 4179-4187, 2021).

Astatine-211 (²¹¹At) could also be relevant in FAP-directed therapy as a valid alternative to ²²⁵Ac based on its short half-life of 7.2 hours and its emission that does produce any long-lived alpha-emitting daughters. Both of these properties can significantly reduce cytotoxicity to the patients compared to ²²⁵Ac-based FAPi derivatives (Albertsson et al., Front Med (Lausanne), 9, 1076210, 2022).

Radiometals, such as Gallium-68, Copper-64, and Zirconium-89, are typically introduced into molecules through chelation. This process involves using chelator groups that form coordinate bonds with the metal ions, ensuring stable attachment. The chelation reaction usually involves mixing the radiometal ions with a precursor molecule containing the chelator group. This mixture is then heated to facilitate the chelation process. Common chelators include DOTA (1,4,7,10-tetraazacyclododecane-1,4,7,10-tetraacetic acid) and NOTA (1,4,7-triazacyclononane-1,4,7-triacetic acid), which form strong, stable complexes with radiometals. This method ensures that the radiometal remains securely bound to the molecule, maintaining the integrity and functionality of the radiopharmaceutical. For example, Gallium-68 is often incorporated into molecules using the DOTA chelator, resulting in compounds like Ga-68 DOTATATE, which targets somatostatin receptors in neuroendocrine tumors.

Radiohalogens, such as lodine-123, Fluorine-18, and Astatine-211, are incorporated into molecules through covalent bonding, typically via nucleophilic substitution reactions. This process involves the replacement of a leaving group in the molecule with the radiohalogen. For instance, in the case of iodine, an electrophilic substitution reaction can be employed, where radioactive iodide is oxidized to a positively charged iodine species that then replaces a leaving group, usually a stannyl group, in an aromatic substitution reaction. This reaction is conducted at room temperature and often yields high efficiency. Another method for introducing iodine into molecules is isotopic exchange, where a non-radioactive iodine atom in the molecule is replaced by a radioactive one. This process usually requires elevated temperatures and acidic conditions, often catalyzed by copper. Astatine-211, being a halogen, can also be attached to aryl rings forming astatoaryl moieties. However, due to its unique chemistry, astatine cannot be stably coupled to tyrosine residues of proteins like iodine can. Instead, it forms bonds with sulfhydryl groups of cysteine, necessitating the synthesis of dedicated precursors with suitable leaving groups like trialkylstannyl. The labeling reaction for astatine often uses oxidation agents such as chloramine-T or N-chlorosuccinimide, which can degrade biomolecules used as targeting vectors.

Overall, the reactions to incorporate chelators usually needs a purification process and heating, which can degrade temperature-sensitive vectors. In contrast, radiohalogens form covalent bonds with targeting vectors, often under harsh and lengthy conditions incompatible with peptides and other sensitive biomolecules. To overcome these challenges, synthon-based methods have been developed, allowing radiolabeling under mild conditions without exposing the vector to harsh environments. Tetrazine ligation exemplifies this approach and the dual-functional tetrazine compounds facilitate these radiolabeling processes, providing a versatile platform for creating advanced radiopharmaceuticals.

However, common synthesis and radiolabeling procedures are based on standard methodologies that are not always easy to implement in radiopharmacies, hospitals and clinics. Click chemistry has shown great potential in the synthesis of radiopharmaceuticals for imaging and therapy. In particular, the inverse electron demand Diels- Alder cycloaddition reaction (IEDDA) between tetrazines and dienophiles has shown the fastest reaction kinetics in click chemistry, and it has been utilized in radiosynthesis of various labeled radiopharmaceuticals. The main limitation of this reaction was the formation of multiple isomers after the click. This issue has been recently solved by the synthesis of a new type of isomer-free *trans-*cyclooctenes (IsoF-TCOs), that combined with an oxidation step result in the formation of a single isomeric product (PCT/EP2023/055930).

The FAP targeting moiety of the present invention is based on a dimeric FAPi that has already been successfully applied for therapy in the clinic. High affinity, selectivity as well as good and prolonged tumor accumulation are reported for this structure. As shown herein, we have found that by synthesizing a compound targeting FAPi, wherein the compound comprises a linker, an IsoF-TCO and a radiolabeled tetrazine, FAPi targeting compounds possessing selected pharmacokinetic properties are obtained. The present compounds can be labeled either with a radiohalogen or a radiometal. The modularity of this approach enables quick and efficient modifications of the radiopharmaceuticals allowing the fast optimization of some properties such as renal excretion and liver accumulation.

Moreover, the radiolabeled compounds provided herein showed a surprisingly higher tumor accumulation compared to [¹¹¹In]In-DOTAGA.Glu.(FAPi)₂ (Martin et al., Cancers, 15(6), 1889, 2023).

### Summary of the invention:

In a first aspect, the present invention relates to a FAP inhibitor ligand of formula (I): 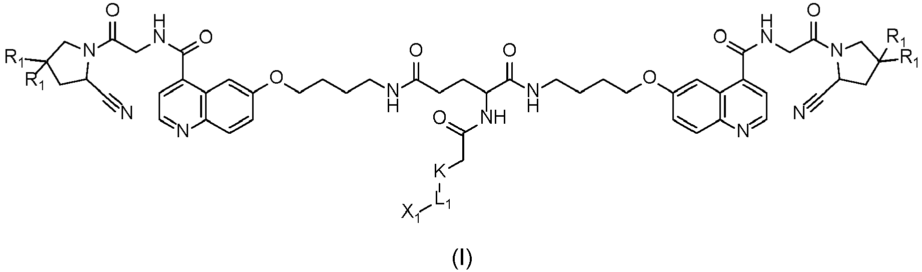 wherein:
R₁ is H or F
K is 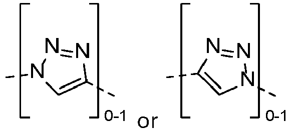
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-,-CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-,-COCH(CH₂CH₂COOH)NH-, -COCH(CH₂C₆H₅)NH-, -COCH(CH₂C₃H₃N₂)NH-,-COCH(CH(CH₃)₂)NH-, -COCH((CHCH₃)CH₂CH₃)NH-, -COCH((CH₂)₄NH₂)NH-,-COCH(CH₂CH(CH₃)₂)NH-, -COCH(CH₂CH₂SCH₃)NH-, -CO(CHCH₂CH₂N)-,-COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-,-COCH(CH(OH)CH₃)NH-, -COCH(CH₂SeH)NH-, -COCH(CH₂C₈H₆N)NH-,-COCH(CH₂C₆H₄OH)NH-, -COCH(CH₂CH₂SeCH₃)NH-;
X₁ is a pyridazine selected from the group consisting of: 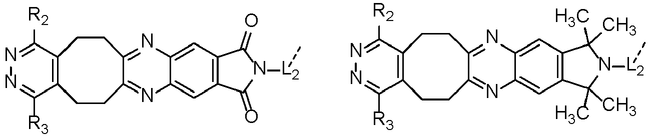 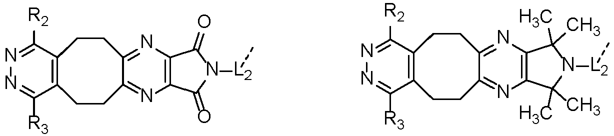 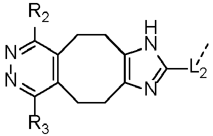
   wherein L₂ is -CO(CH₂)ₙ- or -CO(CH₂CH₂O)ₙ(CH₂)ₘ-,
   wherein n and m are integers independently selected from 1-10, and
R₂ is selected from -H, -Me, and R₃,
R₃ is 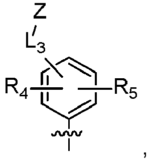 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, -NHCH₂CH₂Y₁,-OCH₂C₆H₄Y₁, -OCH₂CH₂C₆H₄Y₁, -NHCOCH₂CH₂Y₁, -CONHCH₂CH₂Y₁, -NHSO₂CH₂CH₂Y₁,-SO₂NHCH₂CH₂Y₁,
   wherein Y₁ is selected from the group constisting of:
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At, ²¹¹At,
   and wherein L₃ is selected from: -H, -(CH₂)ₙNH-, -O(CH₂)ₙNH-, -S(CH₂)ₙNH-,-SO₂NH(CH₂)ₙNH-, -NHSO₂(CH₂)ₙNH-, -CONH(CH₂)ₙNH-, -NHCO(CH₂)ₙNH-,-(OCH₂CH₂)ₙ(CH₂)ₘNH-, -(CH₂)ₙ(OCH₂CH₂)ₘNH-, -O(CH₂)ₙ(OCH₂CH₂)ₘNH-,-S(CH₂)ₙ(OCH₂CH₂)ₘNH-, -SO₂NH(CH₂)ₙ(OCH₂CH₂)ₘNH-,-NHSO₂(CH₂)ₙ(OCH₂CH₂)ₘNH-, -CONH(CH₂)ₙ(OCH₂CH₂)ₘNH-,-NHCO(CH₂)ₙ(OCH₂CH₂)ₘNH-, -CH₂(OCH₂CH₂)(CH₂)ₘNH-,-OCH₂(OCH₂CH₂)ₙ(CH₂)ₘNH-, -SCH₂(OCH₂CH₂)ₙ(CH₂)ₘNH-,-SO₂NHCH₂(OCH₂CH₂)ₙ(CH₂)ₘNH-, -NHSO₂CH₂(OCH₂CH₂)ₙ(CH₂)ₘNH-,-CONHCH₂(OCH₂CH₂)ₙ(CH₂)ₘNH-, -NHCOCH₂CH₂(OCH₂CH₂)ₙ(CH₂)ₘNH-,-CH₂CH₂(OCH₂CH₂)(CH₂)ₘNH-, -OCH₂CH₂(OCH₂CH₂)ₙ(CH₂)ₘNH-,-SCH₂CH₂(OCH₂CH₂)ₙ(CH₂)ₘNH-, -SO₂NHCH₂CH₂(OCH₂CH₂)ₙ(CH₂)ₘNHCO-,-NHSO₂CH₂CH₂(OCH₂CH₂)ₙ(CH₂)ₘNHCO-, -CONHCH₂CH₂(OCH₂CH₂)ₙ(CH₂)ₘNHCO-, -NHCOCH₂CH₂(OCH₂CH₂)ₙ(CH₂)ₘNHCO-,
      where n and m are independently selected from the group consisting of 0-25;
   and wherein Z is a chelator selected from: -H, 1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N'*-(5-acetyl (hydroxy)aminopentyl*-N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
   and wherein Z is optionally labeled with a metal selected from:
      ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
   and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt,-SO₂NHPr, -NH₂, -NHMe, -NHEt, -NHPr;
and pharmaceutically acceptable salts thereof.

In a second aspect, the present invention relates to a pharmaceutical formulation comprising a FAP inhibitor lignad of formula I.

In a third aspect, the present invention relates to FAP inhibitor ligand of formula I: 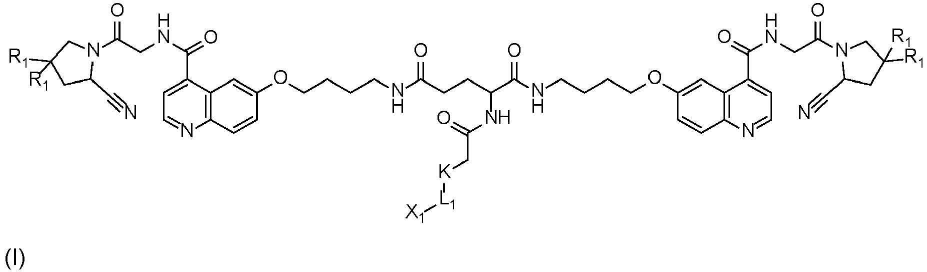 wherein:
R₁ is H or F
K is 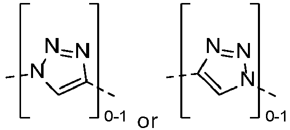
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-,-CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-,-COCH(CH₂CH₂COOH)NH-, -COCH(CH₂C₆H₅)NH-, -COCH(CH₂C₃H₃N₂)NH-,-COCH(CH(CH₃)₂)NH-, -COCH((CHCH₃)CH₂CH₃)NH-, -COCH((CH₂)₄NH₂)NH-,-COCH(CH₂CH(CH₃)₂)NH-, -COCH(CH₂CH₂SCH₃)NH-, -CO(CHCH₂CH₂N)-,-COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, -COCH(CH(OH)CH₃)NH-,-COCH(CH₂SeH)NH-, -COCH(CH₂C₈H₆N)NH-, -COCH(CH₂C₆H₄OH)NH-;-COCH(CH₂CH₂SeCH₃)NH-,
X₁ is a pyridazine selected from the group consisting of: 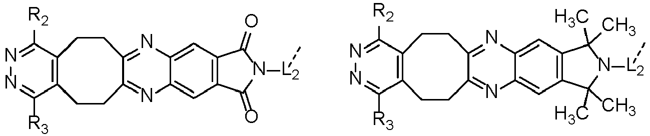 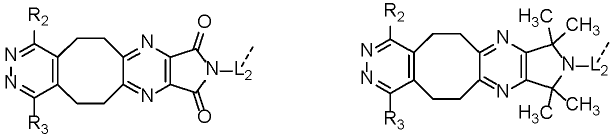 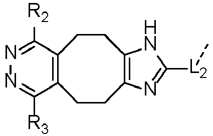
   wherein L₂ is -CO(CH₂)ₙ- or -CO(CH₂CH₂O)ₙ(CH₂)ₘ-,
   wherein n and m are integers independently selected from 1-10,
   R₂ is selected from -H, -Me, and R₃,
   R₃ is 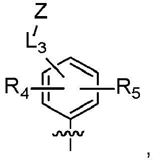 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁,-NHCH₂CH₂Y₁, -OCH₂C₆H₄Y₁, -OCH₂CH₂C₆H₄Y₁, -NHCOCH₂CH₂Y₁,-CONHCH₂CH₂Y₁, -NHSO₂CH₂CH₂Y₁, -SO₂NHCH₂CH₂Y₁,
   wherein Y₁ is selected from the group constisting of;
   ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At,²¹¹At,
   and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-,-(OCH₂CH₂)ₙ(CH₂)ₘNH-, -(CH₂)ₙ(OCH₂CH₂)ₘNH-, -O(CH₂)ₙ(OCH₂CH₂)ₘNH-,-S(CH₂)ₙ(OCH₂CH₂)ₘNH-, -SO₂NH(CH₂)ₙ(OCH₂CH₂)ₘNH-,-NHSO₂(CH₂)ₙ(OCH₂CH₂)ₘNH-, -CONH(CH₂)ₙ(OCH₂CH₂)ₘNH-,-NHCO(CH₂)ₙ(OCH₂CH₂)ₘNH-, -CH₂(OCH₂CH₂)(CH₂)ₘNH-,-OCH₂(OCH₂CH₂)ₙ(CH₂)ₘNH-, -SCH₂(OCH₂CH₂)ₙ(CH₂)ₘNH-,-SO₂NHCH₂(OCH₂CH₂)ₙ(CH₂)ₘNH-, -NHSO₂CH₂(OCH₂CH₂)ₙ(CH₂)ₘNH-,-CONHCH₂(OCH₂CH₂)ₙ(CH₂)ₘNH-, -NHCOCH₂CH₂(OCH₂CH₂)ₙ(CH₂)ₘNH-,-CH₂CH₂(OCH₂CH₂)(CH₂)ₘNH-, -OCH₂CH₂(OCH₂CH₂)ₙ(CH₂)ₘNH-,-SCH₂CH₂(OCH₂CH₂)ₙ(CH₂)ₘNH-, -SO₂NHCH₂CH₂(OCH₂CH₂)ₙ(CH₂)ₘNHCO-,-NHSO₂CH₂CH₂(OCH₂CH₂)ₙ(CH₂)ₘNHCO-, -CONHCH₂CH₂(OCH₂CH₂)ₙ(CH₂)ₘNHCO-, or -NHCOCH₂CH₂(OCH₂CH₂)ₙ(CH₂)ₘNHCO-,
   where n and m are independently selected from the group consisting of 0-25;
   and wherein Z is a chelator selected from: -H, 1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N'*-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
   and wherein Z is optionally labeled with a metal selected from:
      ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁶Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
   and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt,-SO₂NHPr, -NH₂, -NHMe, -NHEt, -NHPr;
and wherein said FAP inhibitor ligand of formula (I) comprises at least one radionuclide; and pharmaceutically acceptable salts thereof, for use as a medicament in therapy, imaging, diagnostics, or theranostics.

In a fourth aspect, the present invention relates to FAP inhibitor ligand of formula I: 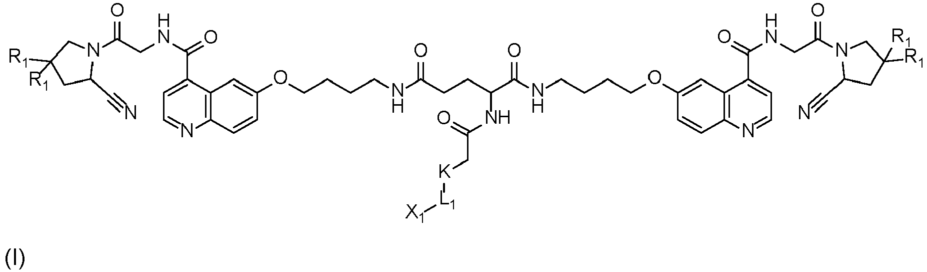 wherein:
R₁ is H or F
K is 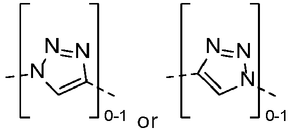
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-,-CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-,-COCH(CH₂CH₂COOH)NH-, -COCH(CH₂C₆H₅)NH-, -COCH(CH₂C₃H₃N₂)NH-,-COCH(CH(CH₃)₂)NH-, -COCH((CHCH₃)CH₂CH₃)NH-, -COCH((CH₂)₄NH₂)NH-,-COCH(CH₂CH(CH₃)₂)NH-, -COCH(CH₂CH₂SCH₃)NH-, -CO(CHCH₂CH₂N)-,-COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-,-COCH(CH(OH)CH₃)NH-, -COCH(CH₂SeH)NH-, -COCH(CH₂C₈H₆N)NH-,-COCH(CH₂C₆H₄OH)NH-; -COCH(CH₂CH₂SeCH₃)NH-,
X₁ is a pyridazine selected from the group consisting of: 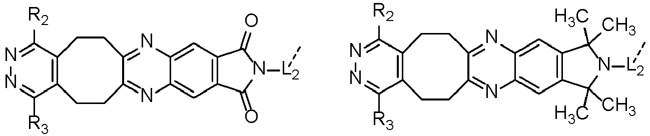 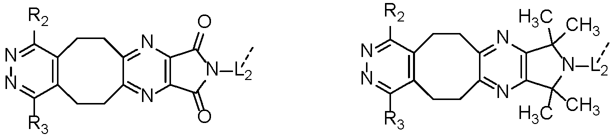 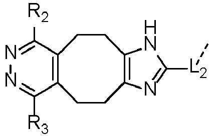
   wherein L₂ is -CO(CH₂)ₙ- or -CO(CH₂CH₂O)ₙ(CH₂)ₘ-,
   wherein n and m are integers independently selected from 1-10,
   R₂ is independently selected from -H, -Me, and R₃,
   R₃ is 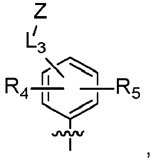 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁,-NHCH₂CH₂Y₁, -OCH₂C₆H₄Y₁, -OCH₂CH₂C₆H₄Y₁, -NHCOCH₂CH₂Y₁,-CONHCH₂CH₂Y₁, -NHSO₂CH₂CH₂Y₁, -SO₂NHCH₂CH₂Y₁,
   wherein Y₁ is selected from the group constisting of;
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At, ²¹¹At,
   and wherein L₃ is selected from: -H, -(CH₂)ₙNH-, -O(CH₂)ₙNH-, -S(CH₂)ₙNH-,-SO₂NH(CH₂)ₙNH-, -NHSO₂(CH₂)ₙNH-, -CONH(CH₂)ₙNH-, -NHCO(CH₂)ₙNH-,-(OCH₂CH₂)ₙ(CH₂)ₘNH-, -(CH₂)ₙ(OCH₂CH₂)ₘNH-, -O(CH₂)ₙ(OCH₂CH₂)ₘNH-,-S(CH₂)ₙ(OCH₂CH₂)ₘNH-, -SO₂NH(CH₂)ₙ(OCH₂CH₂)ₘNH-,-NHSO₂(CH₂)ₙ(OCH₂CH₂)ₘNH-, -CONH(CH₂)ₙ(OCH₂CH₂)ₘNH-,-NHCO(CH₂)ₙ(OCH₂CH₂)ₘNH-, -CH₂(OCH₂CH₂)(CH₂)ₘNH-,-OCH₂(OCH₂CH₂)ₙ(CH₂)ₘNH-, -SCH₂(OCH₂CH₂)ₙ(CH₂)ₘNH-,-SO₂NHCH₂(OCH₂CH₂)ₙ(CH₂)ₘNH-, -NHSO₂CH₂(OCH₂CH₂)ₙ(CH₂)ₘNH-,-CONHCH₂(OCH₂CH₂)ₙ(CH₂)ₘNH-, -NHCOCH₂CH₂(OCH₂CH₂)ₙ(CH₂)ₘNH-,-CH₂CH₂(OCH₂CH₂)(CH₂)ₘNH-, -OCH₂CH₂(OCH₂CH₂)ₙ(CH₂)ₘNH-,-SCH₂CH₂(OCH₂CH₂)ₙ(CH₂)ₘNH-, -SO₂NHCH₂CH₂(OCH₂CH₂)ₙ(CH₂)ₘNHCO-,-NHSO₂CH₂CH₂(OCH₂CH₂)ₙ(CH₂)ₘNHCO-, -CONHCH₂CH₂(OCH₂CH₂)ₙ(CH₂)ₘNHCO-, -NHCOCH₂CH₂(OCH₂CH₂)ₙ(CH₂)ₘNHCO-,
   where n and m are independently selected from the group consisting of 0-25;
   and wherein Z is a chelator selected from: -H, 1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N'*-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
   and wherein Z is optionally labeled with a metal selected from:
      ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁶Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
   and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt,-SO₂NHPr, -NH₂, -NHMe, -NHEt, -NHPr;
and wherein said FAP inhibitor ligand of formula (I) comprises at least one radionuclide; and pharmaceutically acceptable salts thereof for use in the treatment and/ or in the diagnosis of FAP expressing diseases including cancer, such as breast cancer, lung cancer, colorectal cancer, prostate cancer, ovarian cancer, pancreatic cancer, hepatocellular cancers, sarcomas and non-small cell lung cancer; and fibrosis, arthritis, atherosclerosis, and inflammatory diseases like spondyloarthritis.

In a fifth aspect, the present invention relates to FAP inhibitor precursor of formula (II): 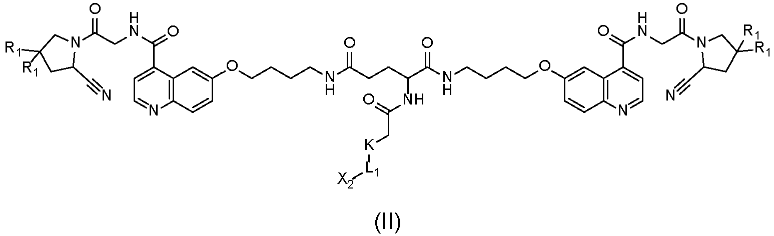 wherein:
R₁ is H or F
K is 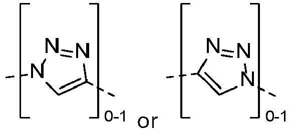
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-,-CH₂C(COOH)NHAₘ-, -CH₂CH₂C(COOH)NHAₘ-
   wherein n and m are integers independently selected from 0-20,
   and A is an amino acid independently selected from the group consisting of; -COCH₂NH-, -COCH(CH₃)NH-, -COCH(CH₂SH)NH-, -COCH(CH₂COOH)NH-,-COCH(CH₂CH₂COOH)NH-, -COCH(CH₂C₆H₅)NH-, -COCH(CH₂C₃H₃N₂)NH-,-COCH(CH(CH₃)₂)NH-, -COCH((CHCH₃)CH₂CH₃)NH-, -COCH((CH₂)₄NH₂)NH-,-COCH(CH₂CH(CH₃)₂)NH-, -COCH(CH₂CH₂SCH₃)NH-, -CO(CHCH₂CH₂N)-,-COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-,-COCH(CH(OH)CH₃)NH-, -COCH(CH₂SeH)NH-, -COCH(CH₂C₈H₆N)NH-,-COCH(CH₂C₆H₄OH)NH-; -COCH(CH₂CH₂SeCH₃)NH-,
X₂ is a cyclooctene selected from the group consisting of: 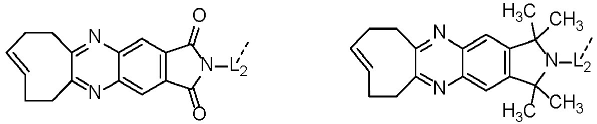 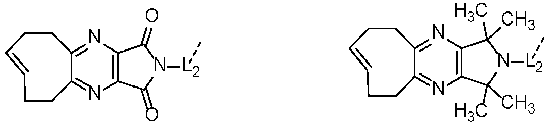 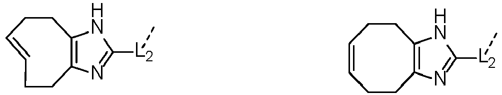 wherein L₂ is -CO(CH₂)ₙ- or -CO(CH₂CH₂O)ₙ(CH₂)ₘ-,
wherein n and m are integers independently selected from 1-10,

### Brief description of the drawings

Figure 1: Scheme showing the synthesis of compound **12.**
Figure 2A and 2B: Scheme showing the synthesis of compound **16.**
Figure 3: Scheme showing the synthesis of compound **17.**
Figure 4A and 4B: Scheme showing the synthesis of compound **LVIII**.
Figure 5: Scheme showing the synthesis of compound **20.**
Figure 6A and 6B: Scheme showing the synthesis of compound **22.**
Figure 7: Scheme showing the synthesis of compound **23.**
Figure 8: Scheme showing the synthesis of compound **I.**
Figure 9A: Scheme showing the synthesis of compound **[⁶⁸Ga]24.**
Figure 9B: Scheme showing the synthesis of compound **[¹¹¹In]25.**
Figure 10: Scheme showing the synthesis of compound **[⁶⁸Ga]19.**
Figure 11: Scheme showing the synthesis of compound **XXXIV.**
Figure 12: Scheme showing the synthesis of compound **XXXV.**
Figure 13: Scheme showing the synthesis of compound **[⁶⁸Ga]25.**
Figure 14: Scheme showing the synthesis of compound **[¹¹¹In]16.**
Figure 15: Scheme showing the synthesis of compound **XXXVII.**
Figure 16: Graphs showing A: Time-activity curves (%ID/mL tissue overtime, h) and B: tumor-to-muscle ratios over time (h) for ¹¹¹In-labeled FAPi derivatives.
Figure 17: Bar diagram showing the effect of FAP blockade (%ID/g) on the uptake of ¹¹¹In-labeled FAPi derivatives.
Figure 18: Table showing biodistribution (%ID/g) of **XXXVII** based on ex vivo gamma counting results.
Figure 19: Table showing biodistribution (%ID/g) of [¹¹¹In]**25** based on ex vivo gamma counting results.
Figure 20: Table showing biodistribution (%ID/g) of **[¹¹¹In]16** based on ex vivo gamma counting results.

### Detailed description of the invention

The FAP inhibitor (FAPi) ligand compounds of the present invention comprises a FAP targeting moiety, a linker, an IsoF-TCO and a radiolabeled tetrazine. The FAPi targeting compounds are possessing advantageous pharmacokinetic properties and enable quick and efficient modifications of the radiopharmaceuticals allowing the fast renal excretion and reduced liver accumulation.

The present FAP inhibitor ligand compounds were developed by labeling tetrazines synthon and ligate this synthon to a precursor moiety containing an IsoF-TCO.

The radiolabeled compounds provided herein show a surprisingly higher tumor accumulation compared to [¹¹¹In]In-DOTAGA.Glu.(FAPi) ([¹¹¹In]**25**).

The present compounds can be labeled with either a radiohalogen or a radiometal providing improved flexible use of these FAP inhibitor ligands compared to ligands that are designed to for labeling with a radiohalogen only or with a radiometal only.

The structure of the FAP inhibitor ligands herein is of formula (I): 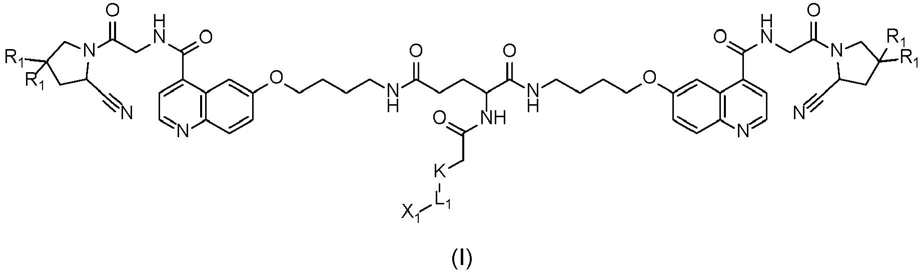 wherein:
R₁ is H or F
K is 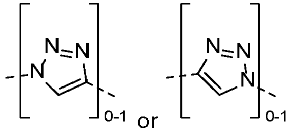
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-,-CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-,-COCH(CH₂CH₂COOH)NH-, -COCH(CH₂C₆H₅)NH-, -COCH(CH₂C₃H₃N₂)NH-,-COCH(CH(CH₃)₂)NH-, -COCH((CHCH₃)CH₂CH₃)NH-, -COCH((CH₂)₄NH₂)NH-,-COCH(CH₂CH(CH₃)₂)NH-, -COCH(CH₂CH₂SCH₃)NH-, -CO(CHCH₂CH₂N)-,-COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-,-COCH(CH(OH)CH₃)NH-, -COCH(CH₂SeH)NH-, -COCH(CH₂C₈H₆N)NH-,-COCH(CH₂C₆H₄OH)NH-, -COCH(CH₂CH₂SeCH₃)NH-;
X₁ is a pyridazine selected from the group consisting of: 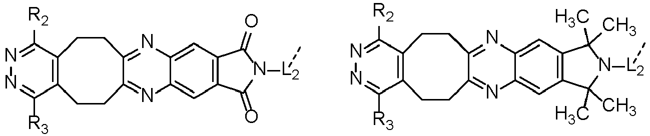 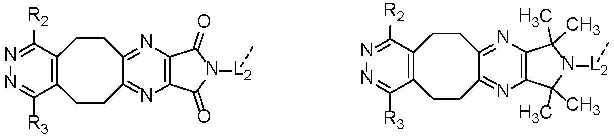 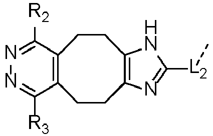
   wherein L₂ is -CO(CH₂)ₙ- or -CO(CH₂CH₂O)ₙ(CH₂)ₘ-,
   wherein n and m are integers independently selected from 1-10, and
R₂ is selected from -H, -Me, and R₃,
R₃ is 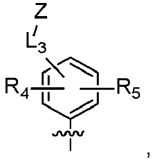 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, -NHCH₂CH₂Y₁,-OCH₂C₆H₄Y₁, -OCH₂CH₂C₆H₄Y₁, -NHCOCH₂CH₂Y₁, -CONHCH₂CH₂Y₁, -NHSO₂CH₂CH₂Y₁,-SO₂NHCH₂CH₂Y₁,
   wherein Y₁ is selected from the group constisting of:
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At, ²¹¹At,
   and wherein L₃ is selected from: -H, -(CH₂)ₙNH-, -O(CH₂)ₙNH-, -S(CH₂)ₙNH-,-SO₂NH(CH₂)ₙNH-, -NHSO₂(CH₂)ₙNH-, -CONH(CH₂)ₙNH-, -NHCO(CH₂)ₙNH-,-(OCH₂CH₂)ₙ(CH₂)ₘNH-, -(CH₂)ₙ(OCH₂CH₂)ₘNH-, -O(CH₂)ₙ(OCH₂CH₂)ₘNH-,-S(CH₂)ₙ(OCH₂CH₂)ₘNH-, -SO₂NH(CH₂)ₙ(OCH₂CH₂)ₘNH-,-NHSO₂(CH₂)ₙ(OCH₂CH₂)ₘNH-, -CONH(CH₂)ₙ(OCH₂CH₂)ₘNH-,-NHCO(CH₂)ₙ(OCH₂CH₂)ₘNH-, -CH₂(OCH₂CH₂)(CH₂)ₘNH-,-OCH₂(OCH₂CH₂)ₙ(CH₂)ₘNH-, -SCH₂(OCH₂CH₂)ₙ(CH₂)ₘNH-,-SO₂NHCH₂(OCH₂CH₂)ₙ(CH₂)ₘNH-, -NHSO₂CH₂(OCH₂CH₂)ₙ(CH₂)ₘNH-,-CONHCH₂(OCH₂CH₂)ₙ(CH₂)ₘNH-, -NHCOCH₂CH₂(OCH₂CH₂)ₙ(CH₂)ₘNH-,-CH₂CH₂(OCH₂CH₂)(CH₂)ₘNH-, -OCH₂CH₂(OCH₂CH₂)ₙ(CH₂)ₘNH-,-SCH₂CH₂(OCH₂CH₂)ₙ(CH₂)ₘNH-, -SO₂NHCH₂CH₂(OCH₂CH₂)ₙ(CH₂)ₘNHCO-,-NHSO₂CH₂CH₂(OCH₂CH₂)ₙ(CH₂)ₘNHCO-, -CONHCH₂CH₂(OCH₂CH₂)ₙ(CH₂)ₘNHCO-, -NHCOCH₂CH₂(OCH₂CH₂)ₙ(CH₂)ₘNHCO-,
   where n and m are independently selected from the group consisting of 0-25;
and wherein Z is a chelator selected from: -H, 1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N'*-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans-*cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
and wherein Z is optionally labeled with a metal selected from:
   ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt, - SO₂NHPr, -NH₂, -NHMe, -NHEt, -NHPr;
and pharmaceutically acceptable salts thereof.

The FAP inhibitor targeting region of the FAP inhibitor ligand of formula (I) is based on the UAMC1110molecule which has shown to effectively bind to FAP with high specificity in cancer-associated fibroblasts.

Apart from the FAP inhibitor targeting region, the compounds of formula (I) comprises the entities K, L₁ and X₁.

K is an triazole, which allows for ease of connection of the targeting region to the linker region.

L₁ is a linker linking X₁ to the FAPi targeting site via K. L₁ is selected from: -(CH₂)ₙNH-, - CH₂(OCH₂CH₂)ₙNH-, -(CH₂)ₙNHAm-, -CH₂(OCH₂CH₂)ₙNHAₘ-, -CH₂C(COOH)NHAₘ-, - CH₂CH₂C(COOH)NHAₘ-. wherein n and m are integers independently selected from 0-20, and A is an amino acid. Thus, the linker may comprise no amino acids or one or more amino acids up to 20 amino acids.

In a preferred embodiment, the FAP inhibitor ligand of formula (I) comprises a linker L₁ that includes one or more amino acids, wherein A represents a polar natural amino acid or its D-enantiomer. The inclusion of polar amino acids in the linker significantly enhances the solubility and stability of the inhibitor in aqueous environments, which is critical for effective biological activity and delivery. Polar amino acids, such as serine, threonine, asparagine, glutamine, or their D-enantiomers, possess side chains that can engage in hydrogen bonding and other interactions with water molecules. This improves the overall hydrophilicity of the linker, thereby facilitating better dispersion and bioavailability of the FAP inhibitor in biological fluids.

Additionally, polar amino acids can influence the overall conformation and flexibility of the linker, allowing for optimal positioning of the inhibitor's active site relative to its target. This can enhance the binding affinity and specificity of the inhibitor, leading to improved inhibition of FAP activity. Furthermore, the use of D-enantiomers of these amino acids may confer resistance to proteolytic degradation, increasing the stability and half-life of the inhibitor in vivo. This stability is particularly advantageous in therapeutic applications, as it can lead to sustained inhibition of FAP activity, thereby enhancing the therapeutic efficacy of the compound.

X₁ is a pyridazine comprising one or two R₃ groups: 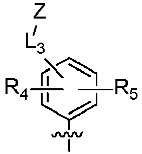 wherein R₄ optionally comprises Y₁ which is a halogen such as a radiohalogen. The group 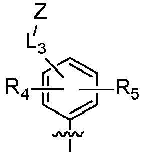 also comprises Z which is a chelator. Chelators are suitable for binding metals including radiometals.

The halogen Y₁ in R₄ is selected from ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At, ²¹¹At. The following halogens from this selection are radionuclides: ³H, ¹⁴C, ¹⁸F, ⁷⁶Br, ⁷⁷Br, ⁸⁰Br, ^{80m}Br, ¹²³I, ¹²⁴I,¹²⁵I, ¹²⁶I, ¹³¹I, ²¹⁰At, and ²¹¹At. FAP inhibitor ligands of formula (I) comprising a radionuclide halogen, also referred to as radiohalogens, can be used for imaging for example in relation to diagnostics, and in therapy. ¹⁸F, ⁷⁶Br, ¹²³I, ¹²⁴I,¹²⁵I, ¹³¹I, are used in PET or SPECT imaging whereas ⁷⁶Br, ⁷⁷Br, ¹²⁶I, ²¹⁰At, and ²¹¹At are used in therapy. ³H, ¹⁴C, ⁸⁰Br, ^{80m}Br, are primarily used for scientific purposes such as tracers or proof-of-concept tools.

The following halogens from the Y₁ selection are stable nuclides: ¹H, ²H, ¹¹C, ¹²C, ¹³C, ¹⁹F, ⁷⁹Br, ⁸¹Br, and ¹²⁷I. FAP inhibitor ligands of formula (I) comprising a stable halogen can be used for scientific purposes or when the compound comprises another radionuclide for example at position Z, the chelator, which can optionally be labelled with a radiometal.

The chelator Z may be unlabled, or labled with a metal selected from: ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th. This group comprises stable metals as well as radionuclides, also referred to as radiometals.

The metals ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, and ²⁰⁹Bi are stable metals whereas ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr ⁹⁴Tc, ^{99m}Tc, ^{103m}Rh, ^{193m}Pt, ^{195m}Pt, ¹⁹⁸Pt, ¹¹¹In, ¹¹³In, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th are radiometals.

FAP inhibitor ligands of formula (I) comprising a radiometal, can be used for imaging for example in relation to diagnostics, and in therapy, or for scientific purposes. ⁴³Sc, ⁴⁴Sc, ⁴⁷Sc, ⁴⁵Ti, ⁵⁵Co, ⁶⁰Cu, ⁶¹Cu, ⁶⁴Cu, ⁶⁷Ga, ⁶⁸Ga, ⁷²As, ⁸⁹Zr, ⁹⁴Tc, ^{99m}Tc, ¹¹¹In, and ¹¹³In, are primarily used for imaging such as PET or SPECT and scientific purposes whereas ⁴⁷Sc, ⁵¹Cr, ^{58m}Co, ⁶⁷Cu, ⁸⁹Sr, ⁸⁶Y, ⁹⁰Y, ⁹⁶Zr, ^{103m}Rh, ^{193m}Pt, ^{195m}Pt, ¹⁹⁸Pt, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th are primarily used in therapy and/or for scientific purposes.

The stable metal can be selected when use is for scientific purposes or when the FAP inhibitor ligand of formula I comprises a radiohalogen.

A FAP inhibitor ligand according to formula I is accordingly applicable for several different purposes depending on the choice of halogen, optionally the choice of metal and moreover whether the halogen and/or metal is a radionuclide.

This flexibility of the structure is of particular relevance in theranostic which combines diagnostic imaging and targeted therapy, a powerful concept in personalized medicine aiding patient selection, dose-finding, and therapy response monitoring. A theranostic pair consists of two radionuclides interchangeable without altering the radiopharmaceutical's pharmacokinetics, enabling applications in both diagnostic imaging and radionuclide therapy.

When the application of the FAP inhibitor ligand of formula (I) is based on the features of a radiohalogen, the chelator is preferably unlabeled or labeled with a stable metal. When the application of the FAP inhibitor ligand of formula (I) is based on the features of a radiometal, R₄ is preferably H.

Thus, when the FAP inhibitor ligand of formula (I) to be applied in imaging, therapy, diagnostic or theranostics, it is preferred that the compound comprises a 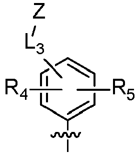 moiety capable of being labelled with a radionuclide.

In a preferred embodiment, the FAP inhibitor ligand of formula (I) is a compound wherein R₄ is selected from Y₁, -OCH₂CH₂Y₁, CH₂Y₁, -SCH₂CH₂Y₁, -NHCH₂CH₂Y₁, -OCH₂C₆H₄Y₁, - OCH₂CH₂C₆H₄Y₁, -NHCOCH₂CH₂Y₁, -CONHCH₂CH₂Y₁, -NHSO₂CH₂CH₂Y₁, - SO₂NHCH₂CH₂Y₁,

In another preferred embodiment the FAP inhibitor ligand of formula (I) is a compound wherein R₄ is selected from Y₁, -OCH₂CH₂Y₁, CH₂Y₁, -SCH₂CH₂Y₁, -NHCH₂CH₂Y₁, -OCH₂C₆H₄Y₁, - OCH₂CH₂C₆H₄Y₁, -NHCOCH₂CH₂Y₁, -CONHCH₂CH₂Y₁, -NHSO₂CH₂CH₂Y₁, - SO₂NHCH₂CH₂Y₁,
and wherein Y₁ is a halogen selected from ³H, ¹¹C, ¹⁸F, ¹²³I, ¹³¹I, ²¹⁰At and ²¹¹At.

These radionuclides are particularly relevant as they offer distinct advantages due to their diverse nuclear properties, making them highly suitable for both diagnostic imaging and therapeutic applications.

For diagnostic imaging, ¹¹C and ¹⁸F are positron-emitting radionuclides commonly used in positron emission tomography (PET). ¹¹C, with a short half-life of approximately 20 minutes, is ideal for labeling small molecules in metabolic studies, allowing real-time imaging of physiological processes. ¹⁸F, on the other hand, has a longer half-life of about 110 minutes, providing greater flexibility in synthesis and broader distribution. Its high positron emission efficiency results in excellent image resolution, making it a popular choice for PET imaging.

¹²³I and ¹³¹I serve both diagnostic and therapeutic purposes. ¹²³I, with a half-life of approximately 13 hours and gamma emissions, is well-suited for single-photon emission computed tomography (SPECT), enabling high-quality imaging with minimal patient radiation exposure. ¹³¹I, with a longer half-life of about 8 days, emits both beta and gamma radiation, making it effective for imaging and therapy, especially in the treatment of thyroid diseases.

For therapeutic applications, ²¹⁰At and ²¹¹At are alpha-emitting radionuclides known for their high linear energy transfer (LET). This characteristic leads to substantial cytotoxic effects confined to targeted cells, minimizing damage to surrounding healthy tissue. ²¹¹At, in particular, with its half-life of approximately 7.2 hours, offers a practical balance between therapeutic efficacy and patient safety, making it suitable for targeted cancer therapies.

³H, with its low-energy beta emissions and long half-life of about 12.3 years, is primarily used in biological and biochemical research. It is beneficial in tracing and analyzing biological pathways and processes, due to its stability and minimal radiological hazard.

The selection of these radionuclides enhances the versatility and functionality of the FAP compounds. They provide a broad range of options for radiolabeling, accommodating both short-lived and long-lived radionuclides.

In a more preferred embodiment, the FAP inhibitor ligand of formula (I) is a compound wherein R₄ is selected from Y₁, -OCH₂CH₂Y₁, CH₂Y₁, -SCH₂CH₂Y₁, -NHCH₂CH₂Y₁, -OCH₂C₆H₄Y₁, - OCH₂CH₂C₆H₄Y₁, -NHCOCH₂CH₂Y₁, -CONHCH₂CH₂Y₁, -NHSO₂CH₂CH₂Y₁, - SO₂NHCH₂CH₂Y₁,
and wherein Y₁ is a radionuclide selected from ¹³¹I or ²¹¹At.

For all embodiments wherein the FAP inhibitor ligand of formula (I) comprises a radiohalogen, that is when Y₁ is selected from: ¹H, ²H, ¹¹C, ¹²C, ¹³C, ¹⁹F, ⁷⁹Br, ⁸¹Br, ¹²⁷I, the chelator Z is unlabeled or Z should be labelled with a stable metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, and ²⁰⁹Bi.

In a preferred embodiment, the FAP inhibitor ligand of formula (I) is selected from the group consisting of **I-XXVIII:**

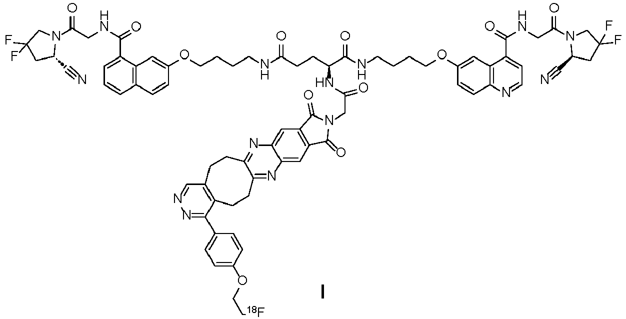

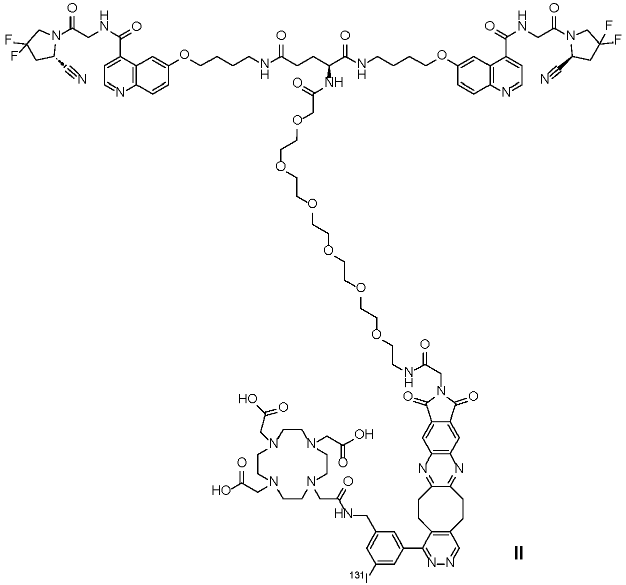

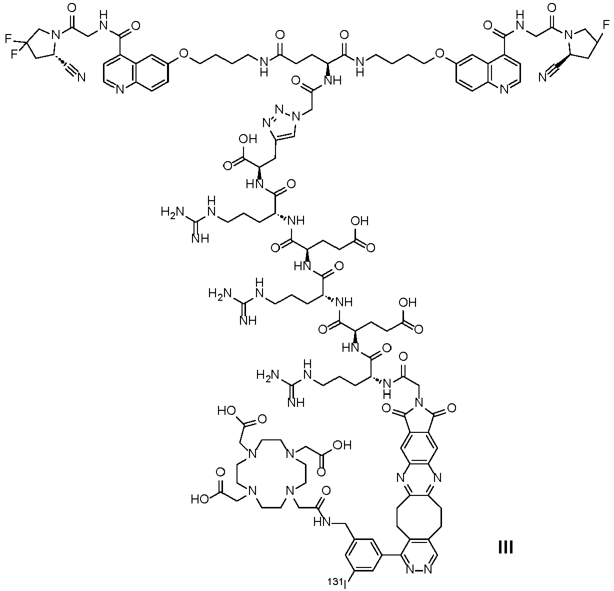

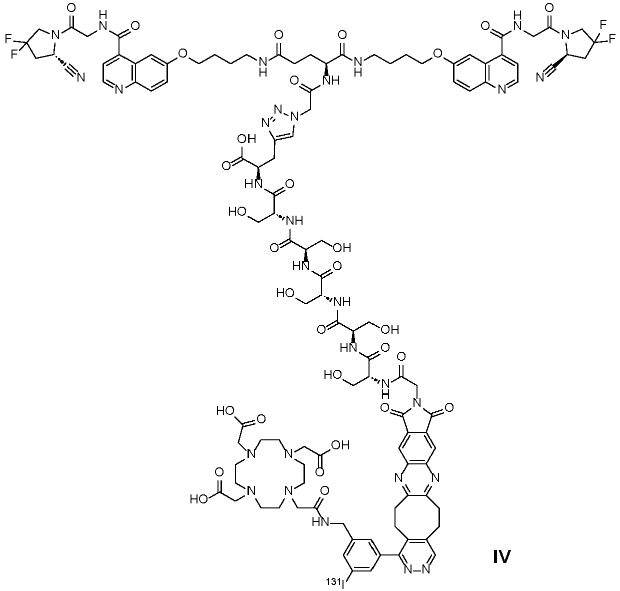

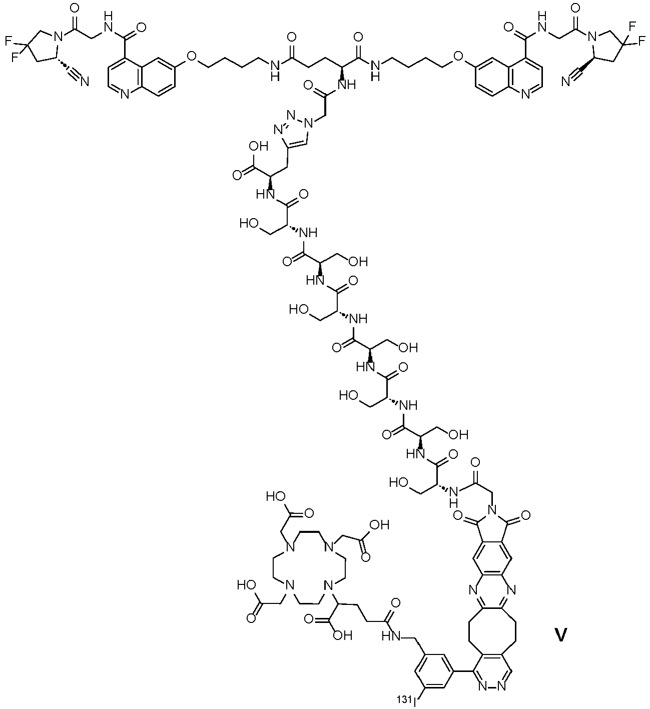

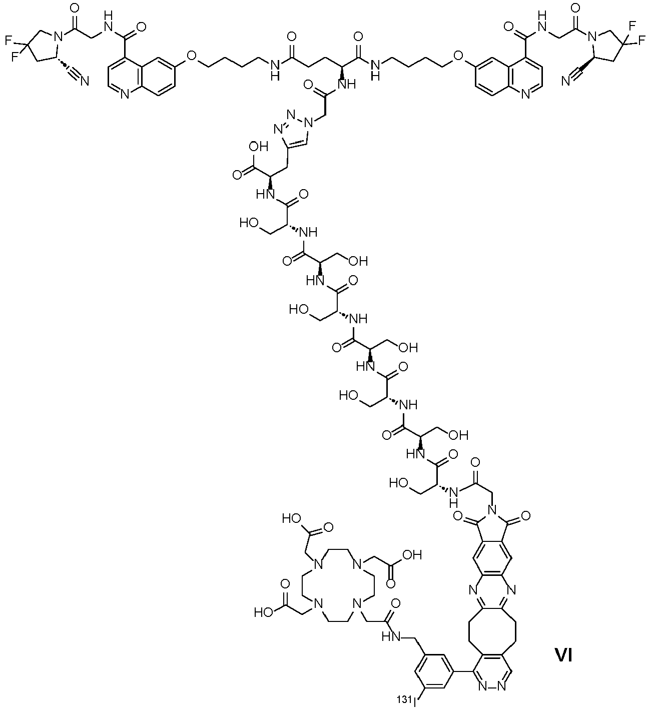

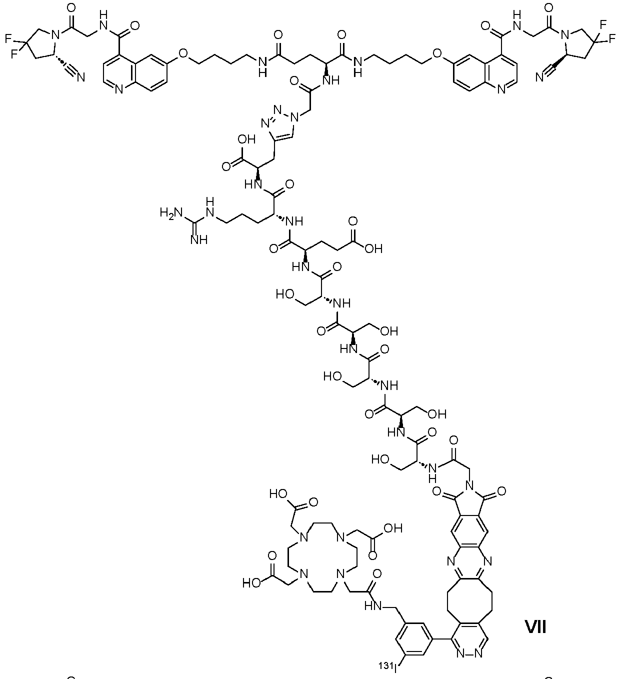

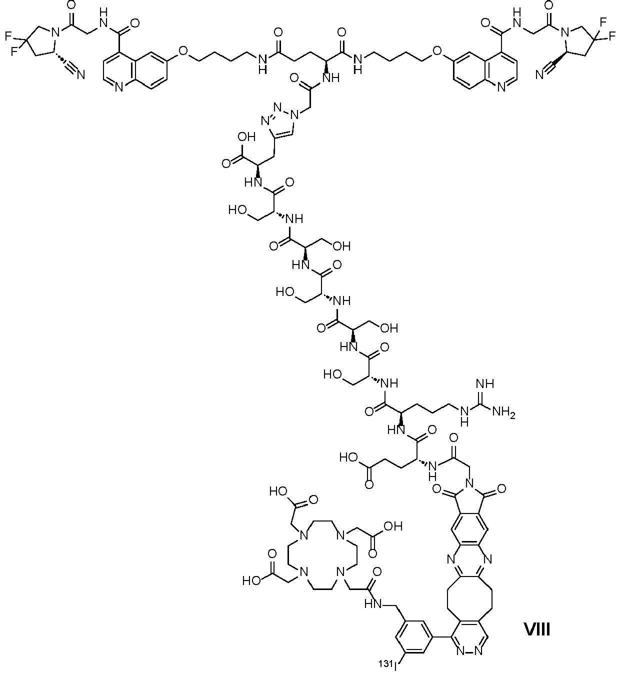

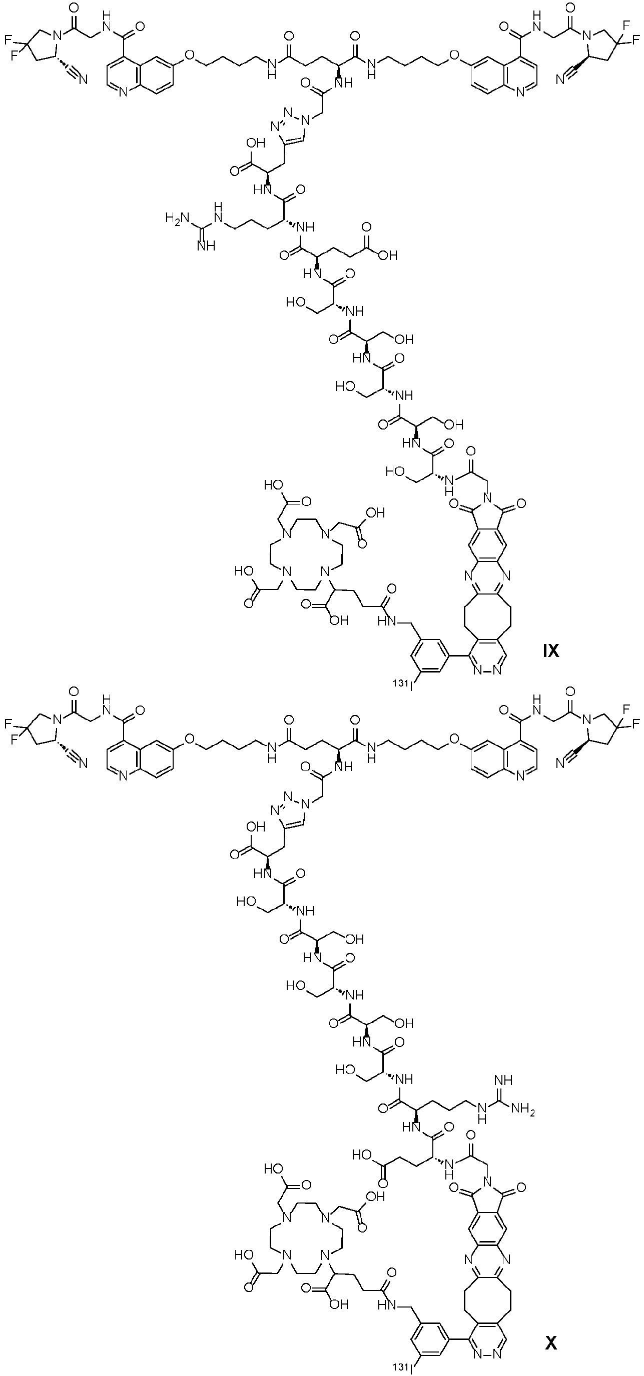

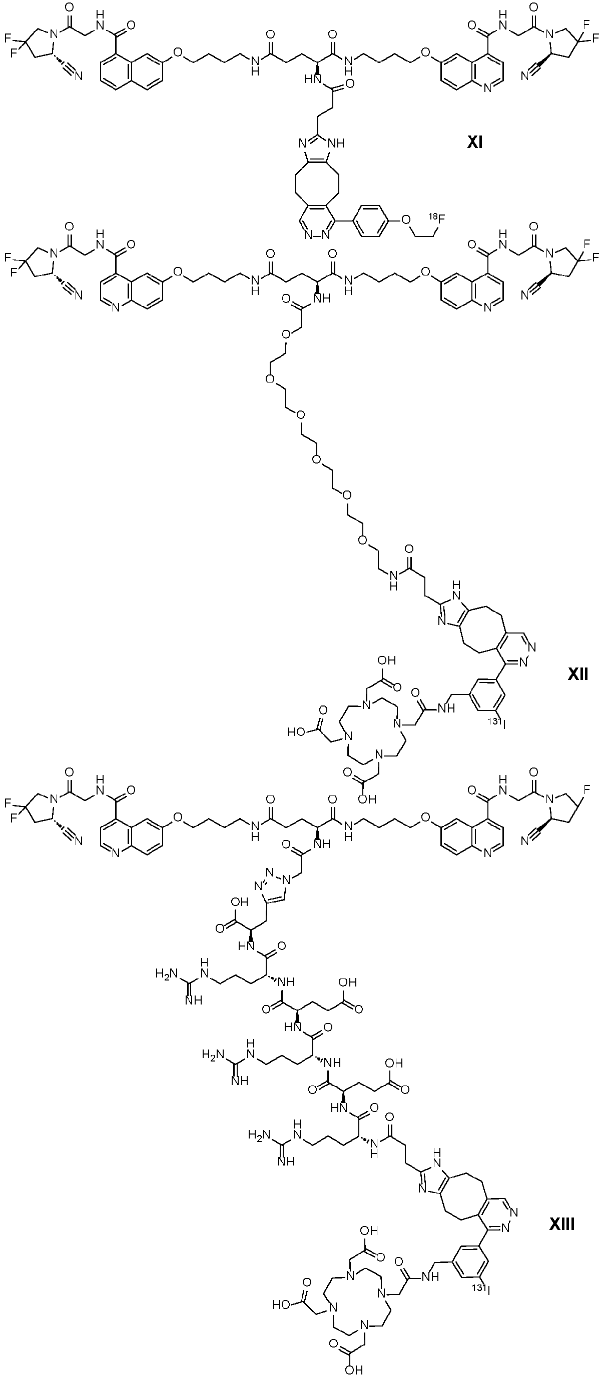

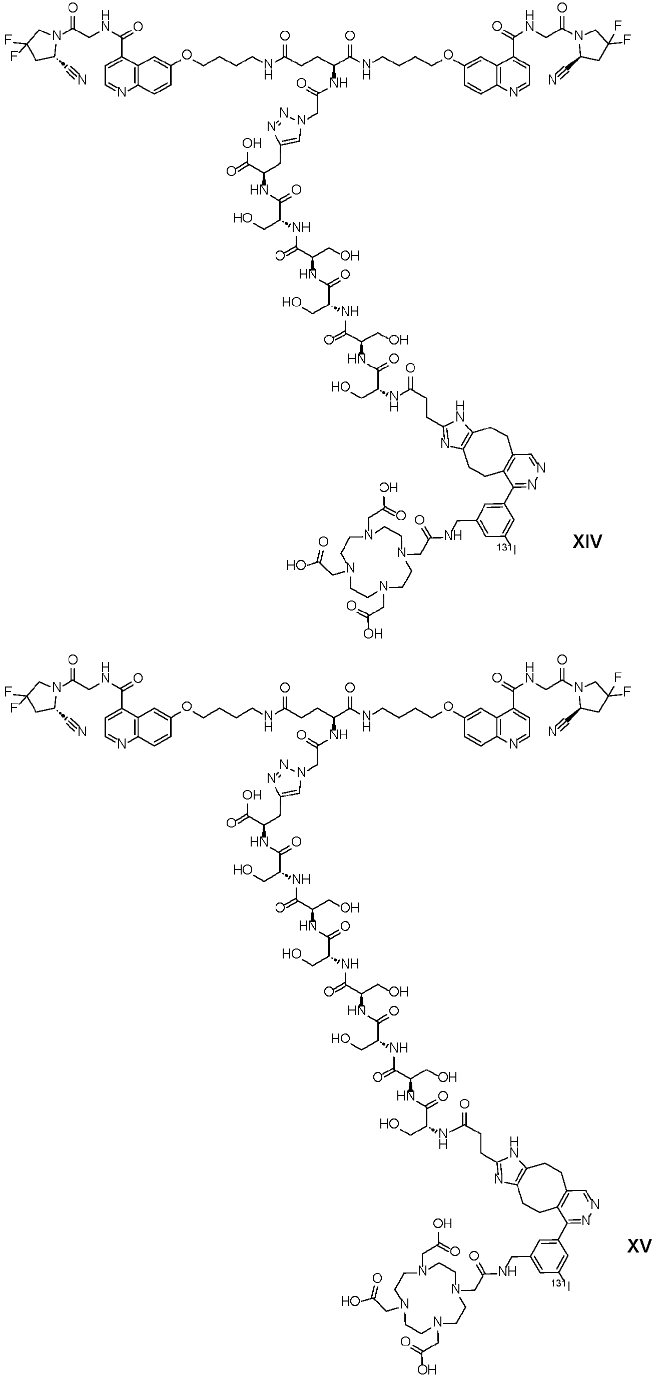

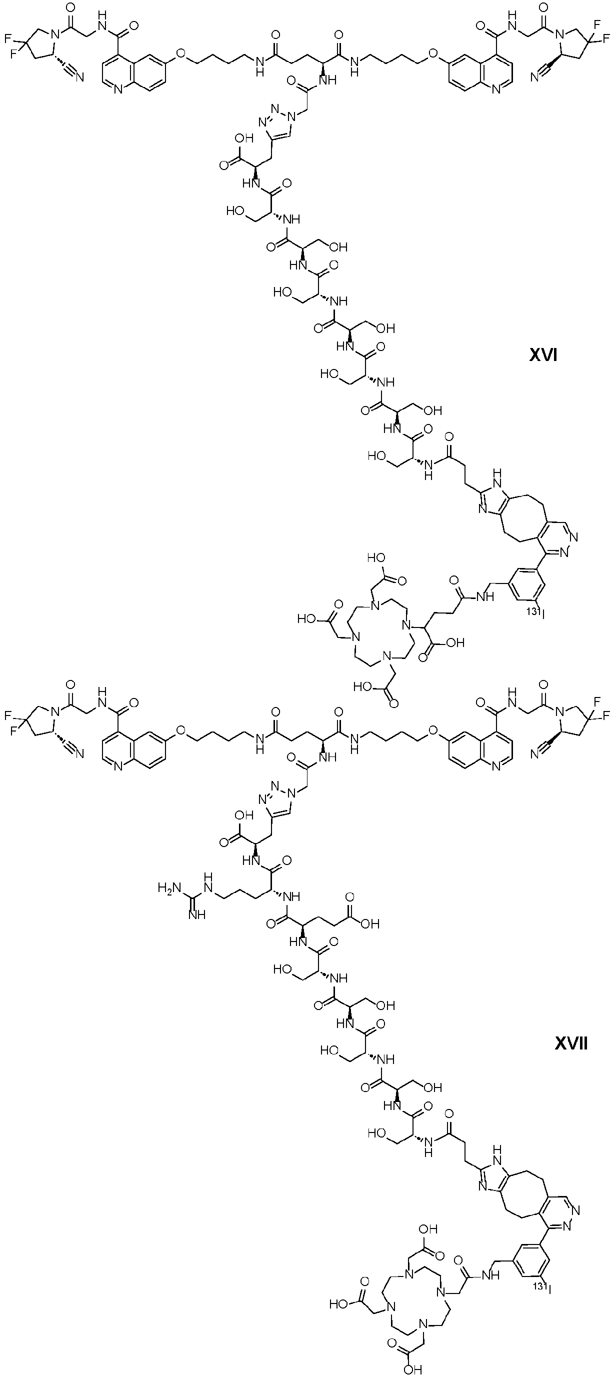

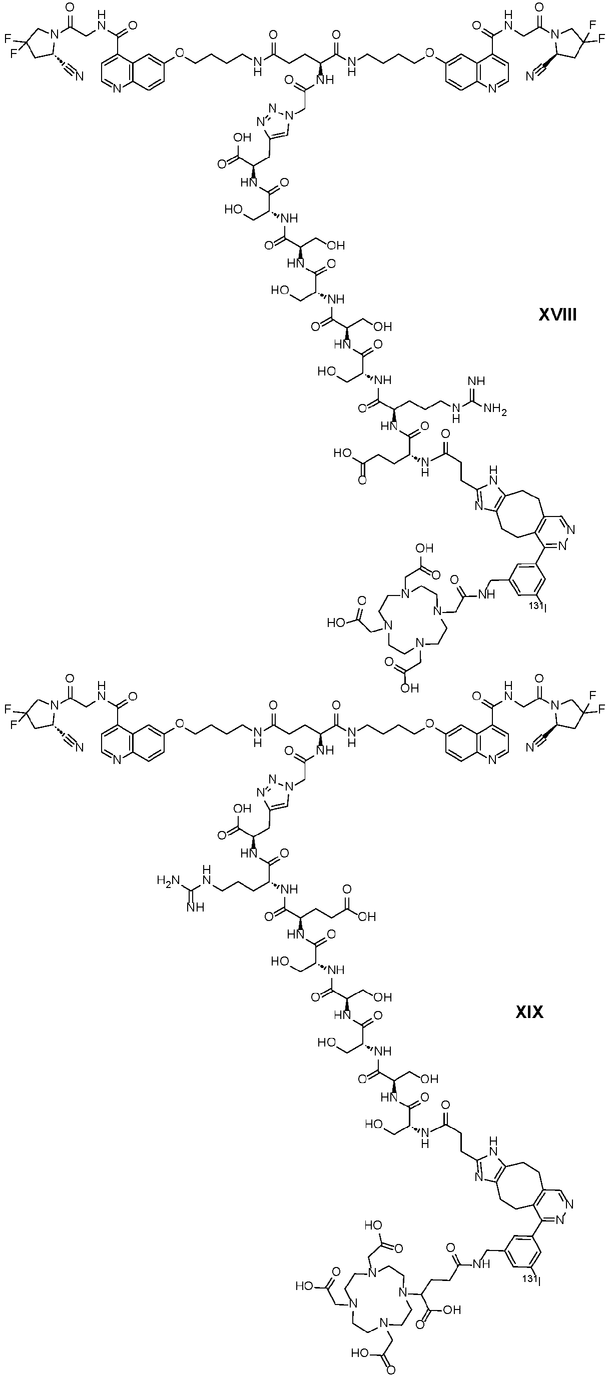

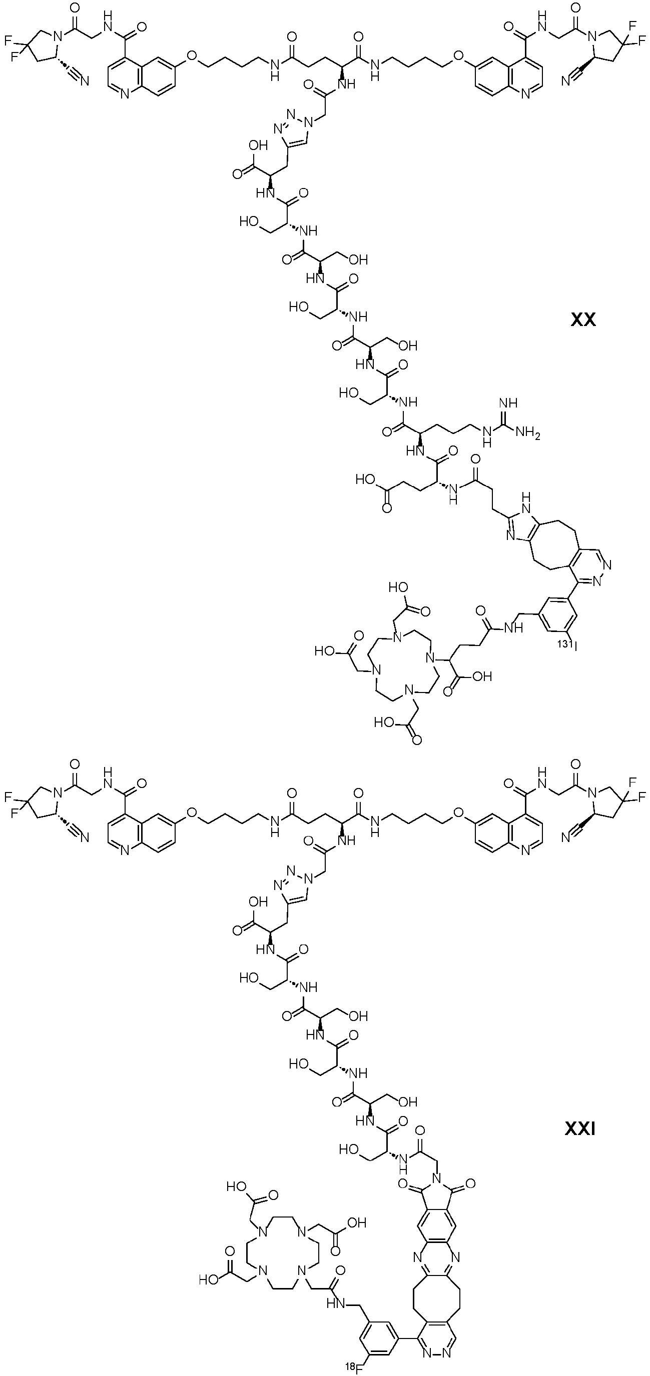

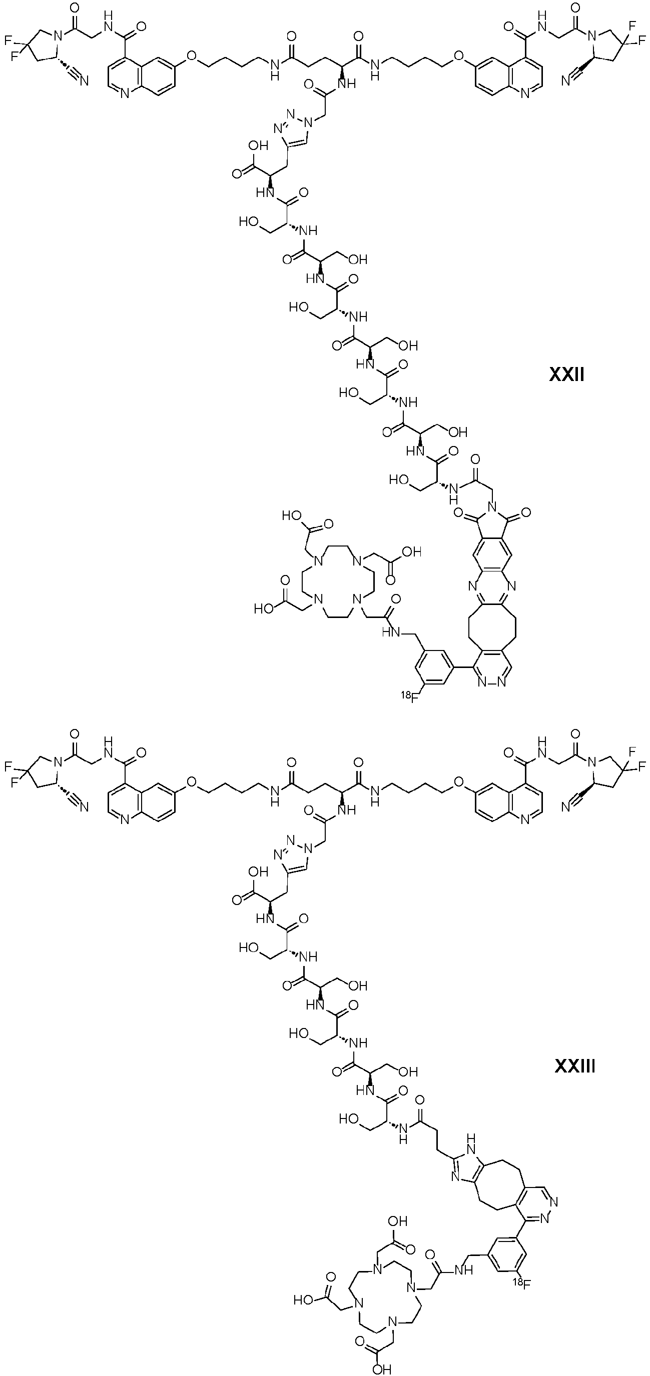

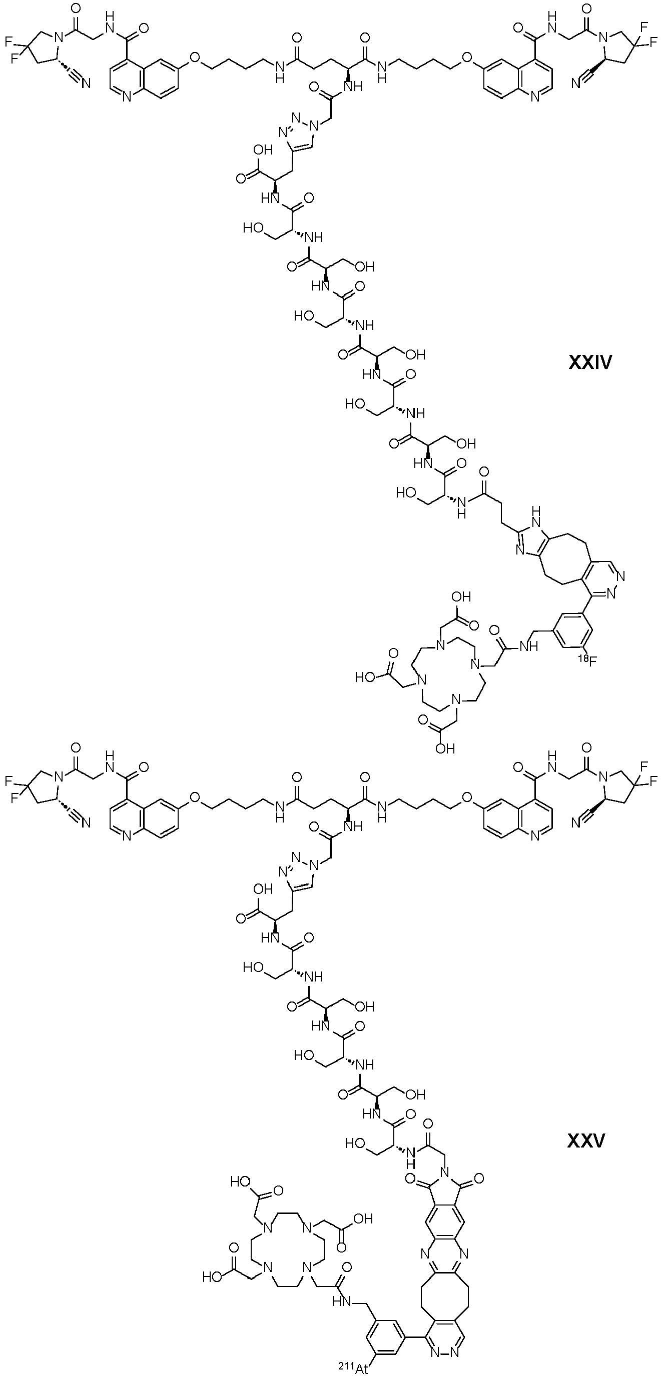

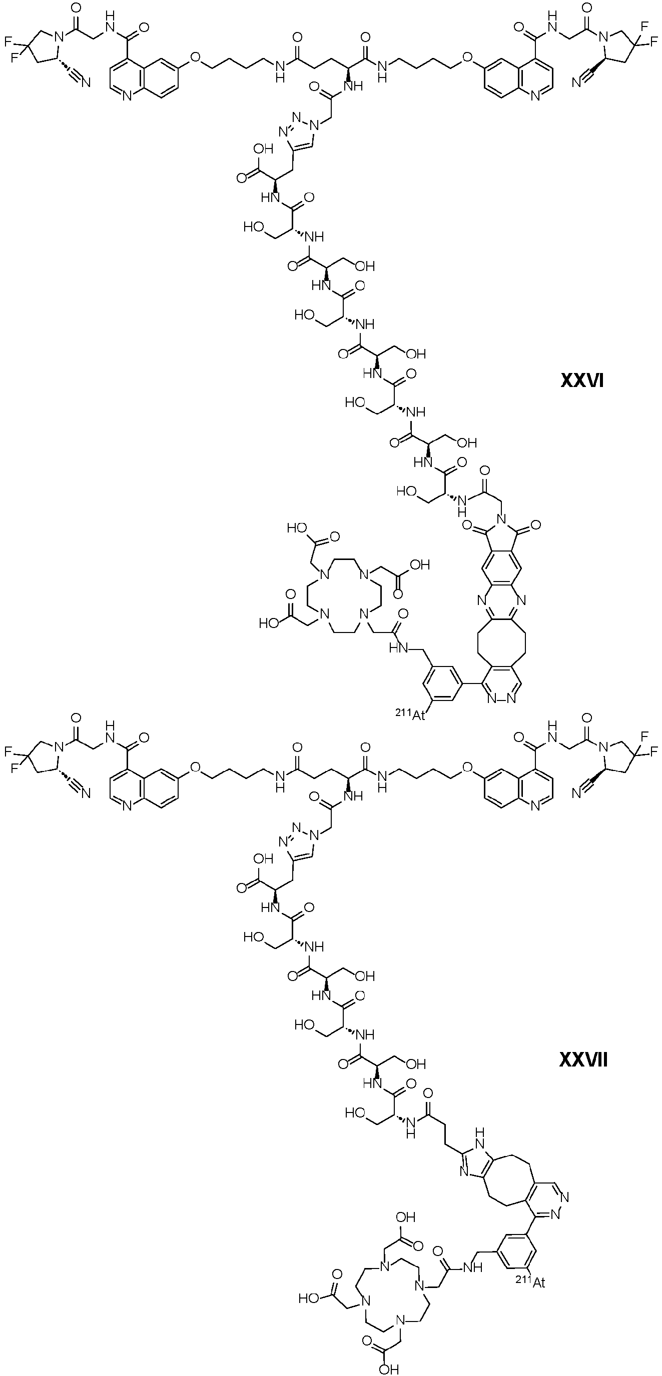

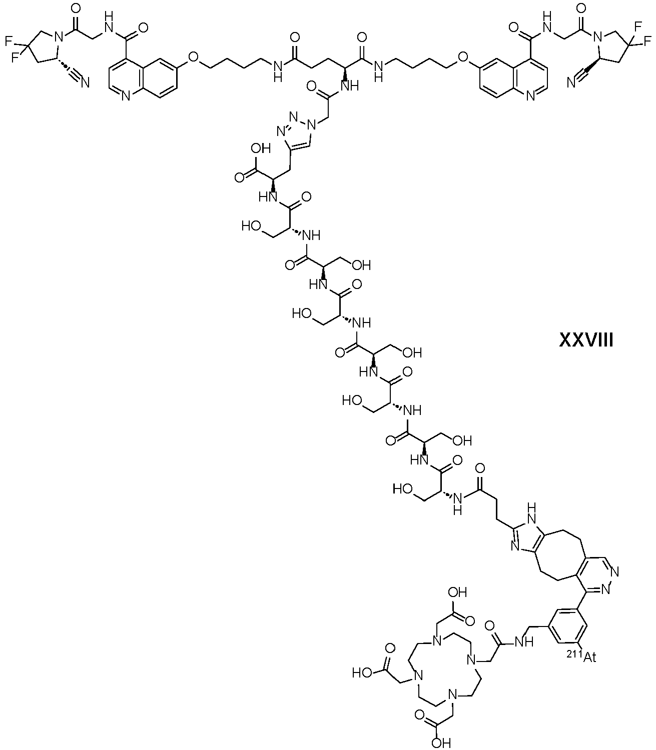

In other embodiments, the FAP inhibitor ligand of formula (I) comprises a metal.

In a preferred embodiment, the FAP inhibitor ligand of formula (I) comprises a radiometal.

Thus, in a preferred embodiment, the FAP inhibitor ligand of formula (I) is a compound the one or two chelator(s) Z is labeled with a radiometal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr,⁹⁴Tc, ^{99m}Tc, ^{103m}Rh, ^{193m}Pt, ^{195m}Pt, ¹⁹⁸Pt, ¹¹¹In, ¹¹³In, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th.

In a more preferred embodiment, the FAP inhibitor ligand of formula (I) is a compound wherein the chelator Z is labeled with a radiometal selected from ⁶⁴Cu, ⁶⁷Cu, ⁶⁸Ga, ⁹⁰Y, ⁸⁹Zr, ^{99m}Tc, ¹¹¹In, ¹⁷⁷Lu, ²⁰³Pb, ²¹²Pb, ²²⁵Ac.

These radiometals are of particular interest because of their superior nuclear properties, making them highly effective for a range of diagnostic and therapeutic applications.

For diagnostic imaging, ⁶⁸Ga and ⁸⁹Zr are particularly valuable. ⁶⁸Ga, a positron emitter with a half-life of approximately 68 minutes, is used in positron emission tomography (PET) imaging. Its availability from a generator system makes it convenient for use in clinical settings, providing high-resolution images for the diagnosis of various conditions. ⁸⁹Zr, with a longer half-life of around 78.4 hours, is suitable for immuno-PET imaging, enabling the tracking of labeled antibodies over several days to study disease progression.

^{99m}Tc is a cornerstone in diagnostic nuclear medicine due to its ideal physical properties, including a half-life of approximately 6 hours and gamma emissions that are optimal for imaging. It is widely used in single-photon emission computed tomography (SPECT) for a variety of diagnostic procedures due to its versatility and excellent imaging characteristics.

For therapeutic applications, ⁹⁰Y and ¹⁷⁷Lu are beta-emitting radionuclides commonly used in targeted radionuclide therapy. ⁹⁰Y has a half-life of approximately 64 hours and is known for its high-energy beta particles, making it effective in treating larger tumors. ¹⁷⁷Lu, with a half-life of about 6.7 days, offers both beta and gamma emissions, allowing for therapeutic treatment and post-therapy imaging. Its relatively long half-life allows for extended radiation delivery, which is beneficial in treating smaller or residual tumor tissues.

⁶⁴Cu and ⁶⁷Cu provide both diagnostic and therapeutic capabilities. ⁶⁴Cu, with a half-life of about 12.7 hours, emits positrons for PET imaging and beta particles for therapy, making it a dual-purpose radionuclide. ⁶⁷Cu, with a half-life of approximately 61.9 hours, emits beta particles and gamma rays, providing both therapeutic and diagnostic capabilities.

¹¹¹In is used for diagnostic imaging and radiotherapy, with a half-life of around 2.8 days and gamma emissions that are suitable for SPECT imaging. It is particularly useful in radiolabeling antibodies and peptides, providing valuable information on disease localization and progression.

The alpha-emitting radionuclides ²¹²Pb and ²²⁵Ac offer potent therapeutic effects due to their high linear energy transfer (LET). ²¹²Pb, with a half-life of about 10.6 hours, decays to ²¹²Bi, providing alpha and beta emissions that are highly effective for targeted therapy. ²²⁵Ac, with a longer half-life of approximately 10 days, emits multiple alpha particles, making it exceptionally potent for targeted cancer therapy, especially in cases where conventional therapies may fail.

²⁰³Pb serves as a diagnostic counterpart to the therapeutic ²¹²Pb, allowing for paired imaging and therapy applications. Its half-life of around 51.9 hours and gamma emissions are suitable for imaging, complementing the therapeutic effects of ²¹²Pb.

Thus, these radionuclides maximizes the versatility and applicability of the FAP compounds.

For all embodiments wherein the FAP inhibitor ligand of formula (I) comprises a radiometal, no radiohalogen should be included in the compound. Accordingly, when the FAP inhibitor ligand of formula (I) is a compound wherein the chelator Z is labeled with a radiometal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁴Tc, ^{99m}Tc, ^{103m}Rh, ^{193m}Pt, ^{195m}Pt, ¹⁹⁸Pt, ¹¹¹In, ¹¹³In, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th; R₄ is selected from -H, -Y₁, -OCH₂CH₂Y₁, -OCH₂CH₂ CH₂Y₁, -SCH₂CH₂Y₁, -NHCH₂CH₂Y₁, -OCH₂C₆H₄Y₁, -OCH₂CH₂C₆H₄Y₁, -NHCOCH₂CH₂Y₁, -CONHCH₂CH₂Y₁, - NHSO₂CH₂CH₂Y₁, -SO₂NHCH₂CH₂Y₁, wherein Y₁ is selected from the group constisting of: ¹H, ²H, ¹¹C, ¹²C, ¹³C, ¹⁹F, ⁷⁹Br, ⁸¹Br, ¹²⁷I.

In a preferred embodiment, the FAP inhibitor ligand of formula (I) is selected from the group consisting of:

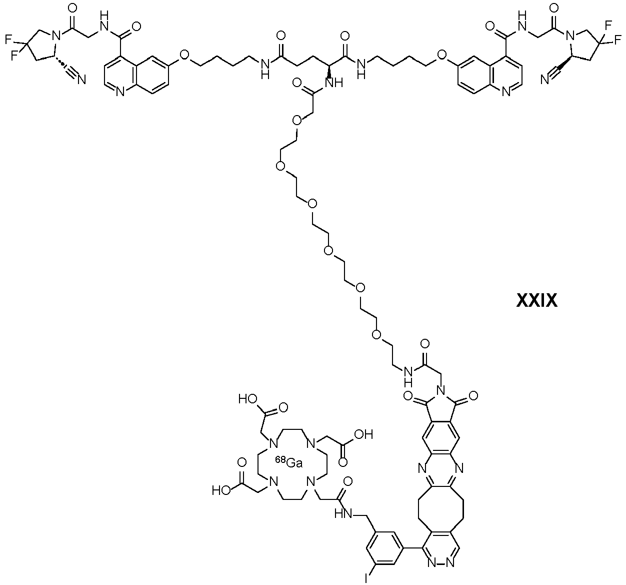

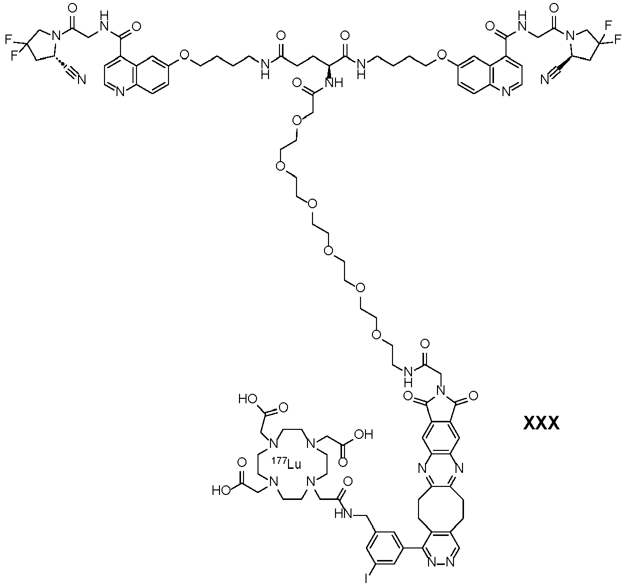

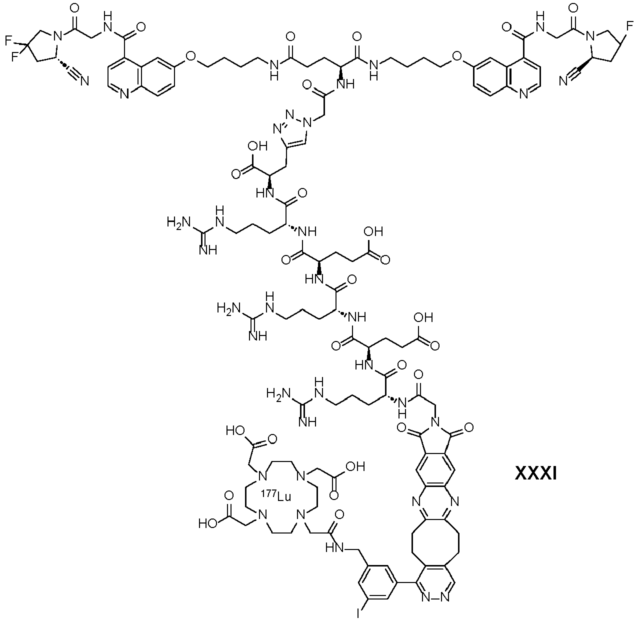

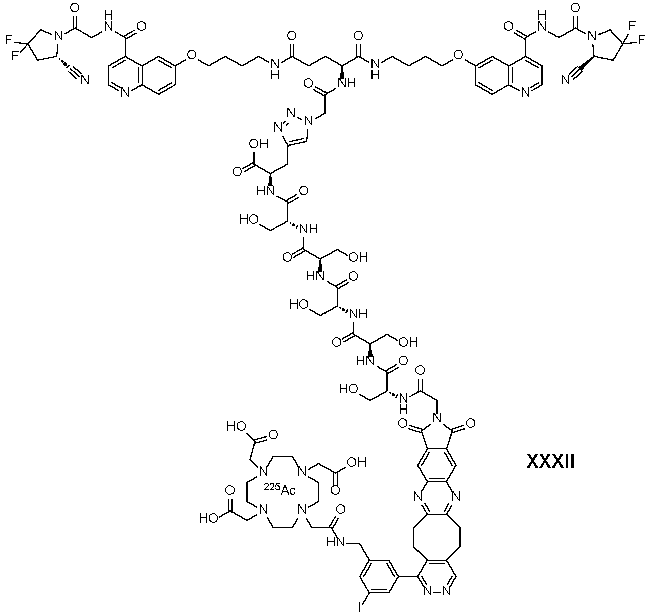

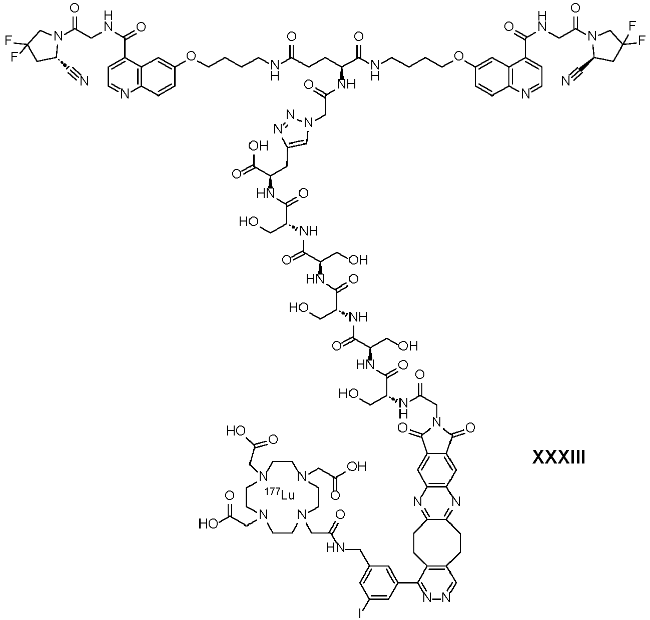

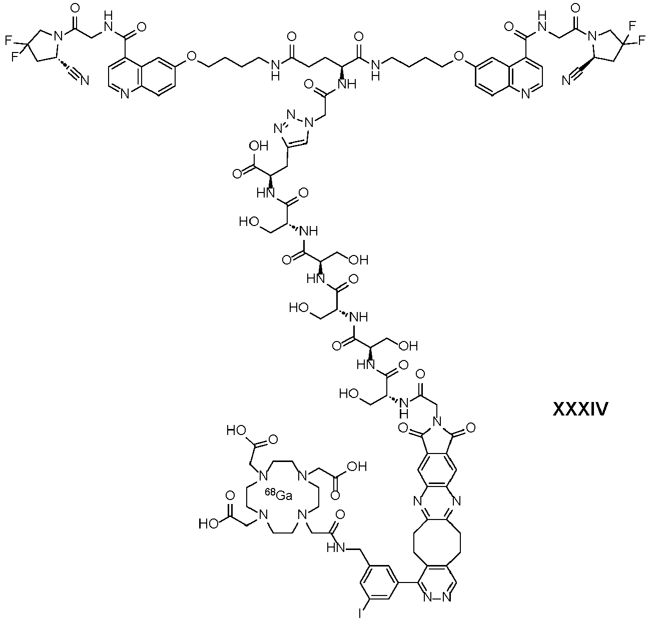

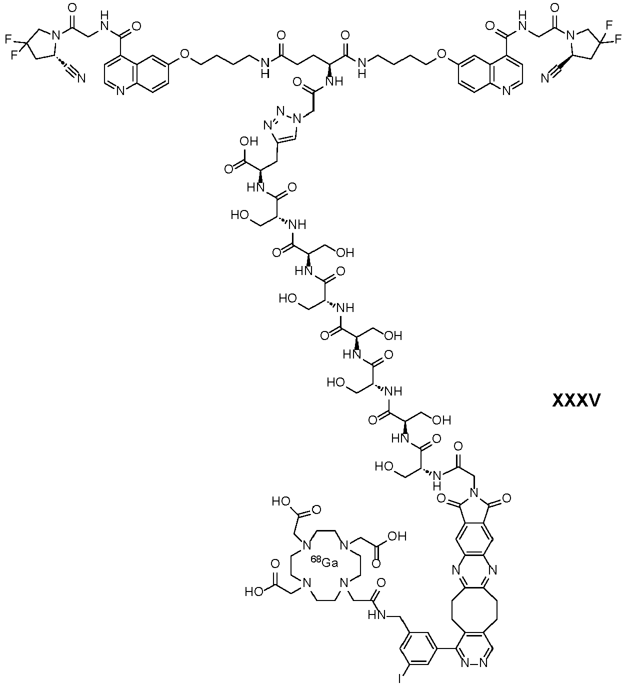

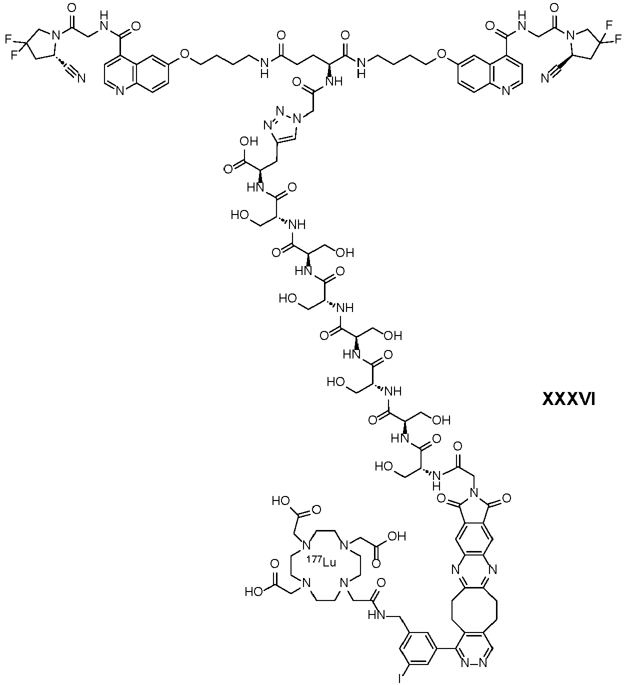

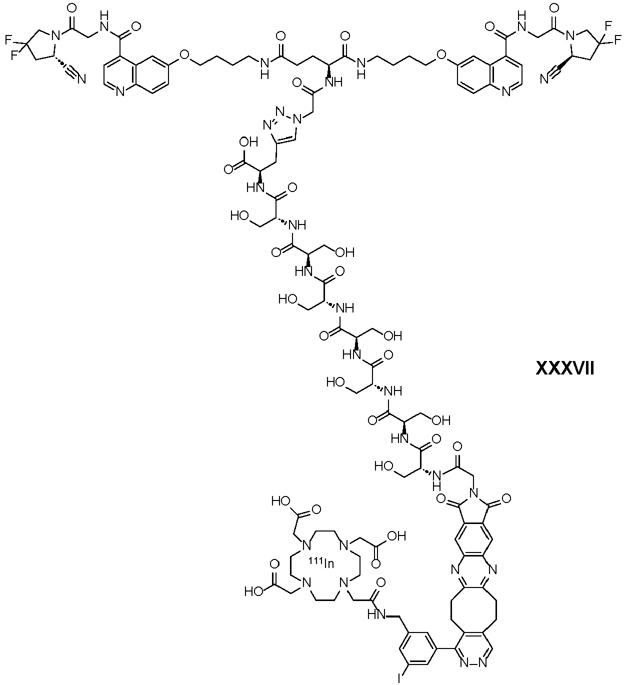

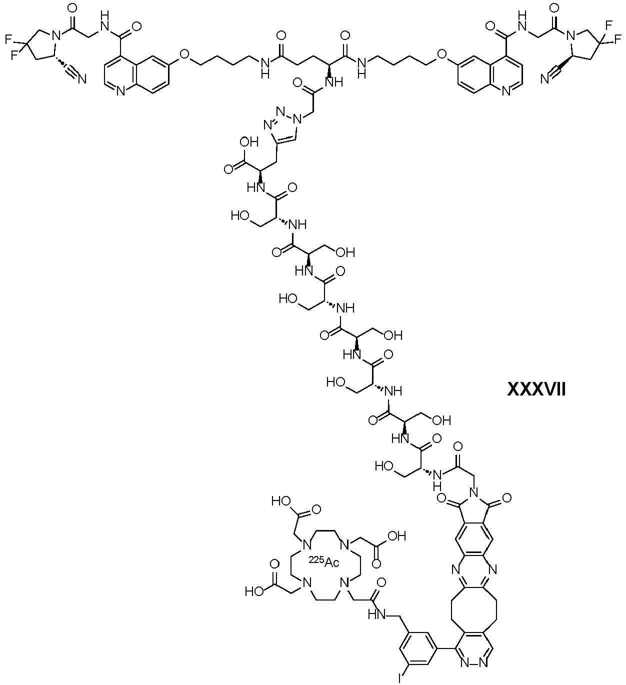

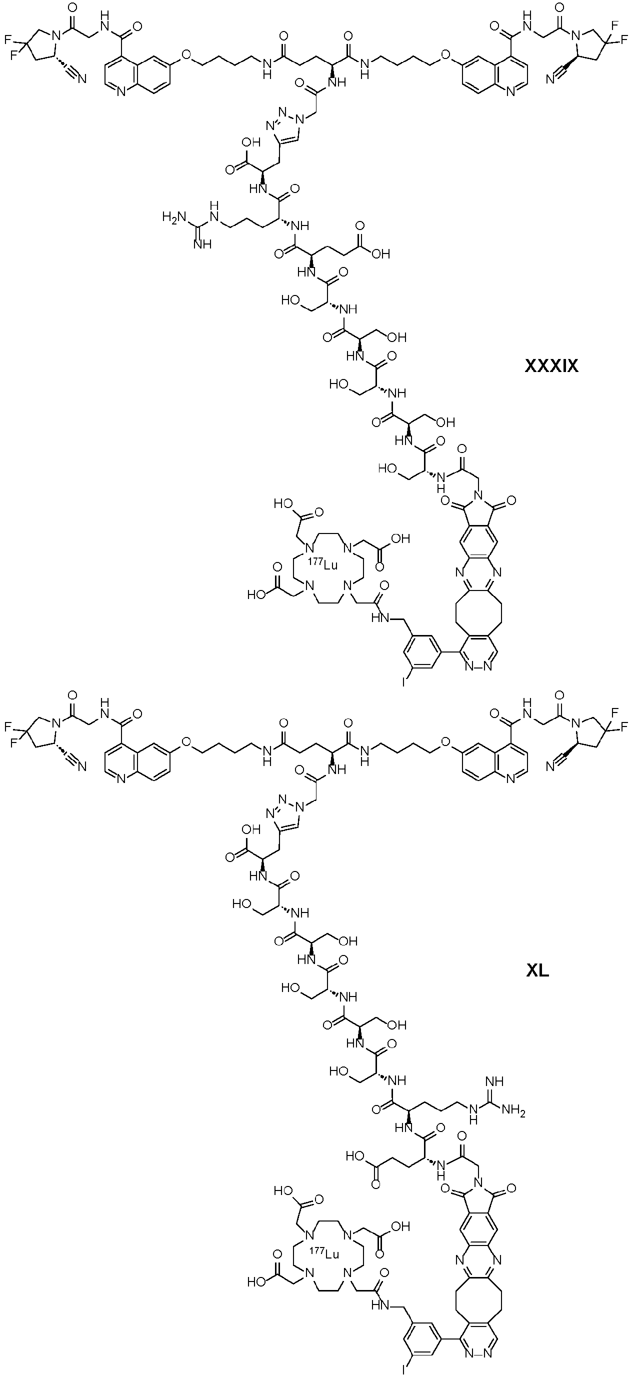

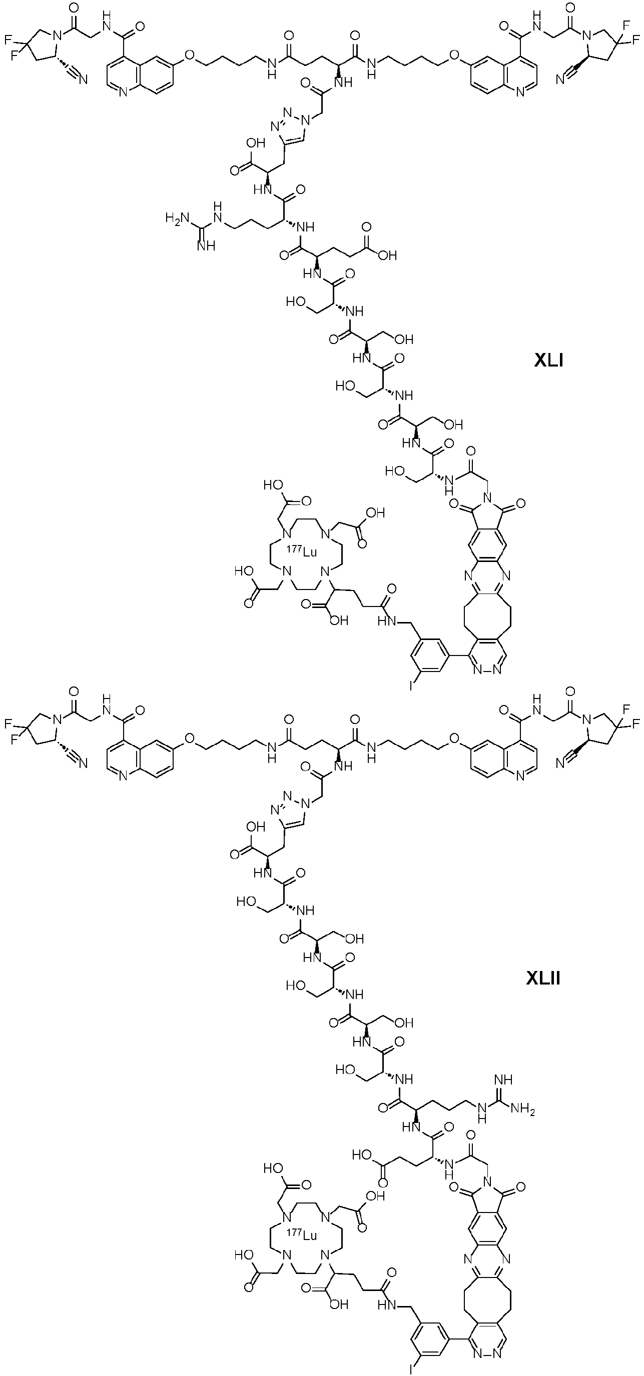

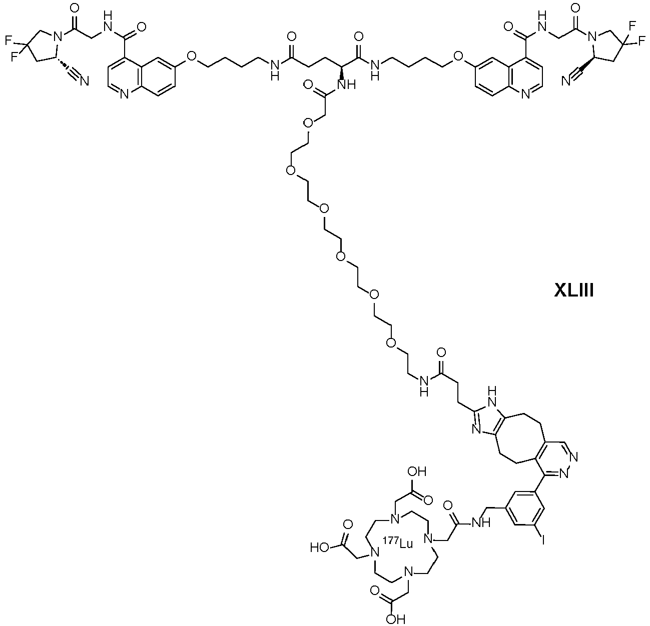

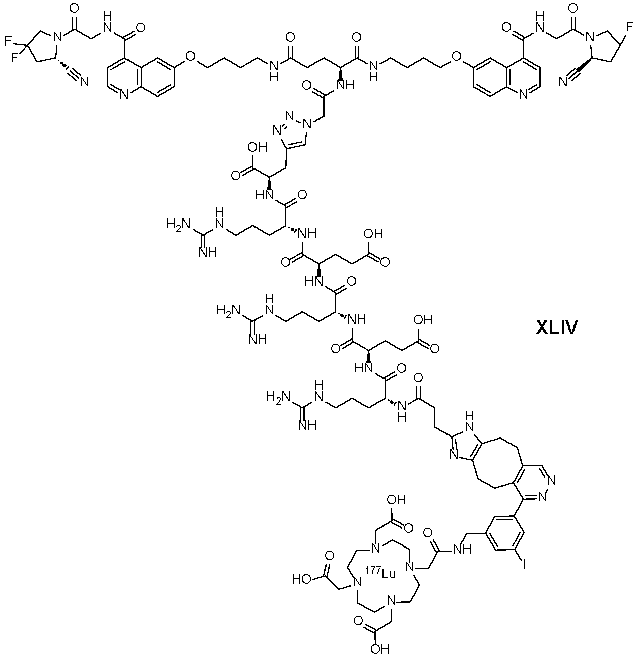

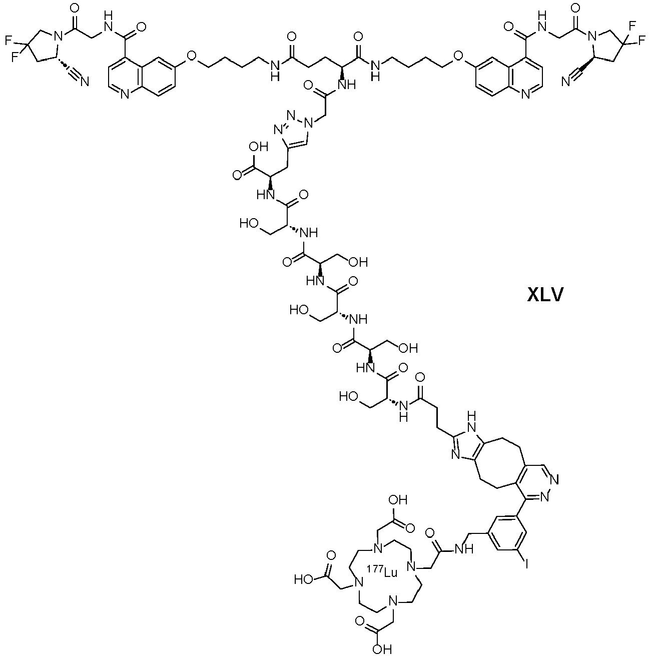

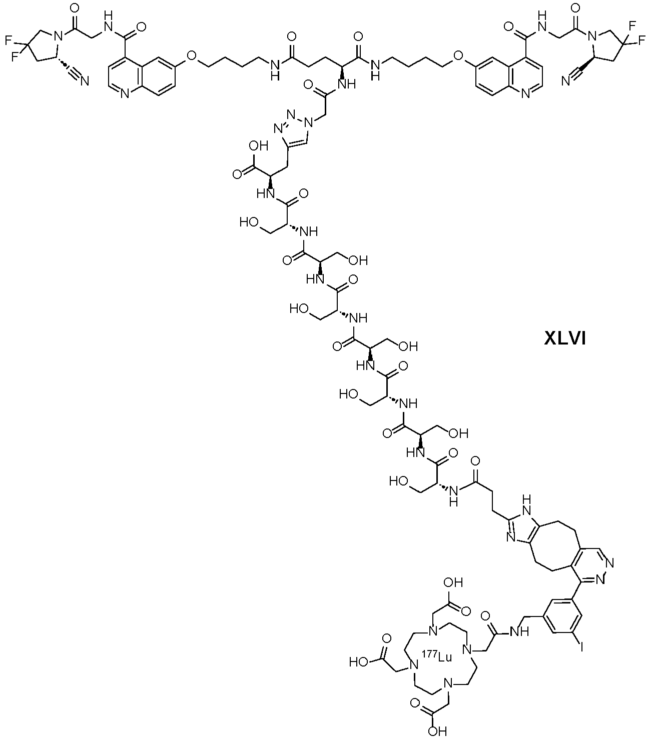

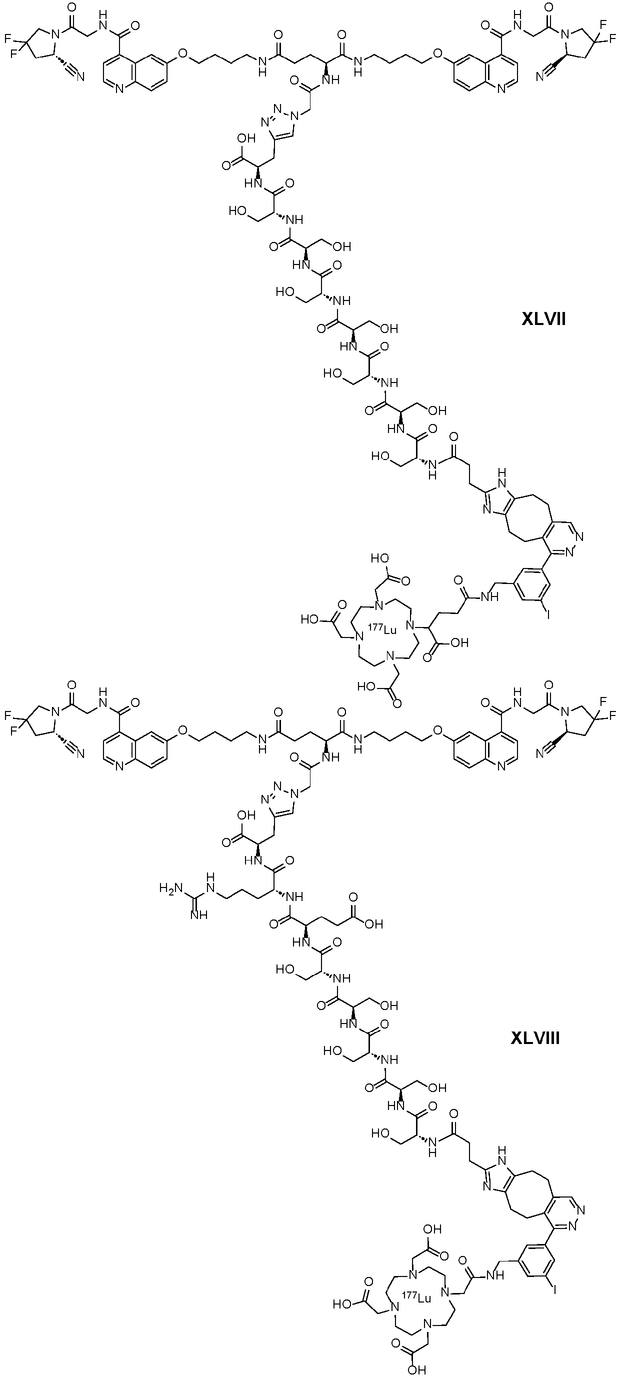

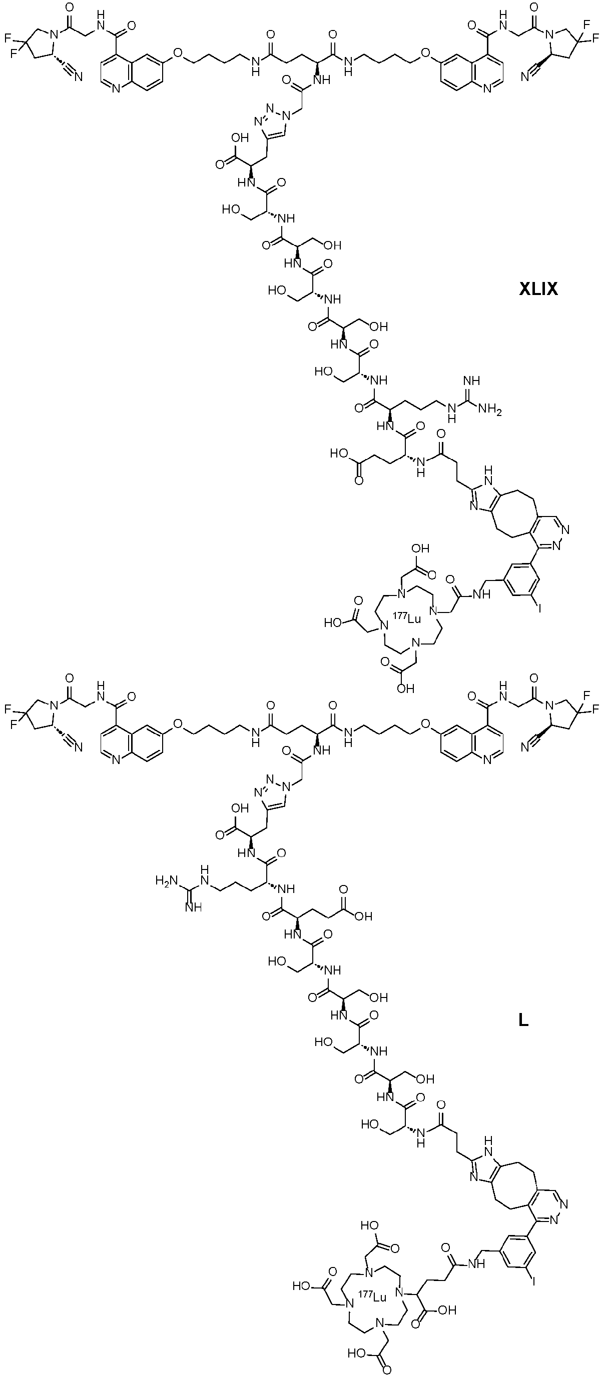

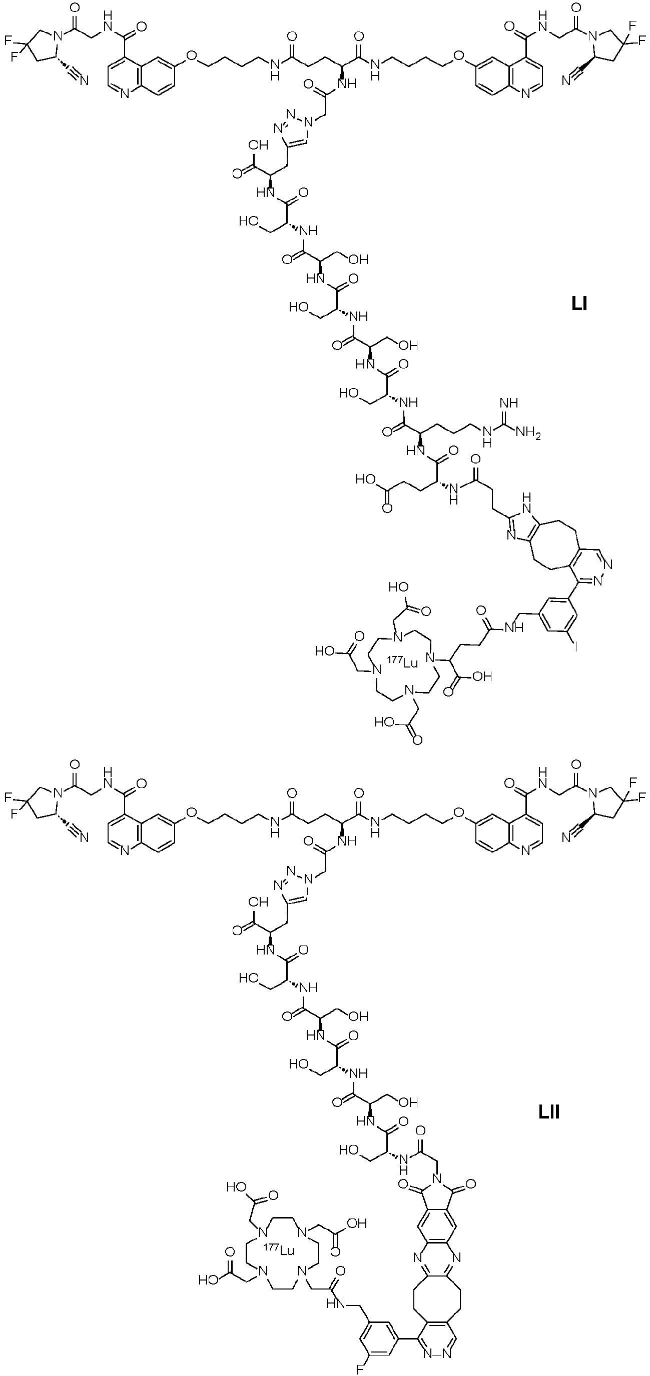

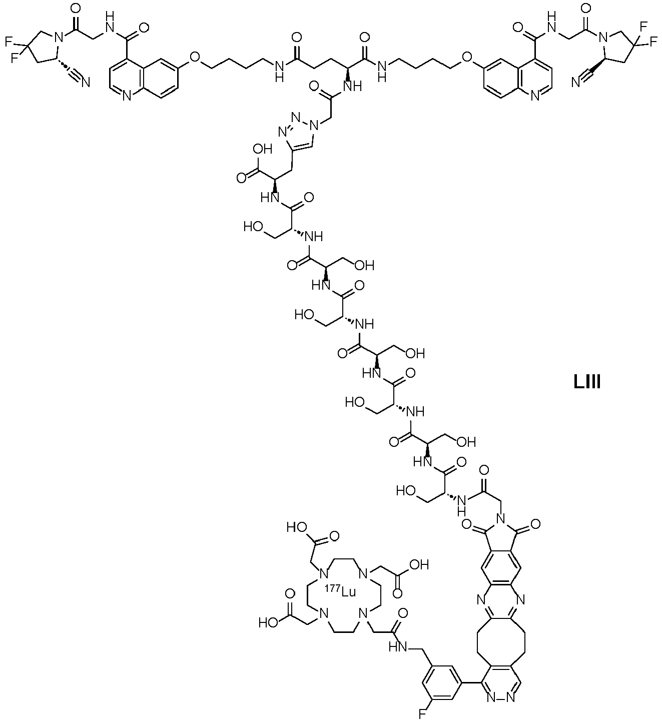

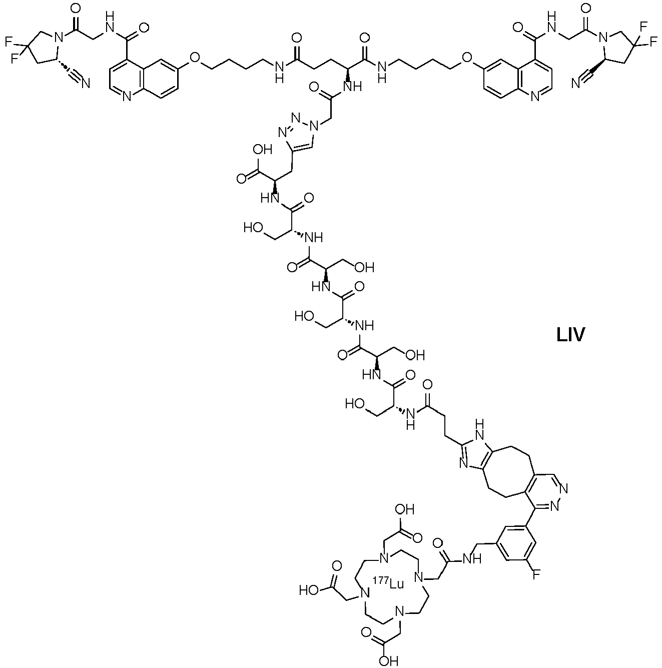

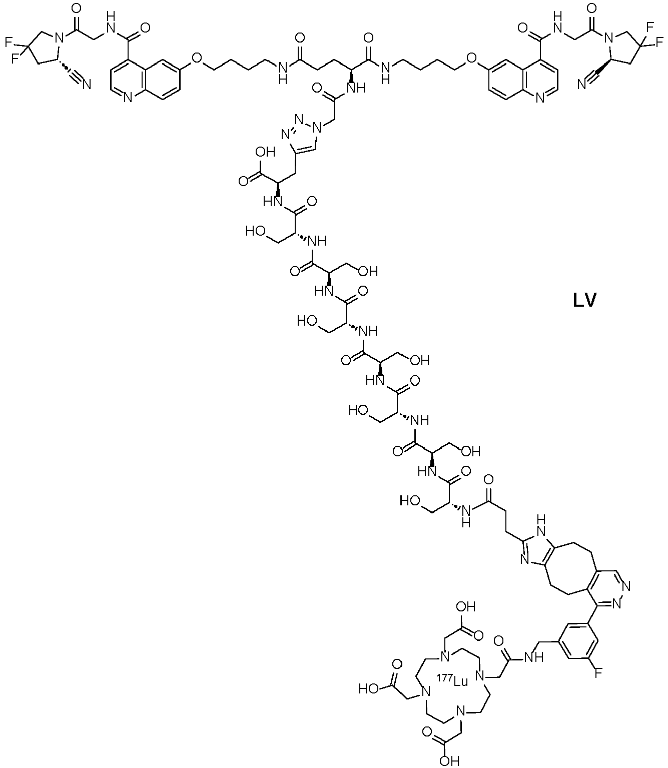

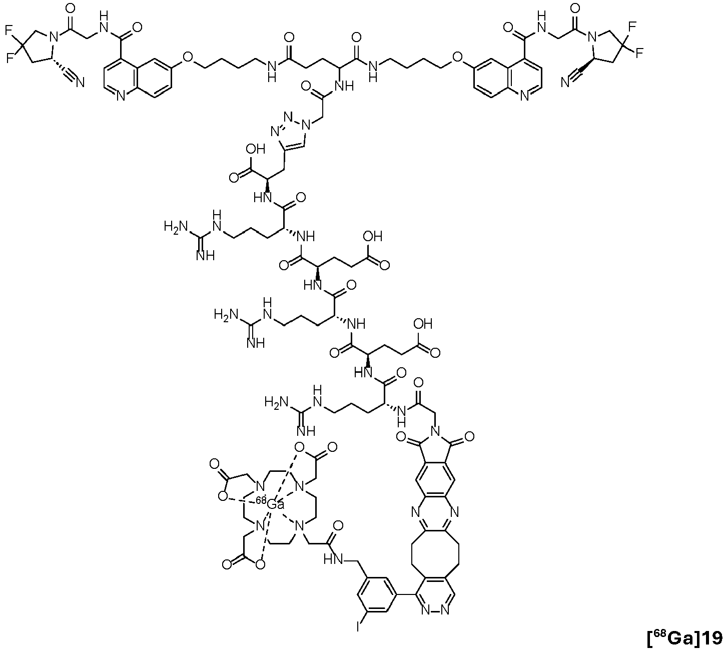

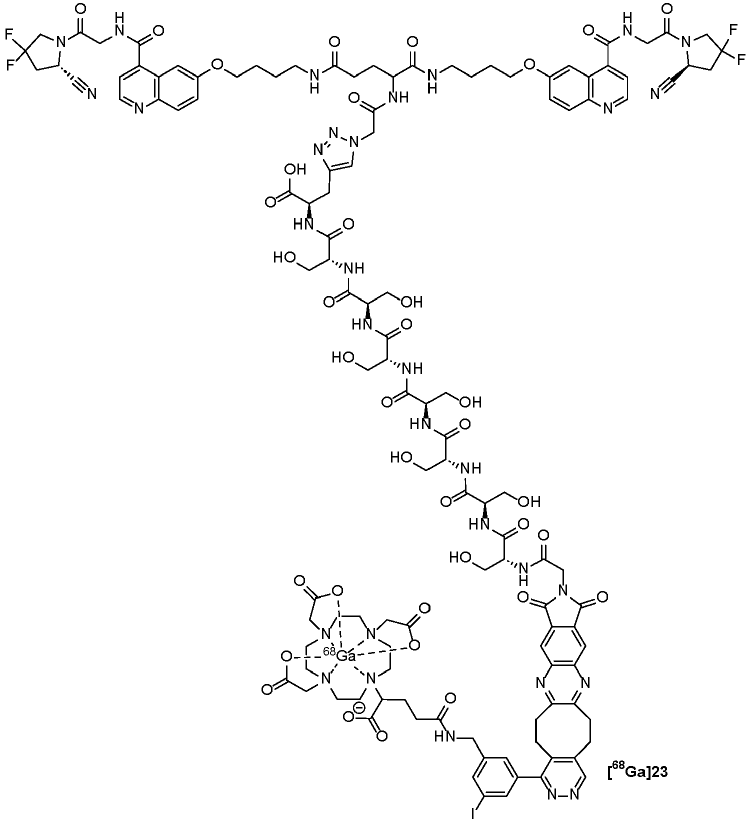

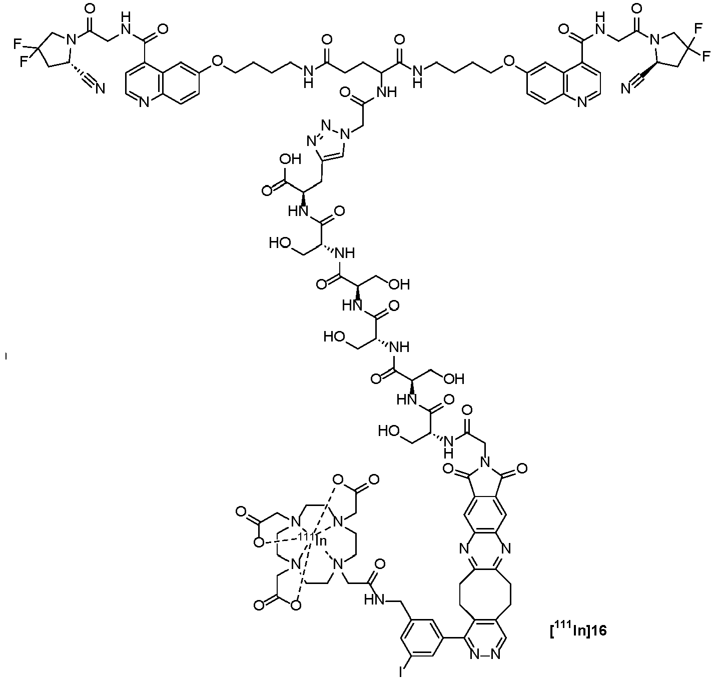

In a second aspect, the present invention relates to a pharmaceutical formulation comprising FAP inhibitor ligand of formula (I): 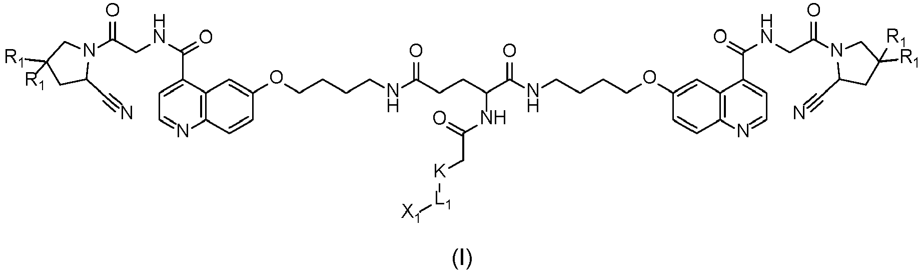 wherein:
R₁ is H or F
K is 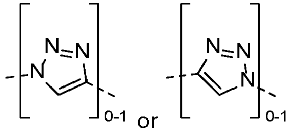
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, - COCH(CH(OH)CH₃)NH-, -COCH(CH₂SeH)NH-, -COCH(CH₂C₈H₆N)NH-, - COCH(CH₂C₆H₄OH)NH-, -COCH(CH₂CH₂SeCH₃)NH-;
X₁ is a pyridazine selected from the group consisting of: 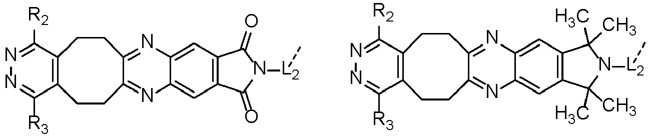 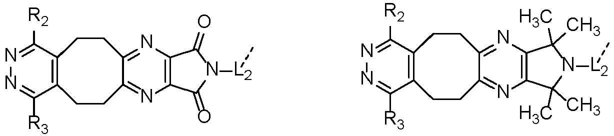 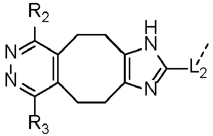
   wherein L₂ is -CO(CH₂)ₙ- or -CO(CH₂CH₂O)ₙ(CH₂)ₘ-,
   wherein n and m are integers independently selected from 1-10, and
R₂ is selected from -H, -Me, and R₃,
R₃ is 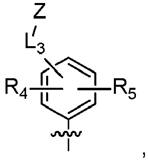 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, -NHCH₂CH₂Y₁, - OCH₂C₆H₄Y₁, -OCH₂CH₂C₆H₄Y₁, -NHCOCH₂CH₂Y₁, -CONHCH₂CH₂Y₁, -NHSO₂CH₂CH₂Y₁, - SO₂NHCH₂CH₂Y₁,
   wherein Y₁ is selected from the group constisting of:
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At, ²¹¹At,
   and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, - NHSO₂(CH₂)ₙ(OCH₂CH₂)ₘNH-, -CONH(CH₂)ₙ(OCH₂CH₂)ₘNH-, - NHCO(CH₂)ₙ(OCH₂CH₂)ₘNH-, -CH₂(OCH₂CH₂)(CH₂)ₘNH-, - OCH₂(OCH₂CH₂)ₙ(CH₂)ₘNH-, -SCH₂(OCH₂CH₂)ₙ(CH₂)ₘNH-, - SO₂NHCH₂(OCH₂CH₂)ₙ(CH₂)ₘNH-, -NHSO₂CH₂(OCH₂CH₂)ₙ(CH₂)ₘNH-, - CONHCH₂(OCH₂CH₂)ₙ(CH₂)ₘNH-, -NHCOCH₂CH₂(OCH₂CH₂)ₙ(CH₂)ₘNH-, - CH₂CH₂(OCH₂CH₂)(CH₂)ₘNH-, -OCH₂CH₂(OCH₂CH₂)ₙ(CH₂)ₘNH-, - SCH₂CH₂(OCH₂CH₂)ₙ(CH₂)ₘNH-, -SO₂NHCH₂CH₂(OCH₂CH₂)ₙ(CH₂)ₘNHCO-, - NHSO₂CH₂CH₂(OCH₂CH₂)ₙ(CH₂)ₘNHCO-, -CONHCH₂CH₂(OCH₂CH₂)ₙ(CH₂)ₘNHCO-, -NHCOCH₂CH₂(OCH₂CH₂)ₙ(CH₂)ₘNHCO-,
      where n and m are independently selected from the group consisting of 0-25;
   and wherein Z is a chelator selected from: -H, 1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N'*-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
   and wherein Z is optionally labeled with a metal selected from:
      ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
   and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt, - SO₂NHPr, -NH₂, -NHMe, -NHEt, -NHPr;
or pharmaceutically acceptable salts thereof.

The formulation is preferably an intravenous formulation and could be formulated in accordance with other radiopharmaceutical formulations in the art. Such formulations may typically comprise buffering agents, stabilizers, chelating agents, solvents, isotonicity adjusting agents.

The FAP inhibitor ligand of formula (I) are particularly useful in therapy, imaging such as diagnosis and theranostics when the compound comprises at least one radionuclide, such as a radiohalogen or a radiometal.

In a third aspect, the present invention accordingly relates to FAP inhibitor ligand of formula (I): 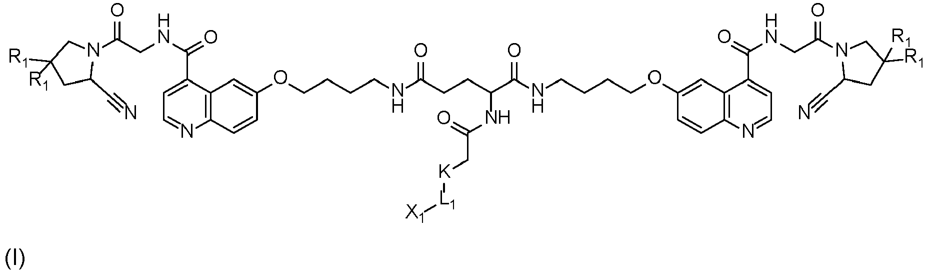 wherein:
R₁ is H or F
K is 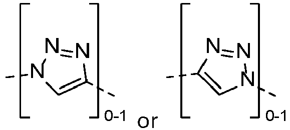
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-,
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, - COCH(CH(OH)CH₃)NH-, -COCH(CH₂SeH)NH-, -COCH(CH₂C₈H₆N)NH-, - COCH(CH₂C₆H₄OH)NH-; -COCH(CH₂CH₂SeCH₃)NH-,
X₁ is a pyridazine selected from the group consisting of: 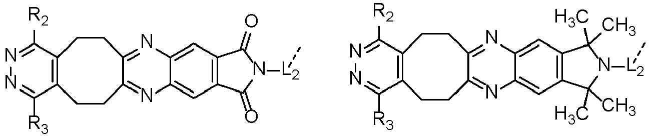 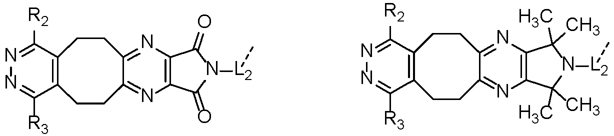 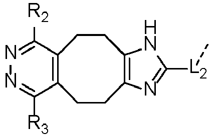
   wherein L₂ is -CO(CH₂)ₙ- or -CO(CH₂CH₂O)ₙ(CH₂)ₘ-,
   wherein n and m are integers independently selected from 1-10,
   and R₂ is selected from -H, -Me, and R₃,
   R₃ is 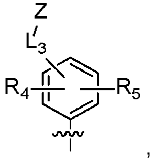 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, - NHCH₂CH₂Y₁, -OCH₂C₆H₄Y₁, -OCH₂CH₂C₆H₄Y₁, -NHCOCH₂CH₂Y₁, - CONHCH₂CH₂Y₁, -NHSO₂CH₂CH₂Y₁, -SO₂NHCH₂CH₂Y₁,
   wherein Y₁ is selected from the group constisting of;
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At, ²¹¹At,
   and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, - NHSO₂(CH₂)ₙ(OCH₂CH₂)ₘNH-, -CONH(CH₂)ₙ(OCH₂CH₂)ₘNH-, - NHCO(CH₂)ₙ(OCH₂CH₂)ₘNH-, -CH₂(OCH₂CH₂)(CH₂)ₘNH-, - OCH₂(OCH₂CH₂)ₙ(CH₂)ₘNH-, -SCH₂(OCH₂CH₂)ₙ(CH₂)ₘNH-, - SO₂NHCH₂(OCH₂CH₂)ₙ(CH₂)ₘNH-, -NHSO₂CH₂(OCH₂CH₂)ₙ(CH₂)ₘNH-, - CONHCH₂(OCH₂CH₂)ₙ(CH₂)ₘNH-, -NHCOCH₂CH₂(OCH₂CH₂)ₙ(CH₂)ₘNH-,-CH₂CH₂(OCH₂CH₂)(CH₂)ₘNH-, -OCH₂CH₂(OCH₂CH₂)ₙ(CH₂)ₘNH-, - SCH₂CH₂(OCH₂CH₂)ₙ(CH₂)ₘNH-, -SO₂NHCH₂CH₂(OCH₂CH₂)ₙ(CH₂)ₘNHCO-, - NHSO₂CH₂CH₂(OCH₂CH₂)ₙ(CH₂)ₘNHCO-, -CONHCH₂CH₂(OCH₂CH₂)ₙ(CH₂)ₘNHCO-, -NHCOCH₂CH₂(OCH₂CH₂)ₙ(CH₂)ₘNHCO-,
   where n and m are independently selected from the group consisting of 0-25;
   and wherein Z is a chelator selected from: -H, 1,4,7,10-tetraazacyclododecane-*N*,*N'*,*N'*,*N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N*,*N'*-bis(2-hydroxy-5-(carboxyethyl)benzyl)ethylenediamine *N*,*N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9-triacetic acid (PCTA), *N*'-(5-acetyl (hydroxy)aminopentyl-*N*-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
   and wherein Z is optionally labeled with a metal selected from:
      ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr,⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
   and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt, - SO₂NHPr, -NH₂, -NHMe, -NHEt, -NHPr; and
wherein said FAP inhibitor ligand of formula (I) comprises at least one radionuclide; and pharmaceutically acceptable salts thereof, for use as a medicament in therapy, imaging, diagnostics, or theranostics.

Together with the benefits of the FAP inhibitor ligand of formula (I) in relation to the advantageous pharmacokinetic properties and high tumor accumulation, the fact that the FAP inhibitor ligand of formula (I) is targeting fibroblast activation protein (FAP), which is overexpressed in various cancer-associated fibroblasts (CAFs) and other diseases such as Fibrotic diseases, arthritis, wound healing and inflammatory diseases makes the FAP inhibitor ligand of formula (I) makes it a highly relevant candidate for use in the treatment of and/or diagnosis of such diseases, particularly treatment and/ or in the diagnosis of FAP expressing diseases including cancer, such as breast cancer, lung cancer, colorectal cancer, prostate cancer, ovarian cancer, pancreatic cancer, hepatocellular cancers, sarcomas and non-small cell lung cancer; and fibrosis, arthritis, atherosclerosis, and inflammatory diseases like spondyloarthritis.

Thus, in a fourth aspect, the present invention relates to FAP inhibitor ligand of formula I: 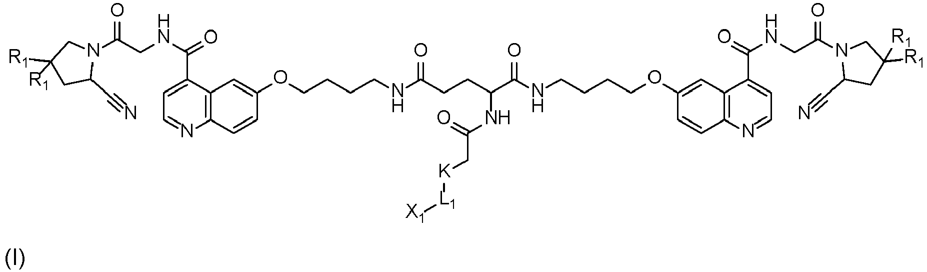 wherein:
R₁ is H or F
K is 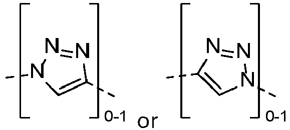
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
   wherein n and m are integers independently selected from 0-20,
   and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, -COCH(CH(OH)CH₃)NH-, - COCH(CH₂SeH)NH-, -COCH(CH₂C₈H₆N)NH-, -COCH(CH₂C₆H₄OH)NH-; - COCH(CH₂CH₂SeCH₃)NH-,
X₁ is a pyridazine selected from the group consisting of: 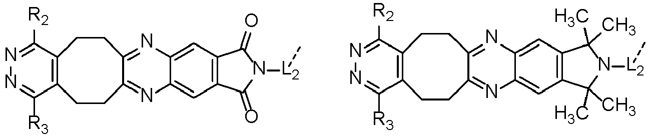 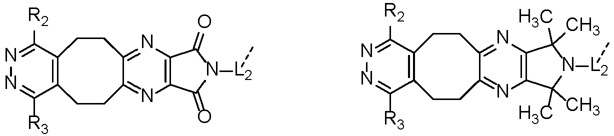 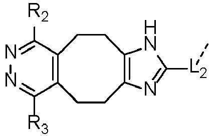
   wherein L₂ is -CO(CH₂)ₙ- or -CO(CH₂CH₂O)ₙ(CH₂)ₘ-,
   wherein n and m are integers independently selected from 1-10,
   and R₂ is selected from -H, -Me, and R₃,
   R₃ is 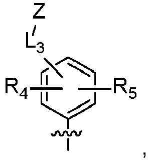 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, - NHCH₂CH₂Y₁, -OCH₂C₆H₄Y₁, -OCH₂CH₂C₆H₄Y₁, -NHCOCH₂CH₂Y₁, - CONHCH₂CH₂Y₁, -NHSO₂CH₂CH₂Y₁, or -SO₂NHCH₂CH₂Y₁,
   wherein Y₁ is selected from the group constisting of;
      ¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁵I, ¹²⁷I, ¹³¹I, ²¹⁰At, ²¹¹At,
and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, -NHSO₂(CH₂)ₙ(OCH₂CH₂)ₘNH-, -CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, -SCH₂(OCH₂CH₂)ₙ(CH₂)ₘNH-, - SO₂NHCH₂(OCH₂CH₂)ₙ(CH₂)ₘNH-, -NHSO₂CH₂(OCH₂CH₂)ₙ(CH₂)ₘNH-, - CONHCH₂(OCH₂CH₂)ₙ(CH₂)ₘNH-, -NHCOCH₂CH₂(OCH₂CH₂)ₙ(CH₂)ₘNH-, - CH₂CH₂(OCH₂CH₂)(CH₂)ₘNH-, -OCH₂CH₂(OCH₂CH₂)ₙ(CH₂)ₘNH-, - SCH₂CH₂(OCH₂CH₂)ₙ(CH₂)ₘNH-, -SO₂NHCH₂CH₂(OCH₂CH₂)ₙ(CH₂)ₘNHCO-, - NHSO₂CH₂CH₂(OCH₂CH₂)ₙ(CH₂)ₘNHCO-, -CONHCH₂CH₂(OCH₂CH₂)ₙ(CH₂)ₘNHCO-, - NHCOCH₂CH₂(OCH₂CH₂)ₙ(CH₂)ₘNHCO-,
   where n and m are independently selected from the group consisting of 0-25;
and wherein Z is a chelator selected from: -H, 1,4,7,10-tetraazacyclododecane-*N*,*N'*,*N'*,*N"-*tetraacetic acid (DOTA), 2-(4,7,10-tris(2-(*tert*-butoxy)-2-oxoethyl)-1,4,7,10-tetraazacyclododecan-1-yl)acetic acid (*t*Bu-DOTA), *N*,*N'*-bis(2-hydroxy-5-(carboxyethyl)benzyl)ethylenediamine *N*,*N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
   and wherein Z is optionally labeled with a metal selected from:
   ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th; and wherein R₅ is selected from a -H, a halogen, -OH, -OMe, -OEt, -OPr, -NHCOH, -NHCOMe, NHCOEt, -NHCOPr, -CONH₂, -CONHMe, -CONHEt, -CONHPr, NHSOH, -NHSO₂Me, NHSO₂Et, -NHSO₂Pr, -SO₂NH₂, -SO₂NHMe, -SO₂NHEt, - SO₂NHPr, -NH₂, -NHMe, -NHEt, -NHPr;
and wherein said FAP inhibitor ligand of formula (I) comprises at least one radionuclide; and pharmaceutically acceptable salts thereof for use in the treatment and/ or in the diagnosis of FAP expressing diseases including cancer, such as breast cancer, lung cancer, colorectal cancer, prostate cancer, ovarian cancer, pancreatic cancer, hepatocellular cancers, sarcomas and non-small cell lung cancer; and fibrosis, arthritis, atherosclerosis, and inflammatory diseases like spondyloarthritis.

In a fifth aspect, the present invention relates to a FAP inhibitor precursor of formula (II): 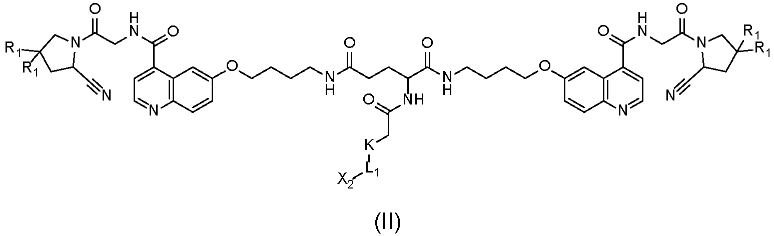 wherein:
R₁ is H or F
K is 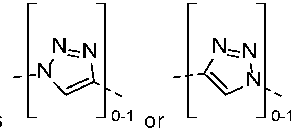
L₁ is selected from:
   -(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
   wherein n and m are integers independently selected from 0-20,
   and A is an amino acid independently selected from the group consisting of; -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, - COCH(CH(OH)CH₃)NH-, -COCH(CH₂SeH)NH-, -COCH(CH₂C₈H₆N)NH-, - COCH(CH₂C₆H₄OH)NH-; -COCH(CH₂CH₂SeCH₃)NH-,
X₂ is a cyclooctene selected from the group consisting of: 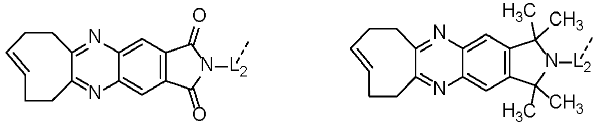 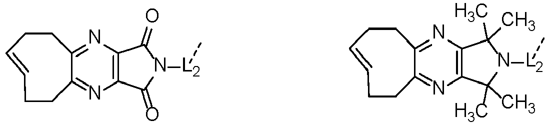 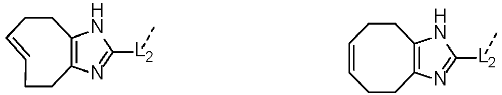 wherein L₂ is -CO(CH₂)ₙ- or -CO(CH₂CH₂O)ₙ(CH₂)ₘ-,
wherein n and m are integers independently selected from 1-10.

These precursors have been found to ligate with a tetrazine in an inverse-electron demand Diels-Alder reaction, to provide the provide the desidered pyridazines in a mild, rapid, chemoselective fashion.

In a preferred embodiment, the FAP inhibitor precursors of formula (II) is selected from the group consisting of **LVI-LXIX:**

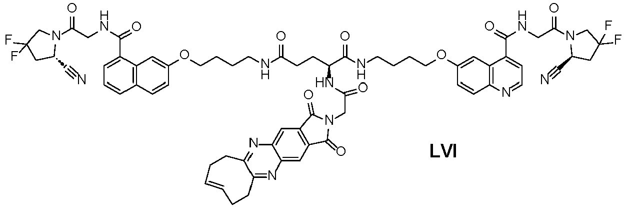

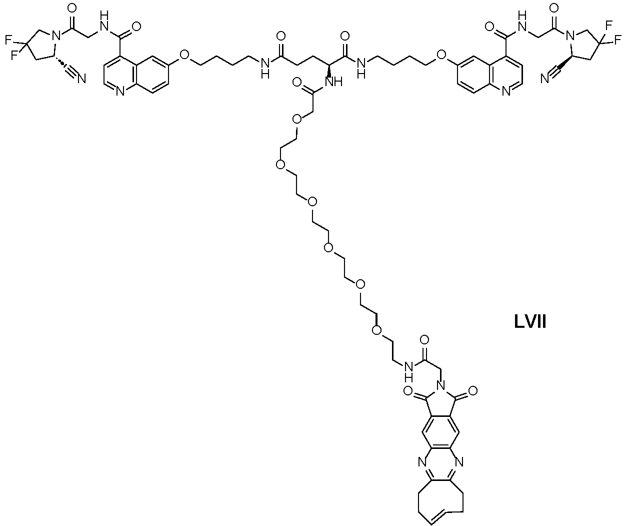

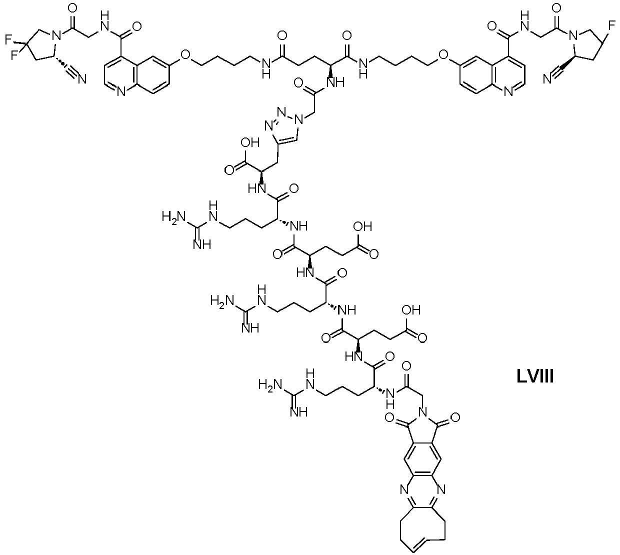

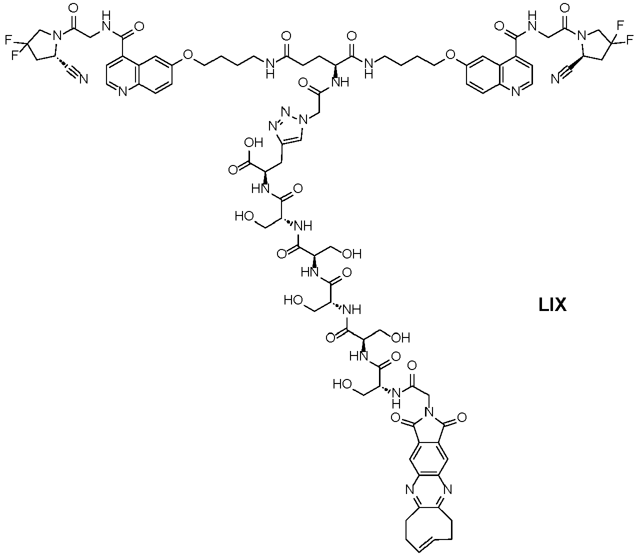

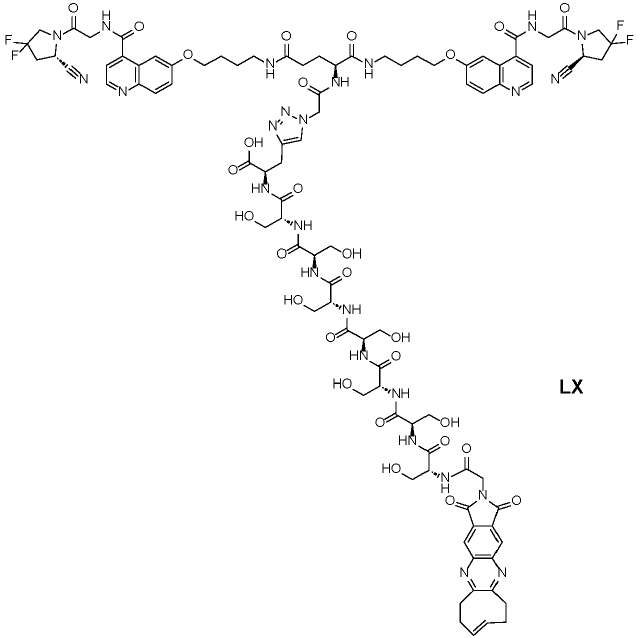

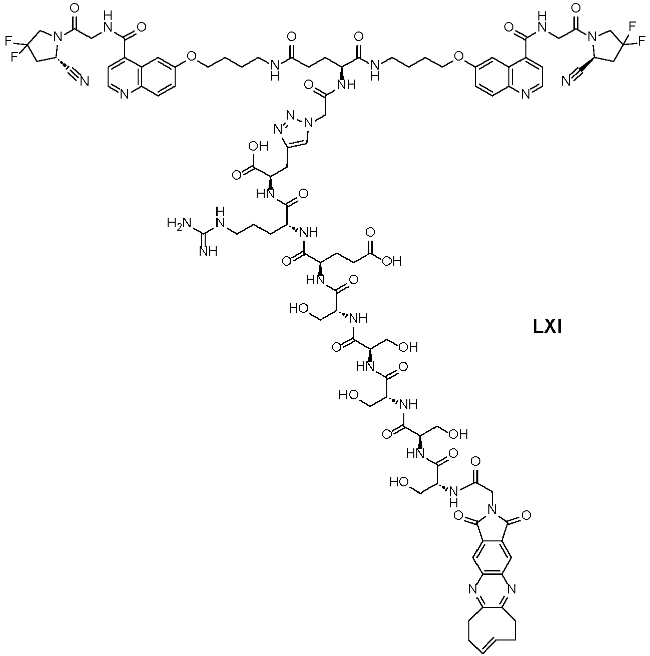

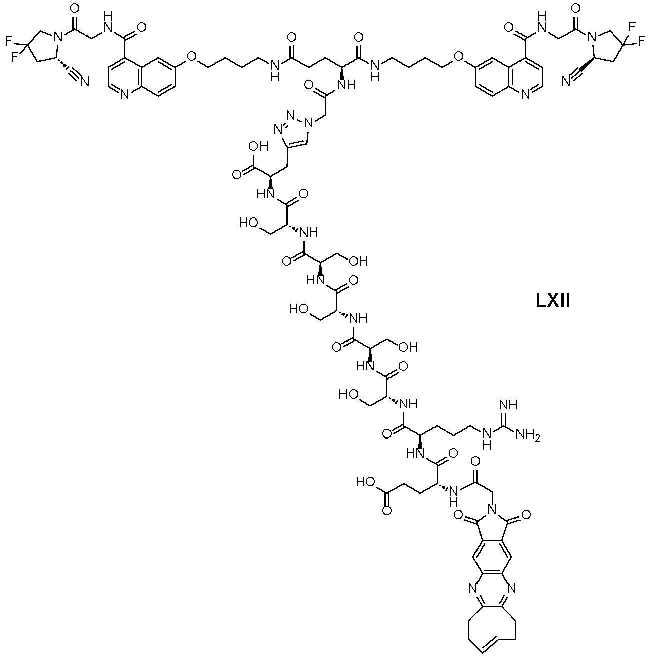

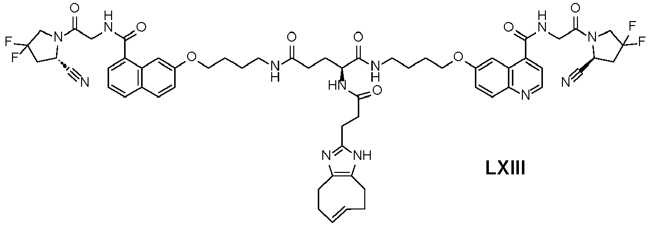

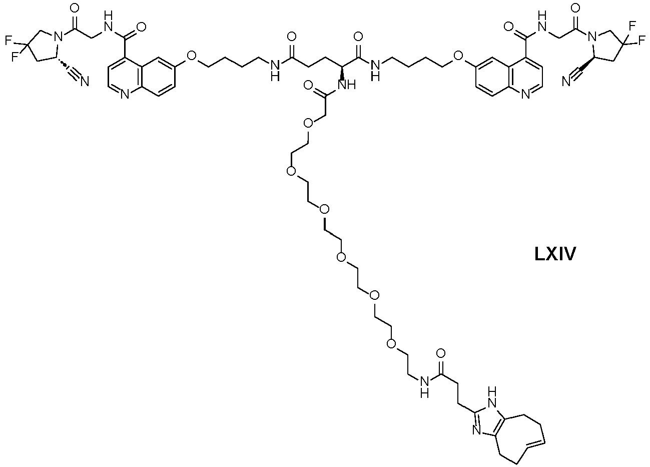

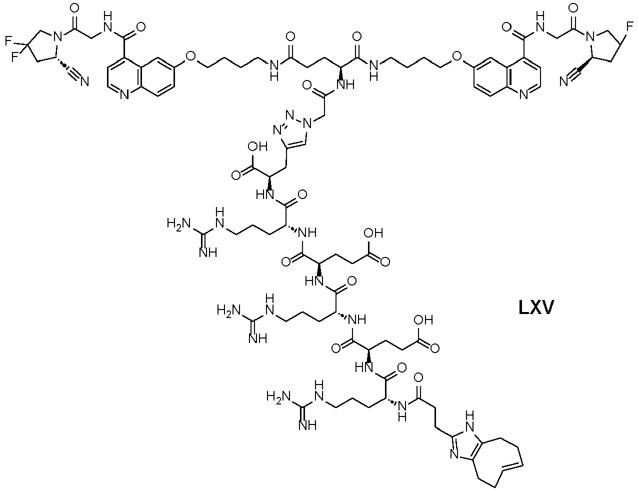

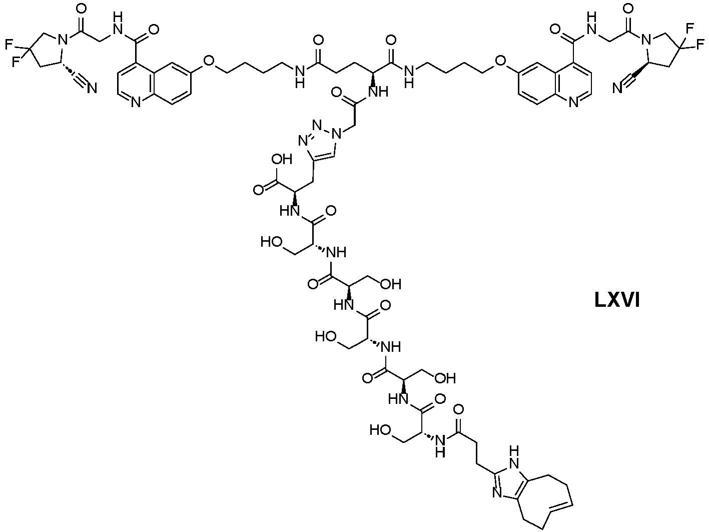

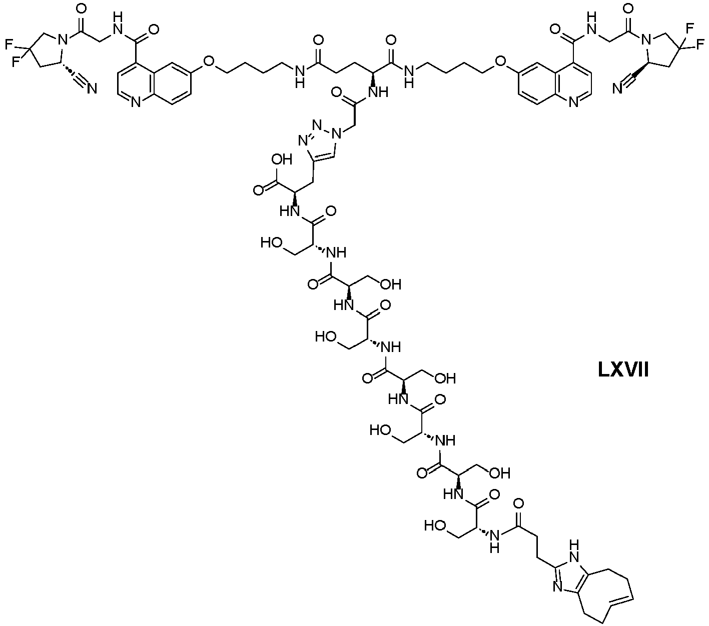

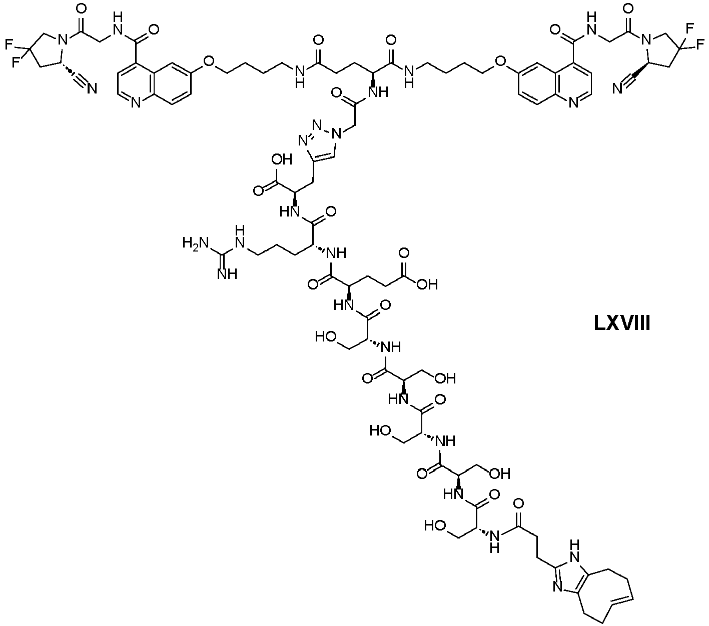

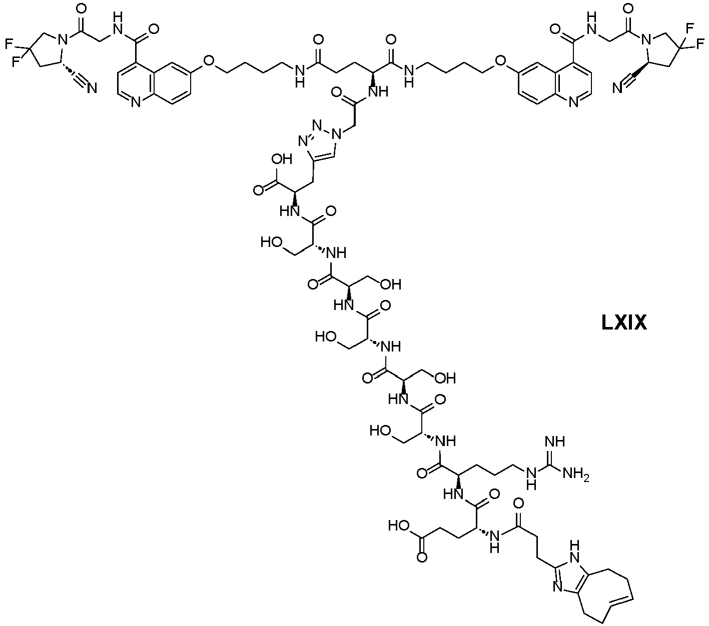

### Examples:

### Example 1: Synthesis of general reagents

### Synthesis of 3-(4-(2-Fluoroethoxy)phenyl)-1,2,4,5-tetrazine (Compound 1)

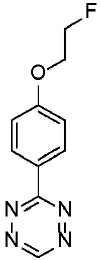

The compound was synthesized as previously described in the literature to afford 0.36 g (37%) of the desired compound as a red oil (García-Vázquez et al., Pharmaceuticals, 15, 2022). R_{f} = 0.33 (Heptane/EtOAc 80/20); ¹H NMR (400 MHz, CDCl₃) δ 10.07 (s, 1H), 8.53 (d, *J=* 8.9 Hz, 2H), 7.06 (d, *J* = 8.9 Hz, 2H), 4.89 - 4.78 (m, 1H), 4.74 - 4.62 (m, 1H), 4.43 - 4.29 (m, 1H), 4.27 - 4.16 (m, 1H); ¹³C NMR (101 MHz, CDCl₃) δ 166.06, 162.56, 157.40, 130.26, 124.56, 115.38, 81.63 (d, *J* = 171.5 Hz), 67.29 (d, *J* = 20.6 Hz).

### Synthesis of 2-(4-(1,2,4,5-Tetrazin-3-yl)phenoxy)ethyl 4-nitrobenzenesulfonate (Compound 2)

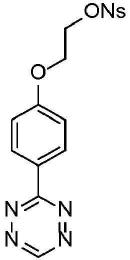

The compound was synthesized as described in the literature 0.12 g (65%) of the desired product as a red solid (García-Vázquez et al., Pharmaceuticals, 15, 2022). R_{f} = 0.41 (Heptane/EtOAc 50/50); ¹H NMR (400 MHz, DMSO) δ 10.52 (s, 1H), 8.97 - 8.31 (m, 5H), 8.33 - 7.87 (m, 2H), 7.11 (d, *J* = 8.9 Hz, 2H), 4.97 - 4.50 (m, 2H), 4.48 - 4.06 (m, 2H); ¹³C NMR (101 MHz, DMSO) δ 165.57, 161.88, 158.28, 154.85, 147.71, 130.25, 127.38, 125.26, 123.79, 116.05, 75.01, 67.33.

### Synthesis of 2,2,2"-(10-(2-((3-lodo-5-(1,2,4,5-tetrazin-3-yl)benzyl)amino)-2-oxoethyl)-1,4,7,10-tetraazacyclo dodecane-1,4,7-triyl)triacetic acid (Compound 9)

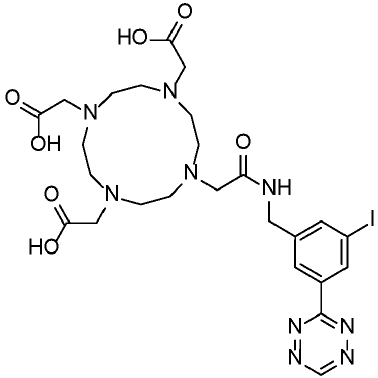

To a solution of compound **8** (0.05 g, 0.14 mmol) and DIPEA (0.19 mL, 1.14 mmol) in DMF (1 mL) was added DOTA-NHS HPF₆/TFA salt (0.12 g, 0.15 mmol). The reaction was stirred at rt for 12 hours and directly purified by preparative HPLC to give 0.065 g (65%) of the desired compound as a red solid. ¹H NMR (600 MHz, D₂O) δ 10.35 (s, 1H), 8.60 (s, 1H), 8.24 (s, 1H), 7.92 (s, 1H), 4.44 (s, 2H), 3.71 (br s, 6H), 3.24 (br s, 16H).

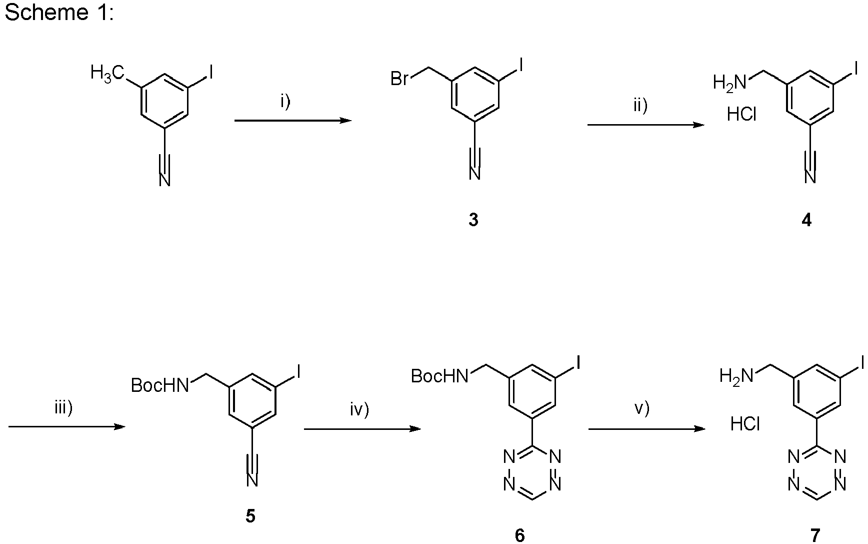

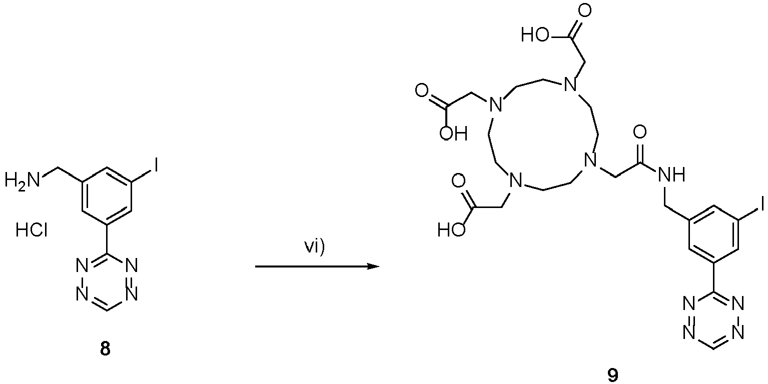

*Reagents and conditions: i*) NBS, AIBN, MeCN, reflux 12 h; *ii*) NH₄OH, THF, 50 °C, 5 h; iii) Boc₂O, Et₃N, DCM, rt, 12 h; iv) CH₂Cl₂, S₈, NH₂NH₂ H₂O, EtOH, 50 °C, 24 h; v) HCl, dioxane, rt, 2 h.

### Synthesis of 2,5-dioxopyrrolidin-1-yl (E)-2-(1,3-dioxo-1,3,6,7,10, 11-hexahydro-2H-cycloocta[bjpyrrolo[3,4-gjquinoxalin-2-yl)acetate (Compound 10)

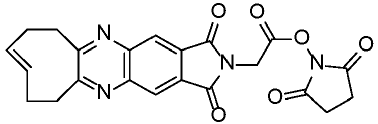

The compound was synthesized as previously reported to give 0.056 g (72%) of the desired compound as a yellow solid (PCT/EP2023/055930). R_{f} = 0.41 (n-Heptane/EtOAc 50/50); ¹H NMR (400 MHz, CDCl₃) δ 8.56 - 8.47 (m, 2H), 6.10 (td, *J=* 12.1, 5.9 Hz, 1H), 5.25 - 5.07 (m, 1H), 4.87 (s, 2H), 3.40 - 3.30 (m, 2H), 3.30 - 3.21 (m, 1H), 3.15 - 3.04 (m, 1H), 2.92 - 2.68 (m, 6H), 2.45 (q, *J=* 11.9 Hz, 1H), 2.33 (dt, *J=* 12.1, 6.1 Hz, 1H); ¹³C NMR (101 MHz, CDCl₃) δ 168.16, 165.58, 163.15, 161.67, 161.31, 143.40, 143.24, 136.54, 132.67, 130.16, 130.00, 126.26, 126.07, 46.74, 41.81, 37.05, 34.19, 28.26, 25.55.

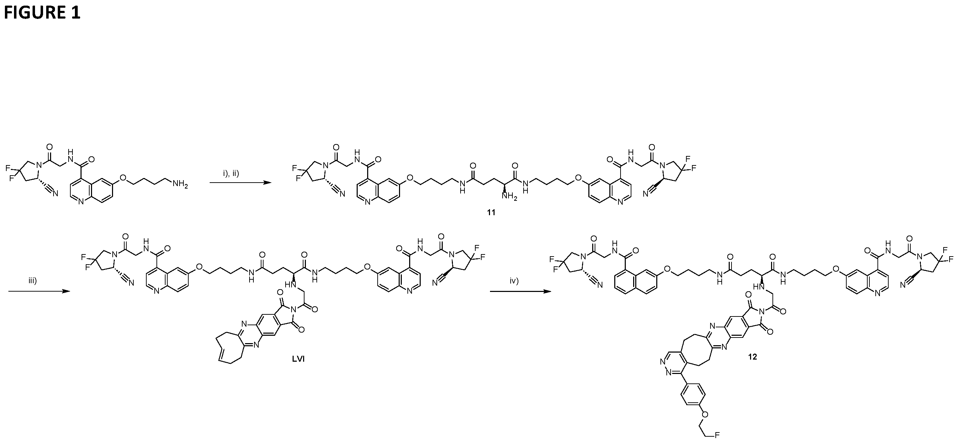

### Example 2: Synthesis of Compound 12 (Figure 1)

Figure 1 is a scheme showing the synthesis of compound **13.** The following reagents and conditions were used in the steps i - iv: i) Boc-Glu-OH; HOBT, EDCl·HCl, DMF, rt, 12 h; ii) HCl, dioxane, rt, 6 h; iii) **10,** N-methyl morpholine, DMF, rt, 12 h.; iv) Tz-1, TFA, H₂O, ACN, rt, 24 h.

### Synthesis of Compound 11 (Figure 1)

The compound was obtained as previously by Martin et al., Cancers, 15, 2023. The spectral data were in agreement what was previously reported. ¹H NMR (400 MHz, MeOD) δ 8.72 (t, *J* = 4.5 Hz, 2H), 8.00 - 7.86 (m, 4H), 7.55 (dd, *J* = 4.5, 2.6 Hz, 2H), 7.43 (dd, *J* = 9.3, 2.7 Hz, 2H), 5.17 (dt, *J* = 9.5, 2.8 Hz, 2H), 4.40 - 3.90 (m, 15H), 3.30 - 3.18 (m, 4H), 3.03 - 2.75 (m, 4H), 2.26 (t, *J* = 7.5 Hz, 2H), 1.96 - 1.80 (m, 4H), 1.80 - 1.65 (m, 4H); MS (ESI⁺): *m*/*z* (%) = 325.5 (100, [M+3H]³⁺), 487.8 (35, [M+2H]²⁺), 974.3 (10, [M+H]⁺), calculated for C₄₇H₅₁F₄N₁₁O₈: 973.3 [M].

### Synthesis of Compound LVI (Figure 1)

To a solution of **11** (0.033 g, 0.033 mmol) and **10** (0.014 g, 0.033 mmol) in dry DMF (2 mL) under argon, was added 4-methylmorpholine (0.009 mL, 0.083 mmol). The reaction was stirred at room temperature for 12 h. The mixture was then diluted with 10 mL of 1%TFA in H₂O and purified by preparative HPLC. The collected fractions were freeze-dried to give 0.03 g (69%) of the desired compound as a white solid. MS (ESI+): m/z (%) = 432.0 (30, [M+3H]³⁺), 647.3 (100, [M+2H]²⁺), 1293.4 (20, [M+H]⁺), calculated for C₆₅H₆₄F₄N₁₄O₁₁: 1292.4 [M].

### Synthesis of Compound 12 (Figure 1)

To a solution of **LVI** (0.003 g, 0.0023 mmol) in ACN (2 mL) and 1% TFA in H₂O (2 mL) was added Tz **1** (0.001 g, 0.0026 mmol) in 1%TFA in H₂O (1 mL). The reaction was stirred at room temperature for 12 h. The mixture was directly purified by preparative HPLC. The collected fractions were freeze-dried to give 0.002 g (62%) of the desired compound as a white solid. MS (ESI+): m/z (%) = 495.4 (100, [M+3H]³⁺), 742.4 (60, [M+2H]²⁺), 1483.4 (30, [M+H]⁺), calculated for C₇₆H₇₂F₅I N₁₅O₁₂: 1482.4[M].

### Example 3: Synthesis of Compound 13 (Scheme 2)

Scheme 2 shows the synthesis of compound **13.** The grey dot in the intermediate structures symbolizes a resin. The following reagents and conditions were used in the steps i - iii: i) a) DIPEA, CH₂Cl₂, rt, 2 h, b) 50% piperidine in DMF, rt, 20 min.; ii) Fmoc-tBu-D-Ser-OH, HATU, DIPEA, DMF, rt, 2 h, b) 50% piperidine in DMF, rt, 20 min.; iii) TFA, TIPS, H₂O, rt, 2 h.

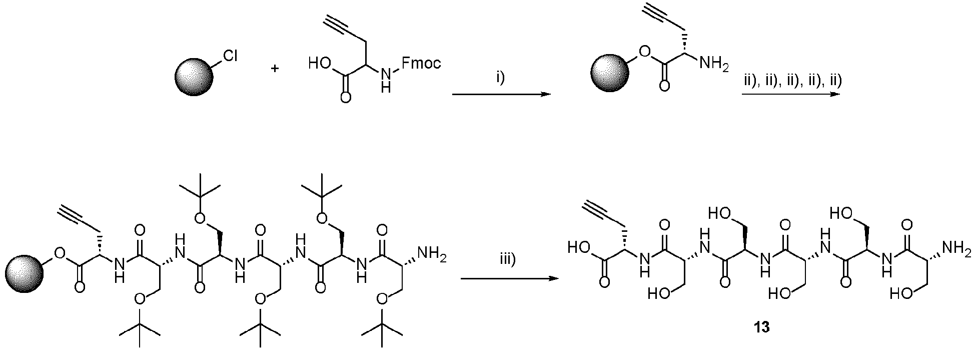

### Synthesis of Compound 13 (Scheme 2)

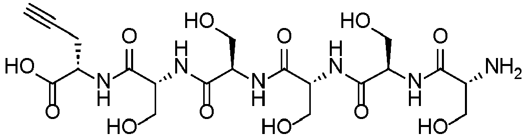

Chlorotrityl resin (1.28 mmol, 1.2 g) was stirred in a round bottom flask in CH₂Cl₂ (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH₂Cl₂ (10 mL). The resin was capped by reacting two times for 20 minutes with 5 mL of a mixture of CH₂Cl₂, MeOH, DIPEA (17:2:1). The Fmoc-protecting group was then cleaved with a mixture of DMF and piperidine (1:1). The product reacted with 2 equiv. of Fmoc-D-Ser(tBu)-OH activated with 1.95 equivalent of HATU and 2 equivalent of DIPEA. Deprotection of the Fmoc group with a mixture of DMF and piperidine (1:1) gave the unprotected immobilized peptide. The D-Ser coupling was repeated four more times to afford the final protected peptide. The product was cleaved from the resin and deprotected with trifluoroacetic acid (TFA), triisopropylsilane (TIPS) and water (95:2.5:2.5). Preparative HPLC afforded 0.14 g of the desired peptide. MS (ESI+): m/z (%) = 547.3 (100, [M-H]⁻), calculated for C₂₀H₃₀N₆O₁₂: 546.2 [M].

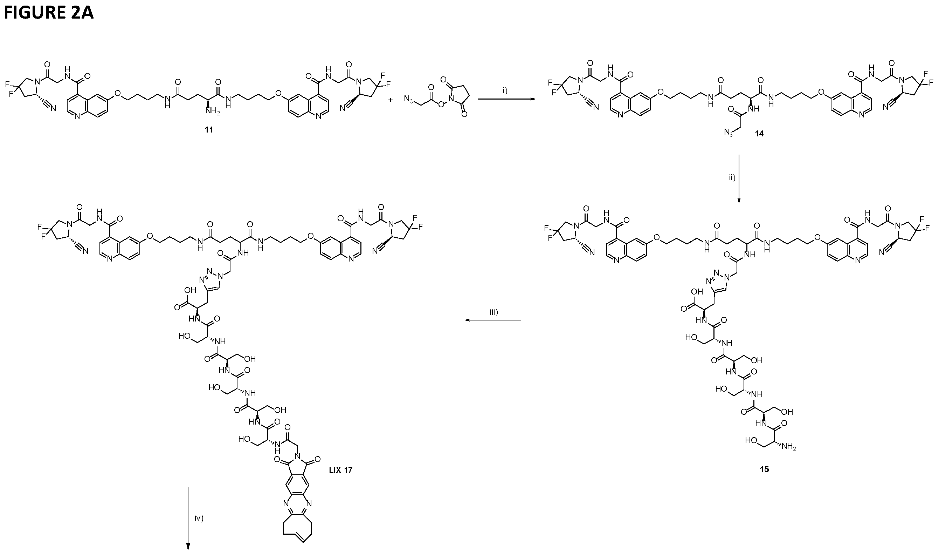

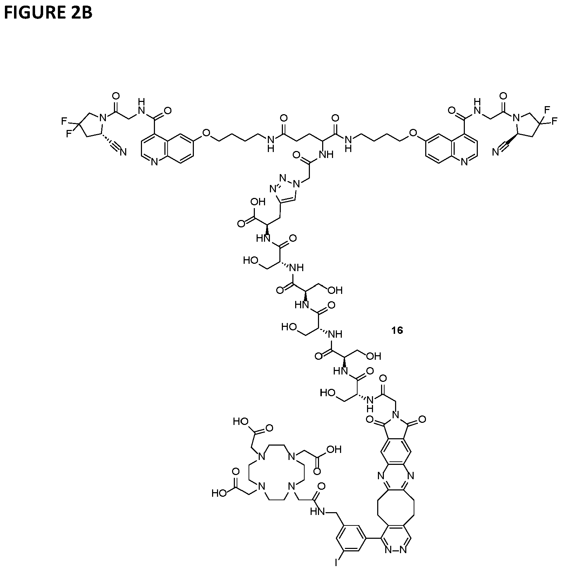

### Example 4: Synthesis of Compound 16 (Figure 2A and 2B)

Figure 2A and 2B is a scheme showing the synthesis of compound **16.** The following reagents and conditions were used in the steps i - iii: i) DIPEA, DMF, rt, 5 h; ii) **13,** CuSO₄·H₂O, sodium ascorbate, BPDS, DMF, H₂O, rt, 12 h; iii) **10,** N-methyl morpholine, DMF, rt, 12 h, iv) 9, ACN, H₂O, TFA, rt, 5 h.

### Synthesis of Compound 14

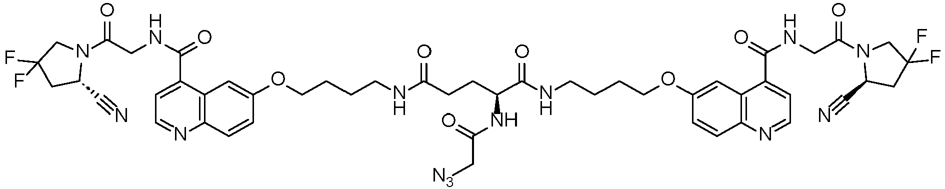

To a solution of **11** (0.049 mmol, 0.05 g) in DMF (2 mL) was added DIPEA (0.12 mmol, 0.021 mL) and 2-azidoacetic NHS ester (0.054 mmol, 0.011 g). The resulting mixture was stirred for 4 hours. The volatiles were then removed under reduced pressure and the residue purified by reversed phase chromatography to give 0.02 g (43%) of the desired product as a white solid. Rf =0.15 (CH₂Cl₂/MeOH, 90/10); ¹H NMR (600 MHz, MeOD) δ 8.90 - 8.84 (m, 2H), 8.06 (t, *J* = 2.3 Hz, 2H), 8.03 (d, *J=* 9.3 Hz, 2H), 7.78 (dd, *J=* 5.0, 2.1 Hz, 2H), 7.61 (dt, *J* = 9.3, 3.0 Hz, 2H), 5.16 (dt, *J* = 9.4, 2.6 Hz, 2H), 4.40 - 4.20 (m, 12H), 4.19 - 4.09 (m, 2H), 3.91 (s, 2H), 3.26 (t, *J* = 7.0 Hz, 2H), 2.99 - 2.75 (m, 4H), 2.25 (t, *J* = 7.4 Hz, 2H), 2.08 (dtd, *J* = 13.0, 7.7, 5.1 Hz, 1H), 1.90 (pd, *J* = 7.8, 2.9 Hz, 6H), 1.74 (qd, *J* = 12.5, 7.1 Hz, 5H); ¹³C NMR (151 MHz, MeOD) δ 174.73, 173.35, 170.15, 169.43, 169.38, 169.34, 161.37, 161.12, 160.61, 160.58, 146.83, 146.72, 145.43, 145.37, 129.22, 128.34, 127.65, 127.55, 127.32, 127.25, 125.92, 120.66, 118.30, 118.28, 105.74, 69.95, 69.91, 57.48 (dt, *J* = 43.0, 21.5 Hz), 54.41, 53.17, 52.95, 52.78, 52.74, 45.97, 45.94, 42.97, 42.96, 40.17, 40.15, 33.10, 29.22, 27.43, 27.34, 26.98, 26.87, 17.28 (dt, *J* = 38.4, 19.1 Hz); MS (ESI+): m/z (%) = 538.3 (100, [M+2H]²⁺), 1057.4 (30, [M+H]⁺), calculated for C₄₉H₅₂F₄N₁₄O₉: 1056.4 [M].

### Synthesis of Compound 15 (Figure 2A)

An aqueous solution of CuSO₄·5H₂O (100 mg/mL; 0.0089 mmol) was mixed with an aqueous solution of sodium ascorbate (300 mg/mL, 0.036 mmol), when the color of the mixture turned yellow a solution of BPDS (25 mg/mL, 0.0089 mmol) in water was added. The resulting blue/green mixture was added to a solution of the peptide (0.032 g, 0.048 mmol) in DMF (0.5 mL). Afterward, **14** (0.047 g, 0.044 mmol) dissolved in DMF (1 mL) was stirred at room temperature for 4 h. Full click was observed. The compound was diluted in water (12 mL) and purified by reversed flash HPLC to give 0.027 g of the compound as a white solid. MS (ESI+): m/z (%) = 402.5 (40, [M+4H]⁴⁺), 536.3 (100, [M+3H]³⁺), 803.7 (80, [M+4H]⁴⁺), calculated for C₆₉H₈₅F₄N₂₀O₂₁: 1605.6 [M].

### Synthesis of Compound LIX (Figure 2A)

To a solution of **15** (0.03 g, 0.013 mmol) and **10** (0.007 g, 0.015 mmol) in dry DMF (2 mL) under argon, was added 4-methylmorpholine (0.012 mL, 0.11 mmol). The reaction was stirred at room temperature for 12 h. The mixture was then diluted with 10 mL of 1%TFA in H₂O and purified by preparative HPLC. The collected fractions were freeze-dried to give 0.007 g (24%) of the desired compound as a white solid. MS (ESI+): m/z (%) = 642.4 (60, [M+2H]²⁺), 962.9 (100, [M+2H]²⁺), 1925.6 (100, [M+H]⁺),calculated for C₈₇H₉₇F₄N₂₃O₂₄: 1923.7 [M].

### Synthesis of Compound 16 (Figure 2B)

To a solution of **LIX** (0.005 g, 0.0026 mmol) in ACN (2 mL) and 1% TFA in H₂O (2 mL) was added **9** (0.002 g, 0.0027 mmol) in 1%TFA in H₂O (1 mL). The reaction was stirred at room temperature for 12 h. The mixture was directly purified by preparative HPLC. The collected fractions were freeze-dried to give 0.004 g (59%) of the desired compound as a white solid. MS (ESI+): m/z (%) = 649.4 (100, [M+4H]⁴⁺), 865.4 (70, [M+3H]³⁺), 1297.9 (40, [M+2H]²⁺), calculated for C₁₁₂H₁₂₉F₄I N₃₀O₃₁: 2592.8 [M].

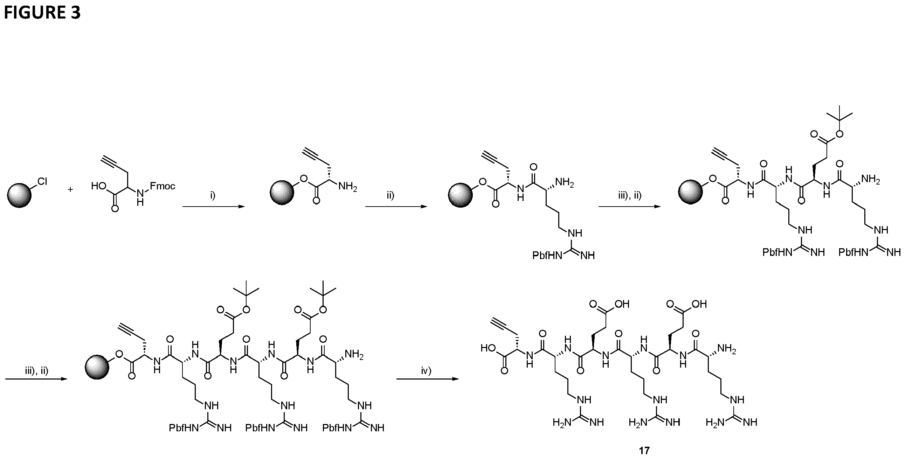

### Example 5: Synthesis of Compound 17 (Figure 3)

Figure 3 is a scheme showing the synthesis of compound **17.** The grey dot in the intermediate structures symbolizes a resin. The following reagents and conditions were used in the steps i - iv: : i) a) DIPEA, CH₂Cl₂, rt, 2 h, b) 50% piperidine in DMF, rt, 20 min.; ii) Fmoc-D-Arg(Pbf)-OH, HATU, DIPEA, DMF, rt, 2 h, b) 50% piperidine in DMF, rt, 20 min.; iii) Fmoc-D-Glu-OH, HATU, DIPEA, DMF, rt, 2 h, b) 50% piperidine in DMF, rt, 20 min.; iv) TFA, TIPS, H₂O, rt, 2 h.

### Synthesis of 6A (Compound 17, Figure 3)

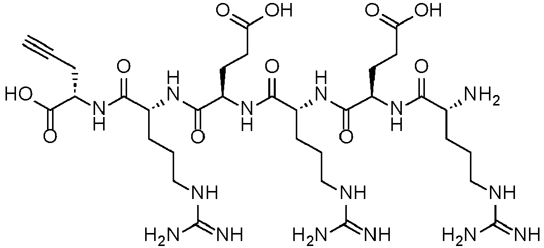

Chlorotrityl resin (1.28 mmol, 1.2 g) was stirred in a round bottom flask in CH₂Cl₂ (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH₂Cl₂ (10 mL). The resin was capped by reacting two times for 20 minutes with 5 mL of a mixture of CH₂Cl₂, MeOH, DIPEA (17:2:1). The Fmoc-protecting group was then cleaved with a mixture of DMF and piperidine (1:1). The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave the desired product. Subsequently, 4 equiv. of Fmoc-D-Glu-OH were activated with 3.92 equiv. of HATU and 4 equiv. of DIPEA in DMF. The solution was added to the resin-immobilized peptide and shaken for 1 hour. The Fmoc-protecting group was cleaved with a mixture of DMF and piperidine. The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave deprotected immobilized resin. The D-Glu and D-Arg coupling was repeated one more time to afford the final protected peptide. The product was cleaved from the resin and deprotected with trifluoroacetic acid (TFA), triisopropylsilane (TIPS) and water (95:2.5:2.5). Preparative HPLC afforded 0.15 g of the desired peptide. MS (ESI+): m/z (%) = 280.9 (100, [M+3H]³⁺), 420.8 (90, [M+2H]²⁺), 840.6 (30, [M+H]⁺), calculated for C₃₃H₅₇N₁₅O₁₁. 839.4 [M].

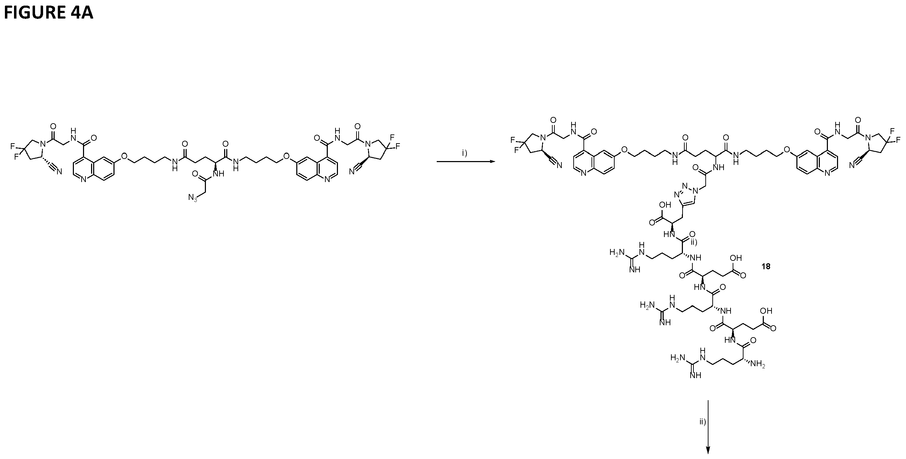

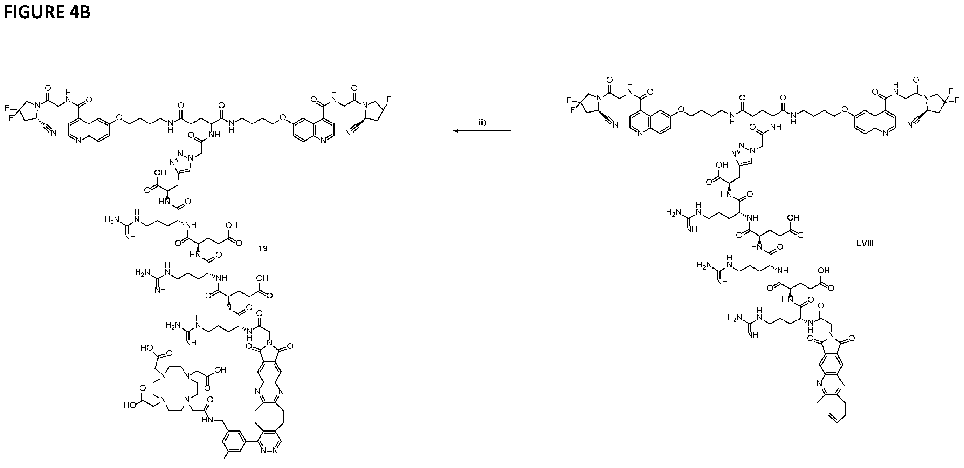

### Example 6: Synthesis of Compound 22, (Figure 4A and 4B)

Figure 4A and 4B is a scheme showing the synthesis of compound **22.** The following reagents and conditions were used in the steps i - iii: i) **19,** CuSO₄·H₂O, sodium ascorbate, BPDS, DMF, H₂O, rt, 12 h; ii) **10,** N-methyl morpholine, DMF, rt, 12 h, iii) **9,** ACN, H₂O, TFA, rt, 5 h.

### Synthesis of Compound 18 (Figure 4A)

An aqueous solution of CuSO₄·5H₂O (100 mg/mL; 0.01 mL; 0.0038 mmol) was mixed with an aqueous solution of sodium ascorbate (300 mg/mL, 0.01 mL; 0.015 mmol), when the color of the mixture turned yellow a solution of BPDS (25 mg/mL, 0.01 mL, 0.0038 mmol) in water was added. The resulting blue/green mixture was added to a solution of **17** (0.024 g, 0.021 mmol) in DMF (0.5 mL). Afterward, **14** (0.02 g, 0.019 mmol) dissolved in DMF (1 mL) was stirred at room temperature for 4 h. Full click was observed. The compound was diluted in water (12 mL) and purified by reversed flash HPLC to give 0.028 g of the compound as a white solid. MS (ESI+): m/z (%) = 380.3 (80, [M+5H]⁵⁺), 475.2 (100, [M+4H]⁴⁺), 633.2 (90, [M+3H]³⁺), 949.0 (50, [M+2H]²⁺), 1896.8 (5, [M+H]⁺), calculated for C₈₂H₁₀₉F₄N₂₉O₂₀: 1895.9 [M].

### Synthesis of Compound LVIII (Figure 4B)

To a solution of **LVIII** (0.03 g, 0.013 mmol) and **10** (0.07 g, 0.015 mmol) in dry DMF (2 mL) under argon, was added 4-methylmorpholine (0.012 mL, 0.11 mmol). The reaction was stirred at room temperature for 12 h. The mixture was then diluted with 10 mL of 1%TFA in H₂O and purified by preparative HPLC. The collected fractions were freeze-dried to give 0.007 g (21%) of the desired compound as a white solid. MS (ESI+): m/z (%) = 444.2 (100, [M+5H]⁵⁺), 555.2 (30, [M+4H]⁴⁺), 739.8 (30, [M+3H]³⁺), 1108.8 (20, [M+2H]²⁺), calculated for C₁₀₀H₁₂₂F₄N₃₂O₂₃. 2214.9 [M].

### Synthesis of Compound 19 (Figure 4B)

To a solution of **11** (0.006 g, 0.0023 mmol) in ACN (2 mL) and 1% TFA in H₂O (2 mL) was added 9 (0.002 g, 0.0026 mmol) in 1%TFA in H₂O (1 mL). The reaction was stirred at room temperature for 12 h. The mixture was directly purified by preparative HPLC. The collected fractions were freeze-dried to give 0.005 g (71%) of the desired compound as a white solid. MS (ESI+): m/z (%) = 482.0 (100, [M+6H]⁶⁺), 578.4 (40, [M+5H]⁵⁺), 722.7 (30, [M+4H]⁴⁺), 962.8 (50, [M+3H]³⁺), calculated for C₁₂₅H₁₅₄F₄I N₃₉O₃₀: 2884.1[M].

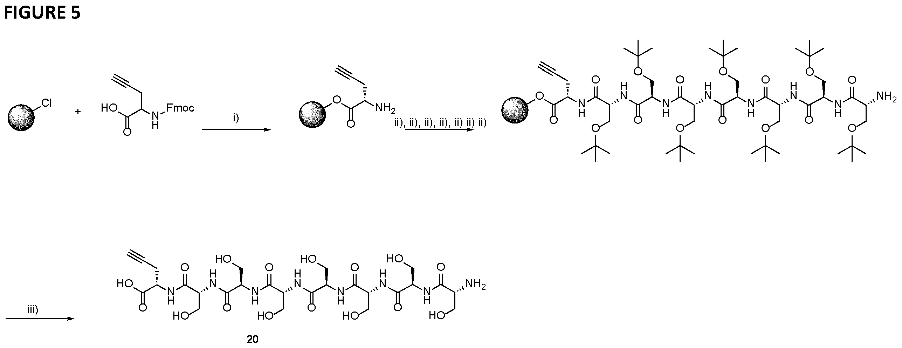

### Example 7: Synthesis of Compound 0 (Figure 5)

Figure 5 is a scheme showing the synthesis of compound **20.** The grey dot in the intermediate structures symbolizes a resin. The following reagents and conditions were used in the steps i - iii: i) a) DIPEA, CH₂Cl₂, rt, 2 h, b) 50% piperidine in DMF, rt, 20 min.; ii) Fmoc-tBu-D-Ser-OH, HATU, DIPEA, DMF, rt, 2 h, b) 50% piperidine in DMF, rt, 20 min.; iii) TFA, TIPS, H₂O, rt, 2 h.

### Synthesis of Compound 20, (Figure 5)

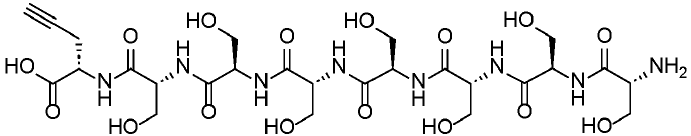

Chlorotrityl resin (1.28 mmol, 1.2 g) was stirred in a round bottom flask in CH₂Cl₂ (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH₂Cl₂ (10 mL). The resin was capped by reacting two times for 20 minutes with 5 mL of a mixture of CH₂Cl₂, MeOH, DIPEA (17:2:1). The Fmoc-protecting group was then cleaved with a mixture of DMF and piperidine (1:1). The product reacted with 2 equiv. of Fmoc-D-Ser(tBu)-OH activated with 1.95 equivalent of HATU and 2 equivalent of DIPEA. Deprotection of the Fmoc group with a mixture of DMF and piperidine (1:1) gave the unprotected immobilized peptide. The D-Ser coupling was repeated six more times to afford the final protected peptide. The product was cleaved from the resin and deprotected with trifluoroacetic acid (TFA), triisopropylsilane (TIPS) and water (95:2.5:2.5). Preparative HPLC afforded 0.12 g of the desired peptide. MS (ESI+): m/z (%) = 721.2 (100, [M-H]⁻), calculated for C₂₀H₃₀N₆O₁₂: 722.3 [M].

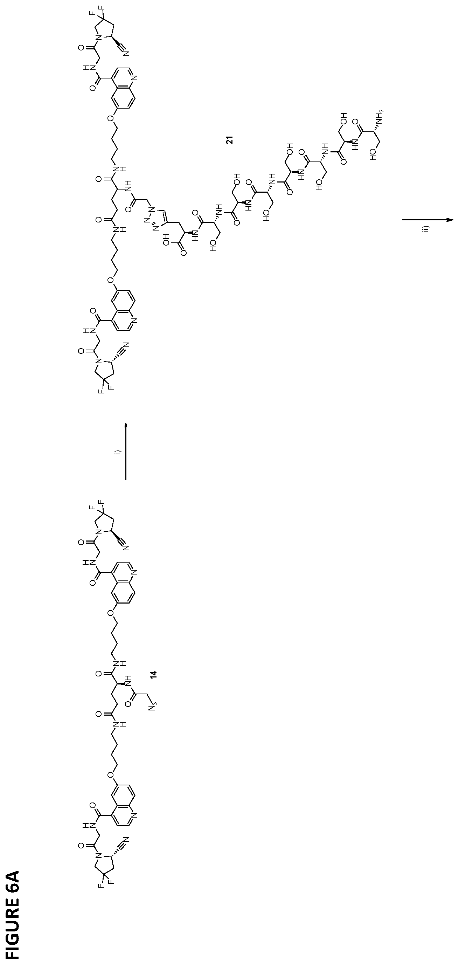

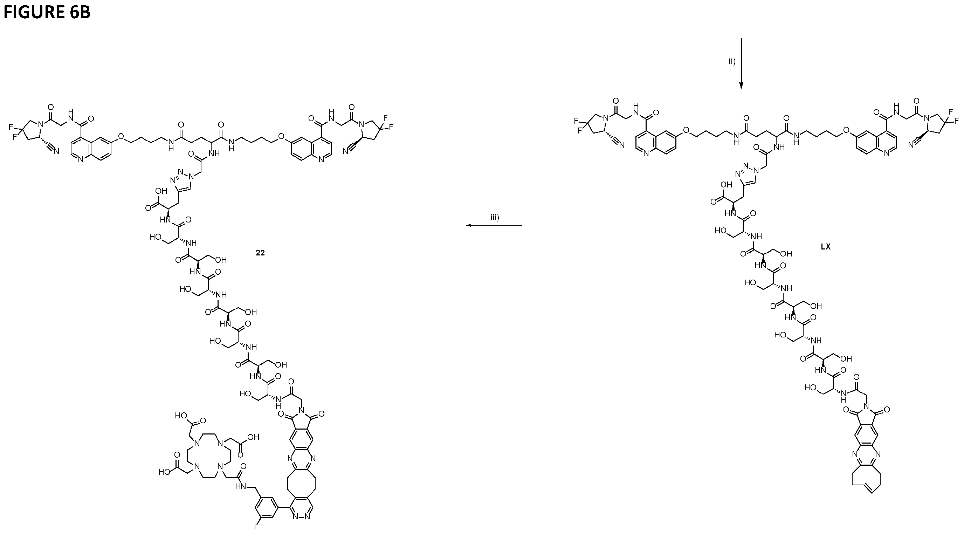

### Example 8: Synthesis of Compound 26 (Figure 6A and 6B)

Figure 6A and 6B is a scheme showing the synthesis of compound **26.** The following reagents and conditions were used in the steps i - iii: i) **20,** CuSO·H₂O, sodium ascorbate, BPDS, DMF, H₂O, rt, 12 h; ii) **10,** N-methyl morpholine, DMF, rt, 12 h, iii) **9,** ACN, H₂O, TFA, rt, 5 h.

### Synthesis of Compound 21 (Figure 6A)

An aqueous solution of CuSO₄·5H₂O (100 mg/mL; 0.01 mL; 0.0066 mmol) was mixed with an aqueous solution of sodium ascorbate (300 mg/mL, 0.03 mL; 0.053 mmol), when the color of the mixture turned yellow a solution of BPDS (25 mg/mL, 0.3 mL, 0.0133 mmol) in water was added. The resulting blue/green mixture was added to a solution of **20** (0.061 g, 0.073 mmol) in DMF (0.5 mL). Afterward, **14** (0.07 g, 0.066 mmol) dissolved in DMF (1 mL) was stirred at room temperature for 4 h. Full click was observed. The compound was diluted in water (12 mL) and purified by reversed flash HPLC to give 0.015 g of the compound as a white solid. MS (ESI+): m/z (%) = 594.1 (100, [M+3H]³⁺), 890.5 (60, [M+2H]²⁺), 1779.7 (5, [M+H]⁺), calculated for C₇₅H₉₄F₄N₂₂O₂₅: 1778.7 [M].

### Synthesis of Compound LX (Figure 6B)

To a solution of **21** (0.02 g, 0.011 mmol) and **10** (0.06 g, 0.013 mmol) in dry DMF (2 mL) under argon, was added 4-methylmorpholine (0.006 mL, 0.053 mmol). The reaction was stirred at room temperature for 12 h. The mixture was then diluted with 10 mL of 1%TFA in H₂O and purified by preparative HPLC. The collected fractions were freeze-dried to give 0.005 g (22%) of the desired compound as a white solid. MS (ESI+): m/z (%) = 700.7 (60, [M+3H]³⁺), 1050.2 (100, [M+2H]²⁺), calculated for C₉₃H₁₀₇F₄N₂₅O₂₈: 2097.8 [M].

### Synthesis of Compound 22 (Figure 6B)

To a solution of **LX** (0.003 g, 0.0014 mmol) in ACN (2 mL) and 1% TFA in H₂O (2 mL) was added **9** (0.001 g, 0.0014 mmol) in 1%TFA in H₂O (1 mL). The reaction was stirred at room temperature for 12 h. The mixture was directly purified by preparative HPLC. The collected fractions were freeze-dried to give 0.001 g (30%) of the desired compound as a white solid. MS (ESI+): m/z (%) = 554.8 (45, [M+5H]⁵⁺), 693.2 (20, [M+4H]⁴⁺), 923.7 (100, [M+3H]³⁺), calculated for C₁₁₈H₁₃₉F₄I N₃₂O₃₅: 2776.9[M].

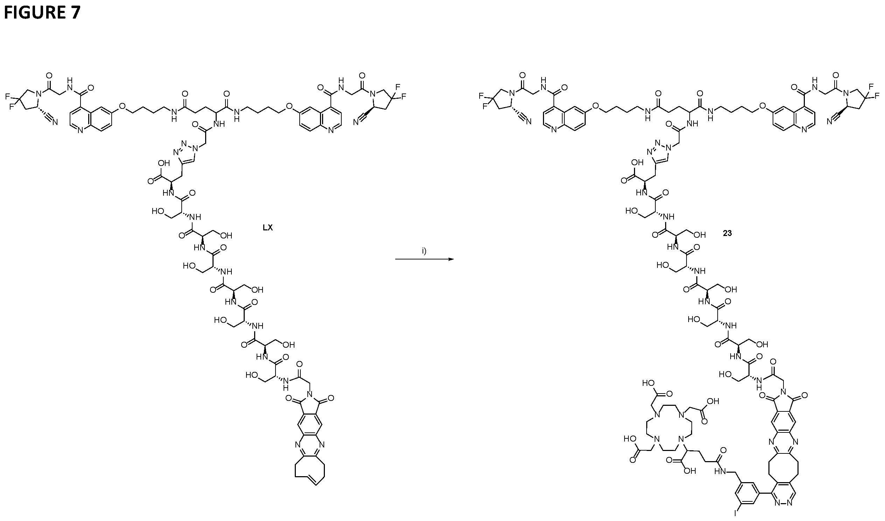

### Example 9: Synthesis of Compound 23 (Figure 7)

Figure 7 is a scheme showing the synthesis of compound **23.** The following reagents and conditions were used in step i: i) H-DOTAGA-ITz, ACN, H₂O, TFA, rt, 5 h.

### Synthesis of Compound 23 (Figure 7)

To a solution of **LX** (0.003 g, 0.0014 mmol) in ACN (2 mL) and 1% TFA in H₂O (2 mL) was added **9** (0.001 g, 0.0014 mmol) in 1%TFA in H₂O (1 mL). The reaction was stirred at room temperature for 12 h. The mixture was directly purified by preparative HPLC. The collected fractions were freeze-dried to give 0.002 g (59%) of the desired compound as a white solid. MS (ESI+): m/z (%) = 569.3 (20, [M+5H]⁵⁺), 711.2 (40, [M+4H]⁴⁺), 947.5 (100, [M+3H]³⁺), calculated for C₁₂₁H₁₄₃F₄I N₃₂O₃₇: 2838.9[M].

### Example 10: Radiolabeling of [¹⁸F]1 before IEDDA conjugation

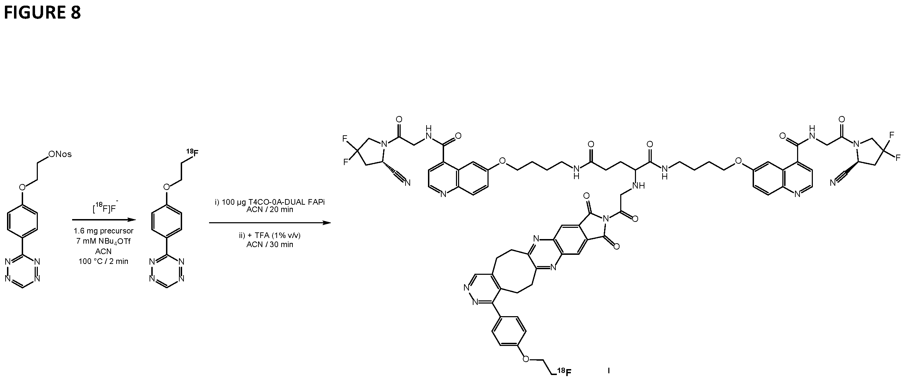

Figure 8 shows the ¹⁸F-radiolabeling of **[¹⁸F]1**, IEDDA conjugation and oxidation steps (step i-ii).

The radiolabeling was performed according to published procedure (García-Vázquez et al., Pharmaceuticals, 15, 2022). The aqueous [¹⁸F]fluoride solution received from the cyclotron was passed through a Sep-Pak Light QMA cartridge preconditioned with 5 mL 0.5 M K₃PO₄. [¹⁸F]Fluoride was eluted from the QMA cartridge into a 4 mL v-shaped vial using Bu₄NOTf solution (20 mM in MeOH, 1 mL). The eluate was dried at 100°C for 5 min under nitrogen or helium flow. After MeOH had evaporated, acetonitrile (0.5 mL) was added to the same vial and evaporated under the same conditions to remove traces of water. Two additions of acetonitrile were performed. Nosyl precursor **2** (1.5 mg) was dissolved in anhydrous acetonitrile (0.3 mL), diluted with tBuOH (0.7 mL) and added to the dried [¹⁸F]fluoride residue. After reacting for 5 min at 100 °C, the reaction was cooled to 80 °C with ambient air flow, diluted with water (2 mL) and purified by semipreparative HPLC: Discovery HS F5 5 µm, 250 mm × 10 mm column, isocratic elution with 50% acetonitrile in 20 mM citrate buffer pH 6.1, elution speed 5 mL/min. The product peak (retention time 9.0 min) was collected, diluted with water (50-100 mL) and passed through a Sep-pak Plus C18 Short solid phase extraction cartridge (Waters, USA) that was preconditioned by flushing it with EtOH/water mixture (1/1 v/v, 10 mL). The Sep-pak cartridge was then flushed with extra water (5 mL), blown with nitrogen, eluted with 1-2 mL of organic solvent (ACN or EtOH) and diluted with water to achieve the necessary concentration of the organic solvent for the next step. **[¹⁸F]1** was confirmed in a radiochemical yield of 16±10% (n=4) and >95% radiochemical purity by analytical radio-HPLC (tᵣₑₜ = 6.7 min): Luna C18(2) 5µm column 150x4.6 mm; mobile phase H2O/ACN + 0.1% TFA (30-65% ACN in 10 min); flow 1.5 mL/min.

### Example 11: IEDDA conjugation of TCO functionalized PSMA derivatives with [¹⁸F]1

### Click and oxidation step:

To the solution of **[¹⁸F]1** in acetonitrile (1 mL, ca. 1.1 GBq) 100 µg **LVI** (50 µL 2 mg/mL in DMSO) was added. Full click consumption/conversion was confirmed via radio-HPLC after 20 min. 10 µL TFA (1% v/v) was added to the solution and left standing at room temperature for 30 min. Figure 8 shows the full synthesis starting from the nosyl precursor **2.**

### Purification and formulation:

Crude oxidized **I** was diluted with water (2.7 mL) and purified by semipreparative HPLC: Luna C18 5µ 250x10 mm column; mobile phase H₂O/ACN +0.1% TFA (30-55% ACN in 11.7 min); flow 4 mL/min. The product peak was collected (tᵣₑₜ = 620-645 s), diluted with water (20 mL) and passed through Sep-Pak C18 Plus cartridge (Waters; preconditioning: 10 mL EtOH/water mixture (1/1 v/v). The Sep-Pak cartridge was washed with water (2 mL) and eluted with 0.7 mL of ethanol.

Ethanolic solution of purified **I** (324 MBq, RCP = 100%) was diluted with 9 mL 20 mM citrate buffer (pH 6) and used for injections. The final product was confirmed via analytical radio-HPLC (tret = 4.0 min): Luna C18(2) 5µm column 150x4.6 mm; mobile phase H₂O/ACN + 0.1% TFA (30-65% ACN in 10 min); flow 1.5 mL/min.

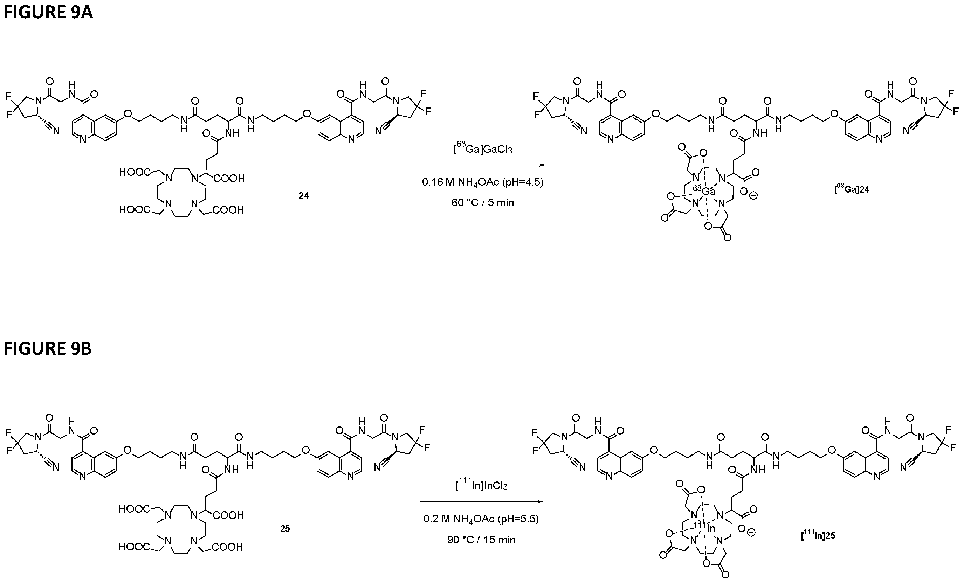

### Example 12: Radiolabeling of [⁶⁸Ga]Ga-DOTAGA.Glu.(FAPi)₂ (Compound [⁶⁸Ga]24, Figure 9A)

Figure 9A is a scheme showing the synthesis of compound **[⁶⁸Ga]24**.

Precursor **24** was synthesized according to published procedure (Martin et al., Cancers, 15(6), 1889, 2023). Precursor **24** (8 µg, 4 µL of 2 mg/mL stock solution in DMSO) and 80 µL 1 M NH₄OAc (pH = 5.5) was added to a solution of [⁶⁸Ga]GaCl₃ (500 µL in 0.1 M HCl, 210 MBq) and was heated/shaken at 60 °C for 5 min (RCC > 90%). After dilution with 5 mL H₂O it was rinsed through Strata-X cartridge from Phenomenex, washed with 0.3 mL H₂O and eluted with 0.5 mL EtOH. After evaporating EtOH (40 °C, 5 min), the product (97 MBq, d.c. RCY = 61% after 30 min synthesis time) was redissolved in 20µL EtOH and 2 mL 0.1 M sterile phosphate buffer (PB, pH=7.0). The final product was obtained with a RCP of 90.5% determined via analytical radio-HPLC (tret = 5.3 min): Aeris Peptide XB-C18 3.6µ 150x4.6 mm column; mobile phase H₂O/ACN + 0.1% TFA (5-75% ACN in 8 min); flow 1.5 mL/min. The compound was confirmed via radio-iTLCs (mobile phases: 0.2 M citrate buffer (pH=4.0) and 0.25 M NH₄OAc (pH=4.0)/MeOH(1:1)).

### Example 13: Radiolabeling of [¹¹1In]In-DOTAGA.Glu.(FAPi)2 (Compound [¹¹¹In]25, Figure 9B)

Figure 9B is a scheme showing the synthesis of compound **[¹¹¹In]25.**

Precursor **25** was synthesized according to published procedure (Martin et al., Cancers, 15(6), 1889, 2023). Precursor **25** (5 µg, 5 µL 1 mg/mL stock solution in H₂O) and 25 µL 1 M NHₐOAc (pH = 5.5) was added to a solution of [111In]InCl₃ (100 µL, ca. 100 MBq) and was shaken at 90 °C for 15 min (RCC > 95%). This was followed by SPE with Sep-Pak tC2 Plus Light cartridge (preconditioning: 2 mL EtOH and 5 mL H₂O; cartridge rinsed with reaction solution, washed with 1 mL H₂O and eluted with 1 mL EtOH/ H₂O (1:1) to yield the final product with RCY = 95% and RCP = 95.2%. This was determined via analytical radio-HPLC (tret = 5.8 min): Luna C18(2) 5µm 150×4.6 mm column; mobile phase H₂O/ACN + 0.1% TFA (10-50% ACN in 8 min); flow 1.5 mL/min. Also confirmed via radio-iTLCs (mobile phases: 0.2 M citrate buffer (pH=4.0) and 1 M NH₄OAc (pH=4.0)/ MeOH(1:1)).

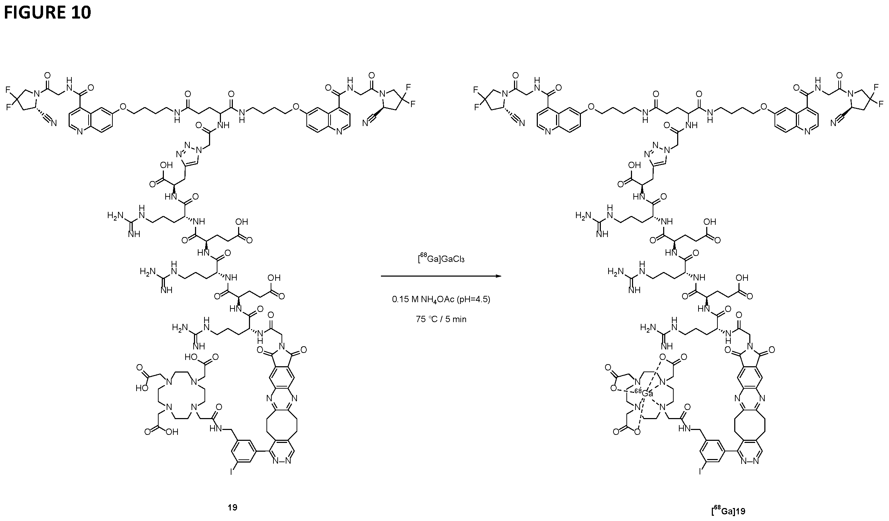

### Example 14: Radiolabeling of compound [⁶⁸Ga]19 (Figure 10)

Figure 10 is a scheme showing the radiolabeling of **compound 19** (**[⁶⁸Ga]19**)**.**

Compound **19** (20 µg, 10 µL of 1 mg/mL stock solution in H₂O/DMSO (9:1)) and 125 µL 1 M AmOAc (pH = 5.5) was added to a solution of [⁶⁸Ga]GaCl₃ (850 µL in 0.1 M HCl, 290 MBq) and was heated/shaken at 75 °C for 5 min (RCC = ca. 93%). This was followed by solid-phase extraction (SPE) with Sep-Pak tC2 Plus Light cartridge from Waters (preconditioning: 2 mL EtOH and 5 mL H₂O; cartridge rinsed with reaction solution, washed with 1 mL H₂O and eluted with 1 mL EtOH/ H₂O (1:1) and further diluted with 4 mL 0.1 M sterile phosphate buffer (PB, pH=7.0) to yield the final product with d.c. RCY = 82% (195 MBq after 20 min synthesis time) and RCP = 99.5% (determined via analytical radio-HPLC: Luna C18 5µ column (150×4.6 mm); mobile phase H2O/ACN+0.1% TFA (10-50% ACN in 8 min); elution flow 1.5 mL/min) and confirmed via radio-iTLCs (mobile phases: 0.2 M citrate buffer (pH=4.0) and 0.25 M NHₐOAc (pH=4.0)/MeOH(1:1)).

The radiotracer was injected within 30 min after the synthesis was finished. The RCP of the final formulation after 120 min was determined to be 94.5% via radio-HPLC using the same conditions.

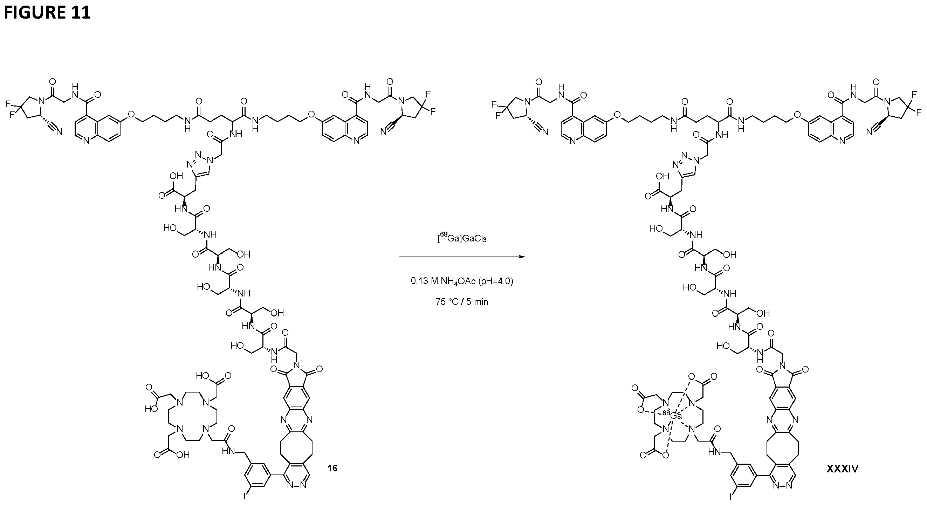

### Example 15: Radiolabeling of compound XXXIV (Figure 11)

Figure 11 is a scheme showing the radiolabeling of compound **16 (XXXIV).**

Compound **16** (20 µg, 10 µL of 2 mg/mL stock solution in DMSO) and 100 µL 1 M AmOAc (pH = 5.5) was added to a solution of [⁶⁸Ga]GaCl₃ (750µL in 0.1 M HCl, 215 MBq) and was heated/shaken at 75 °C for 5 min (RCC = 92.4%). This was followed by SPE with Sep-Pak tC2 Plus Light cartridge (preconditioning: 2 mL EtOH and 5 mL H₂O; cartridge rinsed with reaction solution, washed with 1 mL H₂O and eluted with 0.5 mL EtOH/ H₂O (1:1) and further diluted with 5 mL 0.1 M sterile PB (pH=7.0) to yield the final product with d.c. RCY = 69% (112 MBq after 30 min synthesis time) and RCP = 99.3% (determined via radio-HPLC: Luna C18 5µ column (150x4.6 mm); mobile phase H₂O/ACN+0.1% TFA (10-50% ACN in 8 min); elution flow 1.5 mL/min) and confirmed via radio-iTLCs (mobile phases: 0.2 M citrate buffer (pH=4.0) and 1 M NH₄OAc(pH=4.0)/MeOH(1:1)).

The radiotracer was injected within 30 min after the synthesis was finished. The RCP of the final formulation after 60 min was determined to be 89.2% via radio-HPLC using the same conditions.

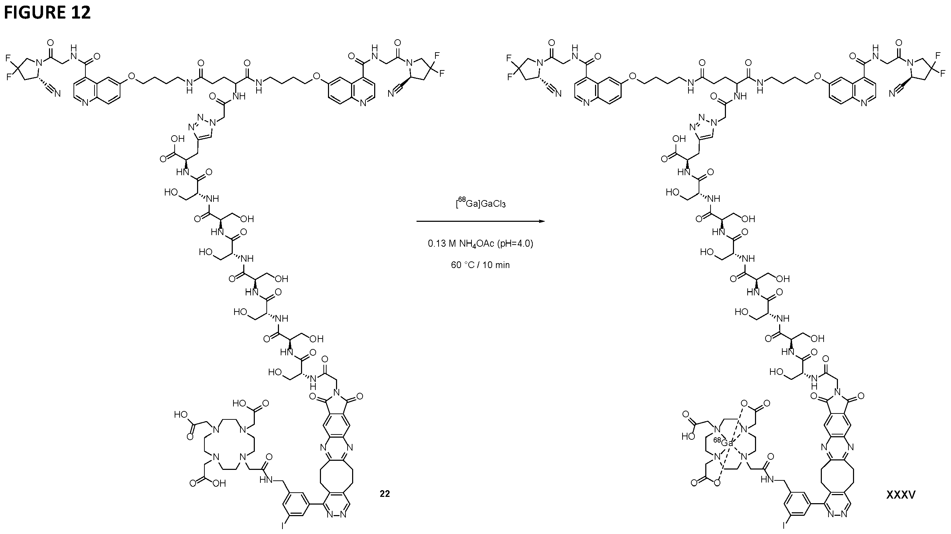

### Example 16: Radiolabeling of compound XXXV (Figure 12)

Figure 12 is a scheme showing the radiolabeling of compound **22 (XXXV).**

Compound **22** (20 µg, 10 µL of 2 mg/mL stock solution in DMSO) and 125 µL 1 M AmOAc (pH = 5.5) was added to a solution of [⁶⁸Ga]GaCl₃ (950µL in 0.1 M HCl, 310 MBq) and was heated/shaken at 60 °C for 10 min (RCC = 91.5%). This was followed by SPE with Sep-Pak tC2 Plus Light cartridge (preconditioning: 2 mL EtOH and 5 mL H₂O; cartridge rinsed with reaction solution, washed with 1 mL H₂O and eluted with 0.5 mL EtOH/ H₂O (1:1) and further diluted with 5 mL 0.1 M sterile phosphate buffer (pH=7.0) + 10 mg/mL ascorbic acid to yield the final product with d.c. RCY = 67% (155 MBq after 30 min synthesis time) and RCP = 98.7% (determined via radio-HPLC: Luna C18 5µ column (150x4.6 mm); mobile phase H₂O/ACN+0.1% TFA (10-50% ACN in 8 min); elution flow 1.5 mL/min) and confirmed via radio-iTLCs (mobile phases: 0.2 M citrate buffer (pH=4.0) and 1 M NH₄OAc(pH=4.0)/MeOH(1:1)).

The radiotracer was injected within 30 min after the synthesis was finished. The RCP of the final formulation after 120 min was determined to be 93.6% via radio-HPLC using the same conditions.

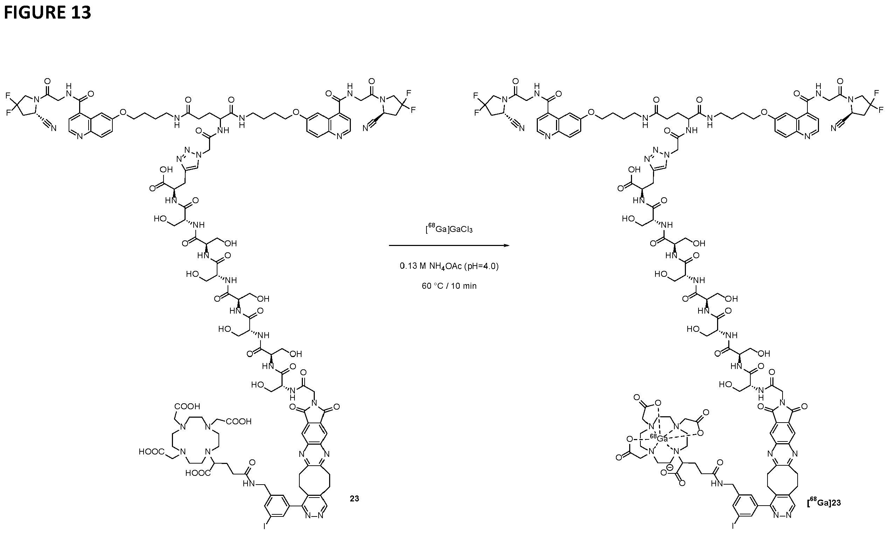

### Example 17: Radiolabeling of compound [⁶⁸Ga]23 (Figure 13)

Figure 13 is a scheme showing the radiolabeling of compound **23** (**[⁶⁸Ga]23**).

Compound 23 (20 µg, 10 µL of 2 mg/mL stock solution in DMSO) and 125 µL 1 M AmOAc (pH = 5.5) was added to a solution of [⁶⁸Ga]GaCl₃ (1000µL in 0.1M HCl, 290 MBq) and was heated/shaken at 60 °C for 10 min (RCC = 91.0%). This was followed by SPE with Sep-Pak tC2 Plus Light cartridge (preconditioning: 2 mL EtOH and 5 mL H₂O; cartridge rinsed with reaction solution, washed with 1 mL H₂O and eluted with 0.6 mL EtOH/ H₂O (1:1) and further diluted with 5.4 mL 0.1 M sterile phosphate buffer (pH=7.0) + 10 mg/mL ascorbic acid to yield the final product with d.c. RCY = 68% (149 MBq after 25 min synthesis time) and RCP = 96.1%

(determined via radio-HPLC: Luna C18 5µ column (150x4.6 mm); mobile phase H₂O/ACN+0.1% TFA (10-50% ACN in 8 min); elution flow 1.5 mL/min) and confirmed via radio-iTLCs (mobile phases: 0.2 M citrate buffer (pH=4.0) and 1M NH₄OAc(pH=3.5)/MeOH(1:1)).

The radiotracer was injected within 30 min after the synthesis was finished. The RCP of the final formulation after 120 min was determined to be 94.7% via radio-HPLC using the same conditions.

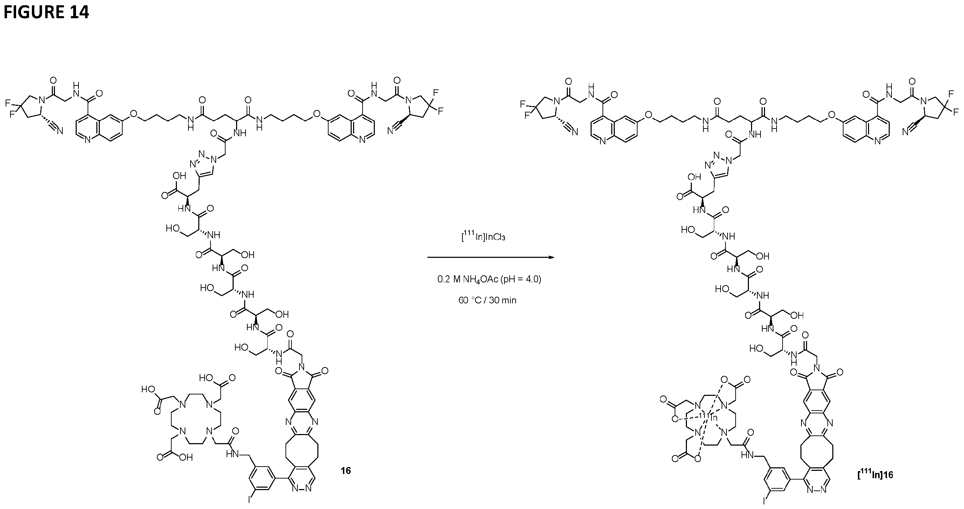

### Example 18: Radiolabeling of compound [¹¹¹In]16 (Figure 14)

Figure 14 is a scheme showing the radiolabeling of **compound 16** (**[¹¹¹In]16**)**.**

Compound **16** (206 µg, 103 µL 2 mg/mL stock solution in DMSO) and 500 µL 1 M NHₐOAc (pH = 4.0) was added to a solution of [¹¹¹In]InCl₃ (2.0 mL, 1.382 GBq) and was shaken at 75 °C for 15 min. More precursor **16** (88 µg, 44 µL 2 mg/mL stock solution in DMSO) was added and heated for another 5 min at 75 °C.

After purification via SPE with Sep-Pak tC2 Plus Light cartridge (preconditioning: 2 mL EtOH and 5 mL H₂O; cartridge rinsed with reaction solution, washed with 1 mL H₂O and eluted with 1 mL EtOH/ H₂O (1:1). The product was obtained with RCY = 80% and RCP = 95.6%, determined via radio-HPLC, (tᵣₑₜ = 4.2 min; RCP = 95.6%, ca. 371 MBq/mL in EtOH/H₂O (1:1)) using Luna C18(2) 5µm 150x4.6 mm column; mobile phase H₂O/ACN + 0.1% TFA (10-50% ACN in 9 min); flow 1.5 mL/min. The product was confirmed via radio-TLCs (mobile phases: 0.2 M citrate buffer (pH=4.0) and 1 M NH₄OAc(pH=4.0)/ MeOH(1:1)). The radiotracer was reformulated in 3 mL saline (371 MBq/mL).

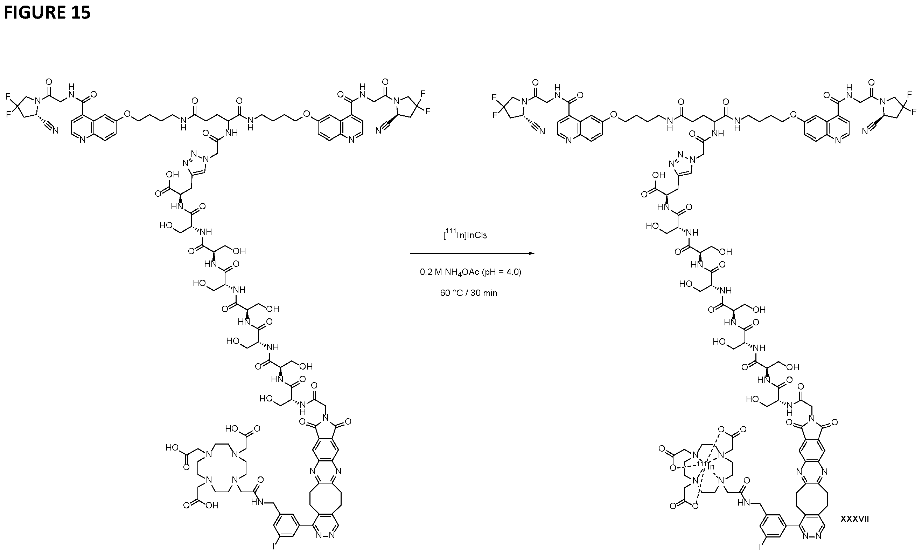

### Example 19: Radiolabeling of compound XXXVII (Figure 15)

Figure 15 is a scheme showing the synthesis of compound **22 (XXXVII).**

Compound **22** (190 µg, 95 µL 2 mg/mL stock solution in DMSO) and 465 µL 1 M NHₐOAc (pH = 4.0) was added to a solution of [¹¹¹In]InCl₃ (1.86 mL, 1.267 GBq) and was shaken at 60 °C for 30 min (RCC = 91.7%). This was followed by SPE with Sep-Pak tC2 Plus Light cartridge (preconditioning: 2 mL EtOH and 5 mL H₂O; cartridge rinsed with reaction solution, washed with 1 mL H₂O and eluted with 1 mL EtOH/ H₂O (1:1). The final product was obtained with RCY = 51% and RCP = 98.4%, determined via radio-HPLC, (tᵣₑₜ = 4.1 min; RCP = 98.4%, ca. 258 MBq/mL in EtOH/H₂O (1:1)) using Luna C18(2) 5µm 150x4.6 mm column; mobile phase H₂O/ACN + 0.1% TFA (10-50% ACN in 9 min); flow 1.5 mL/min. The product confirmed via radio-TLCs (mobile phases: 0.2 M citrate buffer (pH=4.0) and 1 M NH₄OAc(pH=4.0)/ MeOH(1:1)). The radiotracer was reformulated in 2.5 mL saline (258 MBq/mL).

### Examples 20: In vivo SPET/CT imaging and biodistribution of [¹¹¹In]In-labeled FAPi derivatives in tumor bearing mice (u87-mg)

### General information

The biodistribution of radiolabeled FAPi derivatives was investigated in vivo in CD1 nude mice with subcutaneous U87MG tumor xenografts at 7 timepoints after intravenous injection of the radioligands - 1 h, 4 h, 8 h, 24 h, 48 h and 72 h. Three radiolabeled FAPi derivatives were evaluated: [¹¹¹In]**16**, [¹¹¹In]**25** and **XXXVII**. In addition, blocking of FAP-specific tumor uptake by unlabeled ligand UAMC1110 was investigated at 1 h post-injection for all three radiolabeled derivatives.

All experiments were approved by the Ethics Committee of the University of Antwerp, Belgium (file number 2022-63). The use of laboratory rodents was carried out in strict accordance with all mandatory guidelines (EU directives, including the Revised Directive 2010/63/EU on the Protection of Animals used for Scientific Purposes that came into force on 01/01/2013, and the declaration of Helsinki in its latest version).

### Tumor inoculation and follow up

Six- to eight week- old female CD1 nude mice (Charles River) were subcutaneously inoculated in the right hind leg with 6-7 × 10⁶ U87MG cells in 100µL sterile saline. The tumors were allowed to grow until they reached the size of approximately 150 mm³ (9-15 days post-inoculation), whereupon the imaging and biodistribution studies were performed.

### Experimental groups and time points

The mice were split into three cohorts, according to the number of radiolabeled FAPi derivatives tested. Each cohort was further split into groups of 3 animals: 6 groups to investigate baseline biodistribution + 1 group to investigate the effect of FAP blocking with UAMC1110. For [¹¹¹In]**16**, only 5 groups were used for baseline biodistribution.

Radiolabeled FAPi derivatives were injected into the lateral tail vein in 0.2 mL sterile saline. In the FAP blocking groups, the mice received 2 mg/kg (50-55 µg per animal) of UAMC1110 intravenously in sterile saline 1 h before the radioligand injection.

Baseline biodistribution was investigated at 1 h, 4 h, 8 h, 24 h, 48 h and 72 h after radioligand injection. The 8 h timepoint was not investigated for 5S[111In]8. The effect of FAP blocking was investigated at 1 h post-injection for all three radioligands.

Tumor sizes for cohorts and injected activities per experimental group are presented in Table 1.

**Table 1.**

| **Cohort** | **1** | 2 | 3 |
|---|---|---|---|
| Tracer | **XXXVII** | **[^{11I}In]25** | **[¹¹¹In]16** |
| Tumor volume at tracer injection | 220 ± 61 mm³ | 173 ± 75 mm³ | 153 ± 51 mm³ |
| Number of days post tumor inoculation | 15 | 11 | 9 |

| Experimental groups: | Injected activity, MBq (mean ± SD; n = 3) | | |
|---|---|---|---|
| 1h | 15.5 ± 0.3 | 12.0 ± 0.3 | 13.2 ± 0.1 |
| 4h | 18.8 ± 0.1 | 18.2 ± 0.2 | 18.1 ± 0.3 |
| 8h | 19.0 ± 0.5 | 19.3 ± 0.7 | - |
| 24h | 18.8 ± 0.2 | 18.8 ± 0.7 | 18.4 ± 0.1 |
| 48h | 27.6 ± 1.4 | 27.9 ± 0.1 | 27.7 ± 0.5 |
| 72h | 28.2 ± 0.1 | 27.7 ± 0.5 | 23.7 ± 4.8 |

### SPECT/CT imaging and ex vivo dissection

For all timepoints longer than 1 hour post-injection, shortly before the designated timepoint was reached, the mice were anesthetized, placed into the SPECT camera (Vector, Milabs) and imaged for 30 min (8 bed positions, 6 frames of 5 min). For 1 hour timepoint SPECT acquisition (8 bed positions, 12 frames of 5 min) was started immediately after tracer injection. Total body SPECT images were obtained, and a 4 min CT scan was performed at the end of all each SPECT acquisition. At the end of the SPECT/CT scan, blood was collected through cardiac puncture, mice were euthanized by cervical dislocation and dissected for ex vivo gamma counting.

### SPECT/CT image processing

The calibration factor from image gray scale value to activity concentration (kBq/cc) was calculated from a uniform phantom scan with known activity. Raw images from the scanner, reconstructed with OSEM iterative reconstruction (16 subsets, 10 iterations), were then calibrated for activity concentration. Activities in each SPECT frame were decay-corrected relative to the time of injection using the injection time of every animal and start time of every scan frame. Finally, for all scans, all frames were concatenated, also adjusting the time duration of every frame.

SPECT images were analyzed in PMOD software (PMOD Technologies, Zürich, Switzerland). Radioligand uptake was assessed for the tumor, located in the right leg, and muscle in the leg opposite to the tumor. Ellipsoid volumes of interest were drawn over the tumor and muscle. on the organs of interest and adjusted to the size of the organ. Tumor delineation was performed by drawing an iso contour defined by the 50% of the max-min activity range within the volume of interest encompassing the tumor. Uptake was calculated as average % injected dose per mL tissue. For each timepoint, reported tumor uptake values are time-averaged over all frames in the respective acquisition.

### Ex vivo gamma counting

Samples of blood, solid organs, tissues and tumors harvested from mice were weighed, and the radioactivity in the samples was measured using an automatic γ-counter. The uptake levels of the tracers in the organs, tissues and tumors were expressed as percentage of the injected dose per gram (%ID/g).

### Results

Uptake of ¹¹¹In-labeled FAPi derivatives in various tissues and organs of mice determined by ex vivo gamma counting is shown in Tables XA-XC. Biodistribution and excretion profiles of **[¹¹¹In]XXXVII** and **[¹¹¹In]₁₆,** ligands assembled via TCO-tetrazine ligation, are similar to that of the reference ligand **[¹¹¹In]25** ([¹¹¹In]In-DOTAGA.Glu.(FAPi)₂). All three radioligands are primarily excreted via kidneys. Compared to the reference ligand [¹¹¹In]In-DOTAGA.Glu.(FAPi)₂, **[111In]XXXVII** and **[¹¹¹In]16** have considerably higher liver uptake at 1 h post-injection, but at later timepoints liver uptake decreases to same level as the reference ligand.

Figure Y shows tumor uptake of ¹¹¹In-labeled FAPi derivatives. **[¹¹¹In]XXXVII** and **[¹¹¹In]16** showed slightly lower peak uptake in the tumor compared to [¹¹¹In]In-DOTAGA.Glu.(FAPi)₂, but similar long-term retention (Fig. YA) and higher tumor-to-muscle ratios at timepoints of 24 h post-injection and later.

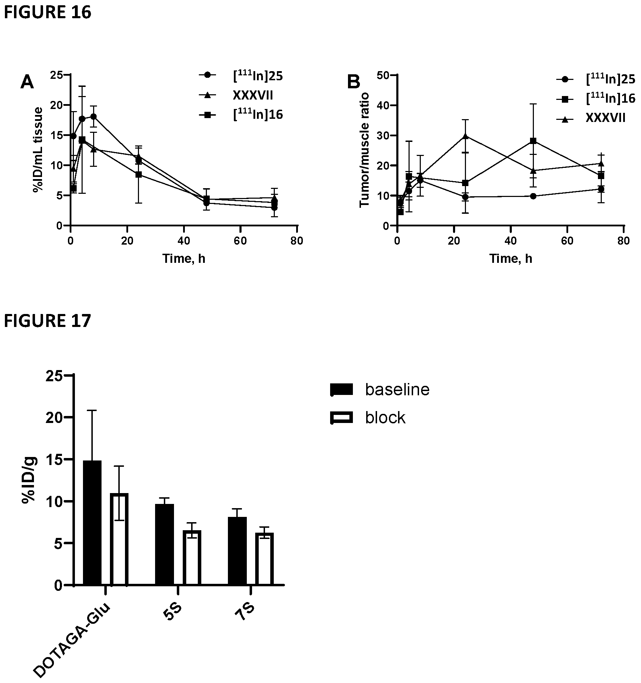

Figure 16 shows the effect of FAP blockade on tumor uptake of ¹¹¹In-labeled FAPi derivatives. Administration of UAMC1110, a selective FAP ligand, led to the decrease in tumor uptake by 24% for [¹¹¹In]In-DOTAGA.Glu.(FAPi)₂, by 32% for **[¹¹¹In]16** and by 23% for **[¹¹¹In]XXXVII** . This indicates that tumor uptake of all three radioligands is dues to specific binding to FAP in the tumor.

Figure 16 shows time-activity curves (A) and tumor-to-muscle ratios (B) for ¹¹¹In-labeled FAPi derivatives determined for U87MG tumor xenografts by SPECT imaging.

Figure 17 shows the effect of FAP blockade (2 mg/kg UAMC1110) on the uptake of ¹¹¹In-labeled FAPi derivatives.

UAMC1110 was administered 1 h before radioligand injection. Tumor uptake is shown at 1 h after radioligand injection.

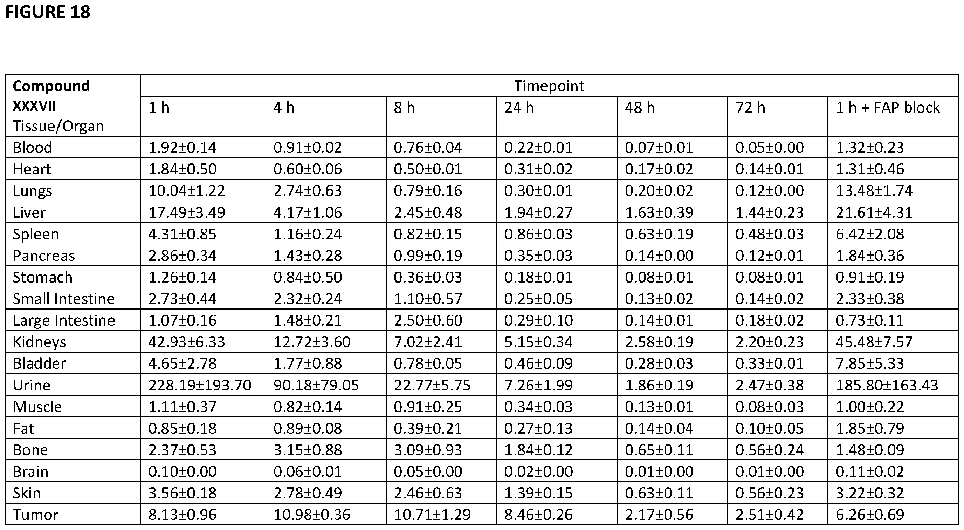

Figure 18 is a table showing the biodistribution (%ID/g) of **[¹¹¹In]XXXVII** based on ex vivo gamma counting results

All data are presented as mean±SD, n=3 for all experimental groups

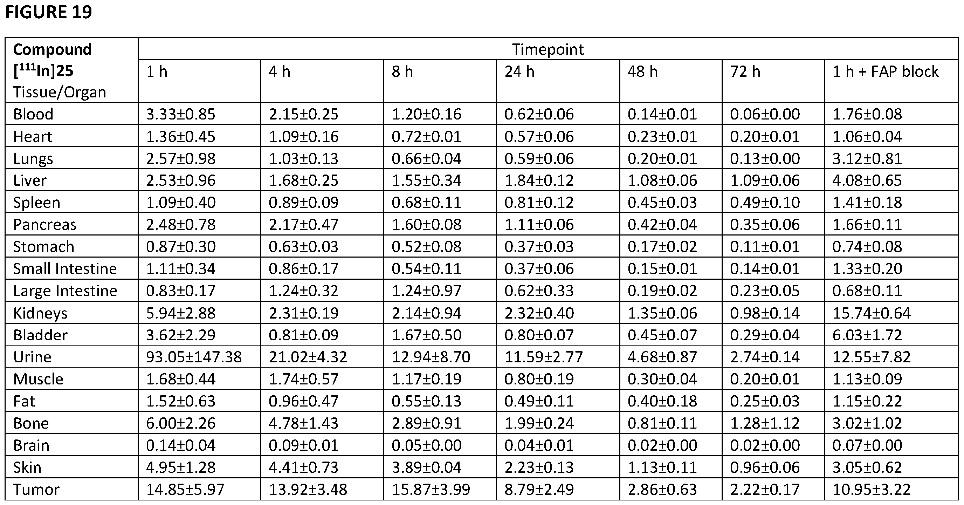

Figure 19 is a table showing the biodistribution (%ID/g) of [¹¹¹In]**25** based on ex vivo gamma counting results

All data are presented as mean±SD, n=3 for all experimental groups

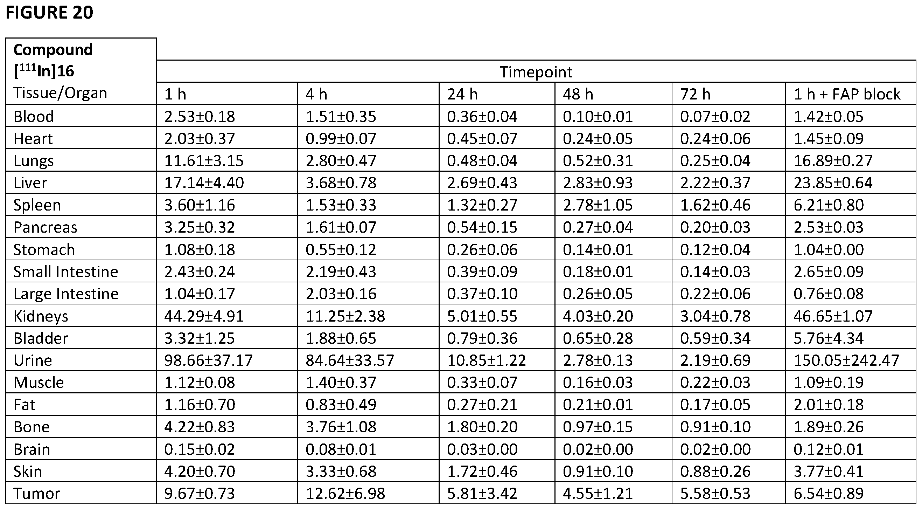

Figure 20 is a table showing the biodistribution (%ID/g) of **[¹¹¹In]16** based on ex vivo gamma counting results

All data are presented as mean±SD, n=3 for all experimental groups

## Claims

1. FAP inhibitor ligand of formula (I): 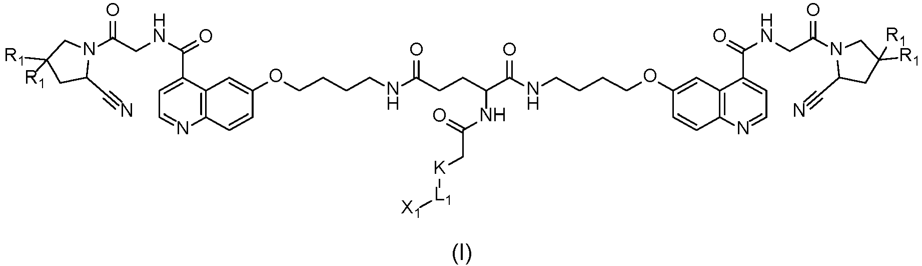 wherein:
R₁ is H or F
K is 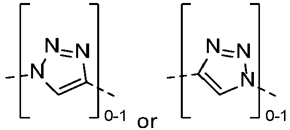
L₁ is selected from:
-(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
wherein n and m are integers independently selected from 0-20,
and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, - COCH(CH(OH)CH₃)NH-, -COCH(CH₂SeH)NH-, -COCH(CH₂C₈H₆N)NH-, - COCH(CH₂C₆H₄OH)NH-, -COCH(CH₂CH₂SeCH₃)NH-;
X₁ is a pyridazine selected from the group consisting of: 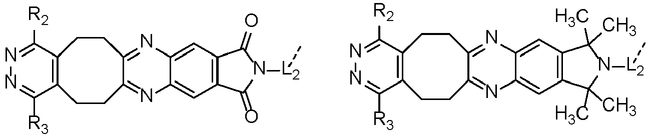 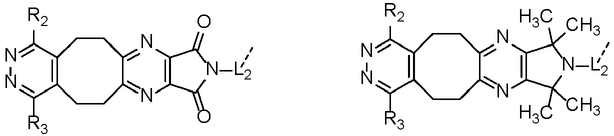 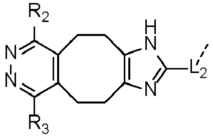
wherein L₂ is -CO(CH₂)ₙ- or -CO(CH₂CH₂O)ₙ(CH₂)ₘ-,
wherein n and m are integers independently selected from 1-10, and
R₂ is selected from -H, -Me, and R₃;
R₃ is 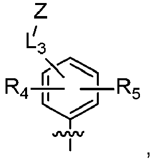 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, -NHCH₂CH₂Y₁, - OCH₂C₆H₄Y₁, -OCH₂CH₂C₆H₄Y₁, -NHCOCH₂CH₂Y₁, -CONHCH₂CH₂Y₁, -NHSO₂CH₂CH₂Y₁, - SO₂NHCH₂CH₂Y₁,
wherein Y₁ is selected from the group constisting of:
¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At, ²¹¹At,
and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, - NHSO₂(CH₂)ₙ(OCH₂CH₂)ₘNH-, -CONH(CH₂)ₙ(OCH₂CH₂)ₘNH-, - NHCO(CH₂)ₙ(OCH₂CH₂)ₘNH-, -CH₂(OCH₂CH₂)(CH₂)ₘNH-, -OCH₂(OCH₂CH₂)ₙ(CH₂)ₘNH-, -SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, -NHSO₂CH₂CH₂(OCH₂CH₂)ₙ(CH₂)ₘNHCO-, - CONHCH₂CH₂(OCH₂CH₂)ₙ(CH₂)ₘNHCO-, -NHCOCH₂CH₂(OCH₂CH₂)ₙ(CH₂)ₘNHCO-,
where n and m are independently selected from the group consisting of 0-25;
and wherein Z is a chelator selected from: -H, 1,4,7,10-tetraazacyclododecane-*N*,*N'*,*N'*,*N"-*tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N*,*N'*-bis(2-hydroxy-5-(carboxyethyl)benzyl)ethylenediamine *N*,*N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
and wherein Z is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
and wherein R₅ is selected from a -H, a halogen, -OH, -OMe, -OEt, -OPr, -NHCOH, - NHCOMe, NHCOEt, -NHCOPr, -CONH₂, -CONHMe, -CONHEt, -CONHPr, NHSOH,-NHSO₂Me, NHSO₂Et, -NHSO₂Pr, -SO₂NH₂, -SO₂NHMe, -SO₂NHEt, -SO₂NHPr, -NH₂, -NHMe, -NHEt, -NHPr;
and pharmaceutically acceptable salts thereof.

2. FAP inhibitor ligand according to claim 1, wherein A is a polar natural amino acid or its D-enantiomer.

3. FAP inhibitor ligand according to claim 1 or 2, wherein Y₁ is a radionuclide selected from ³H, ¹¹C, ¹⁸F, ¹²³I, ¹³¹I, ²¹⁰At and ²¹¹At.

4. FAP inhibitor according to claim 3, wherein Y₁ is ¹³¹I or ²¹¹At.

5. FAP inhibitor ligand according to claims 1 or 2, wherein the chelator Z is labeled with a metal selected from ⁶⁴Cu, ⁶⁷Cu, ⁶⁸Ga, ⁹⁰Y, ⁸⁹Zr, ^{99m}Tc, ¹¹¹In, ¹⁷⁷Lu, ²⁰³Pb, ²¹²Pb, ²²⁵Ac.

6. FAP inhibitor ligand according to any of claims 1 - 3 selected from the group consisting of compounds **I-XXVIII:**

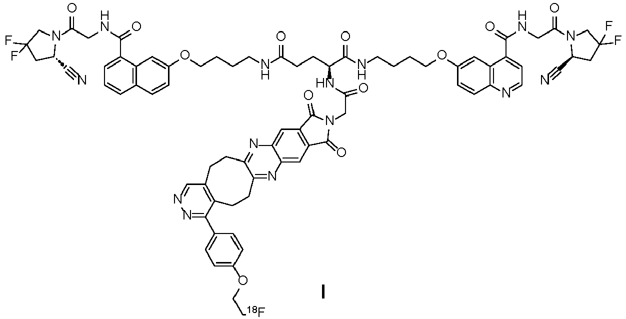

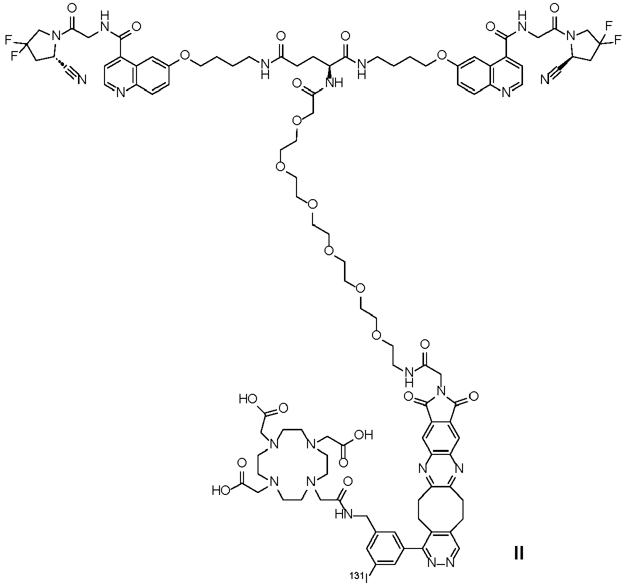

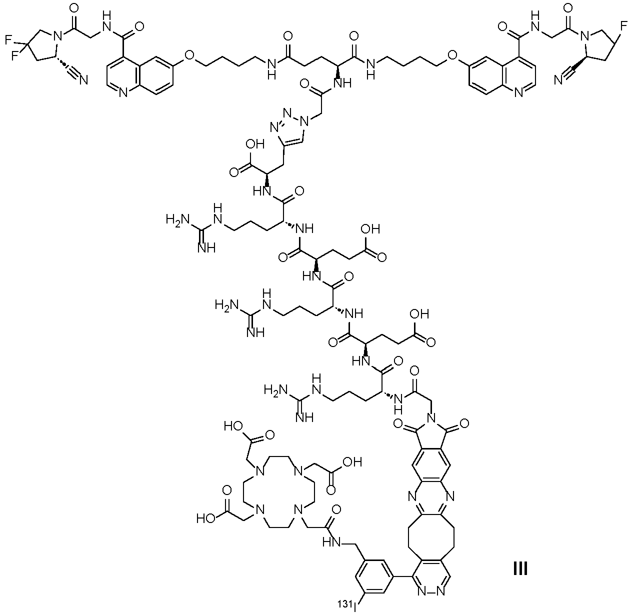

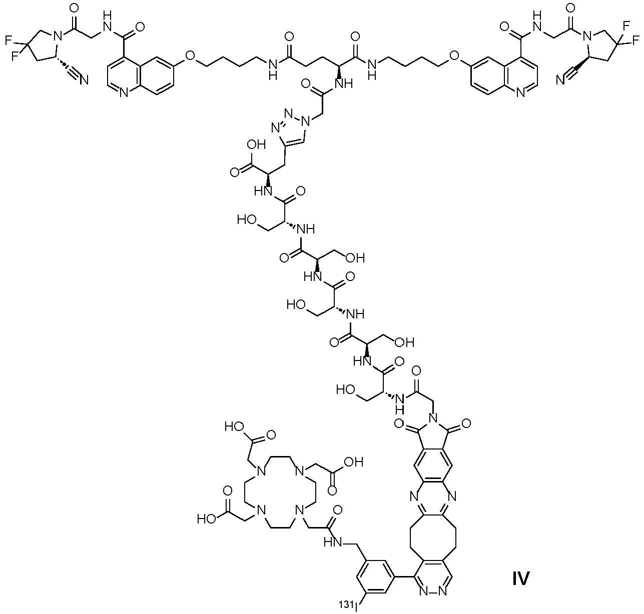

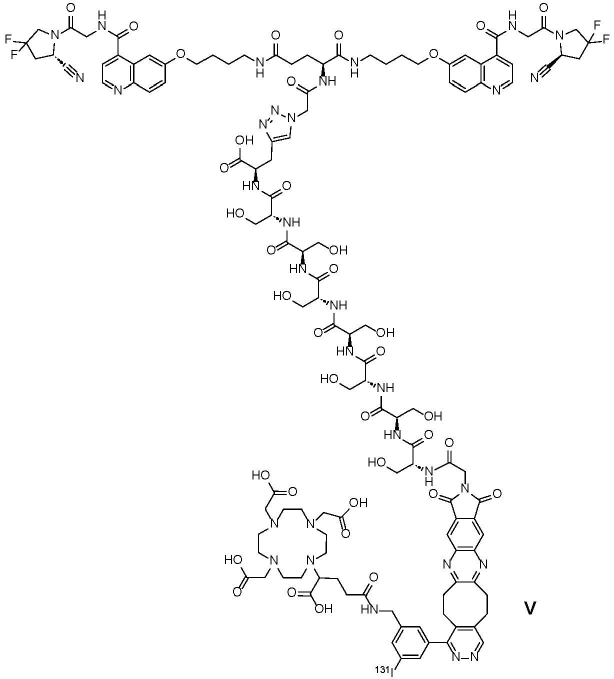

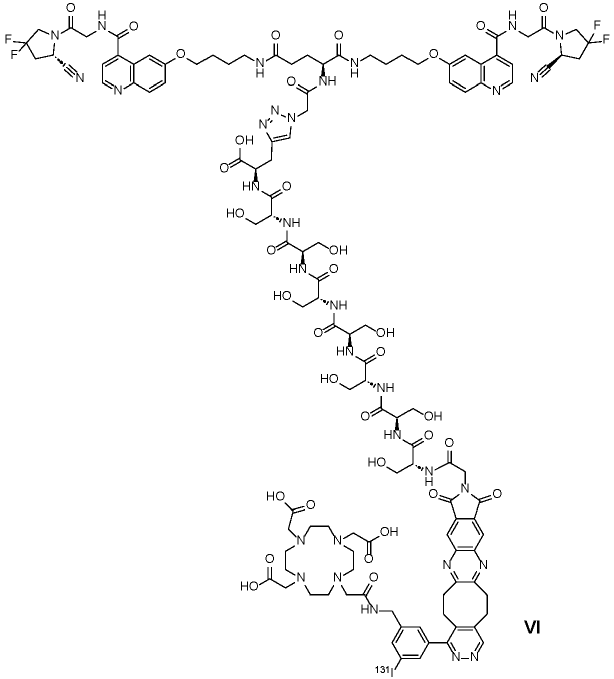

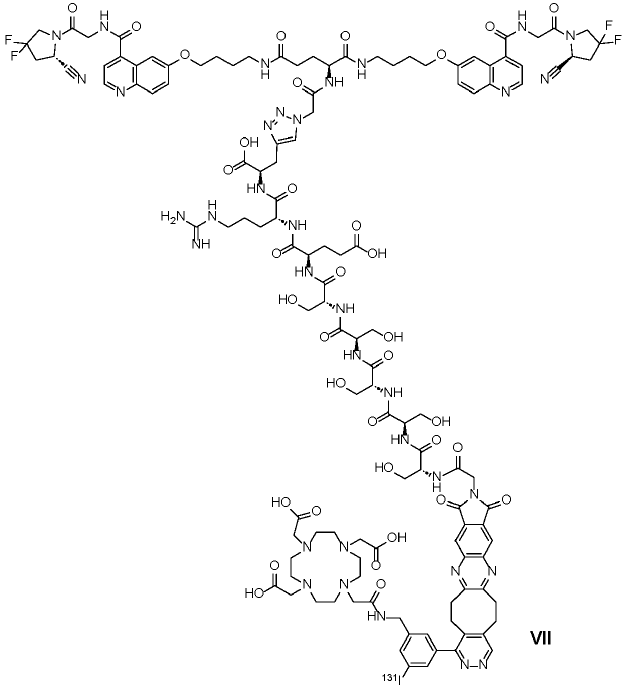

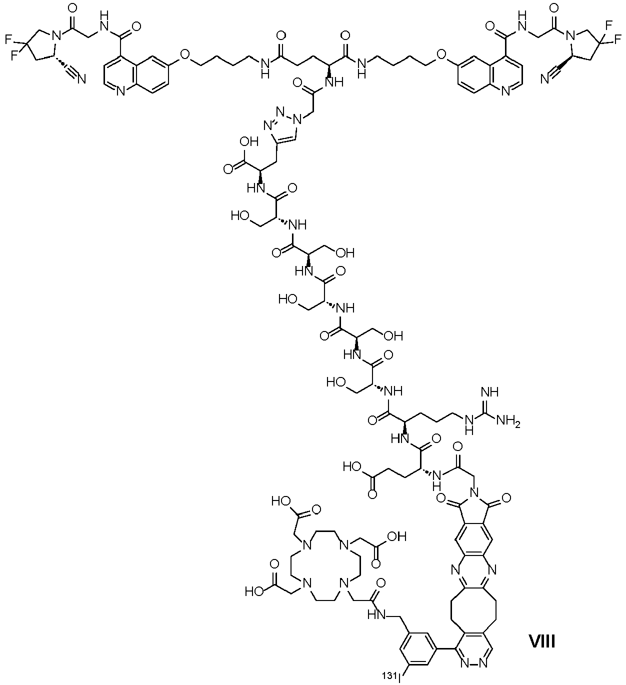

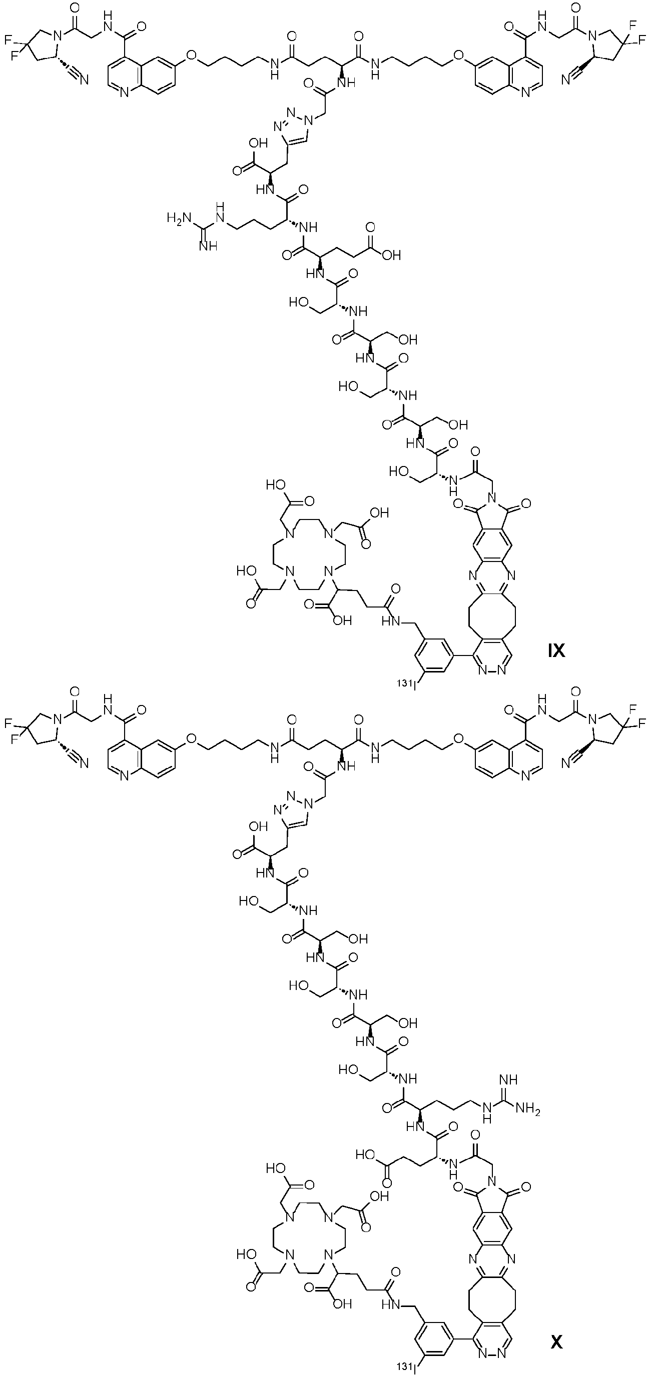

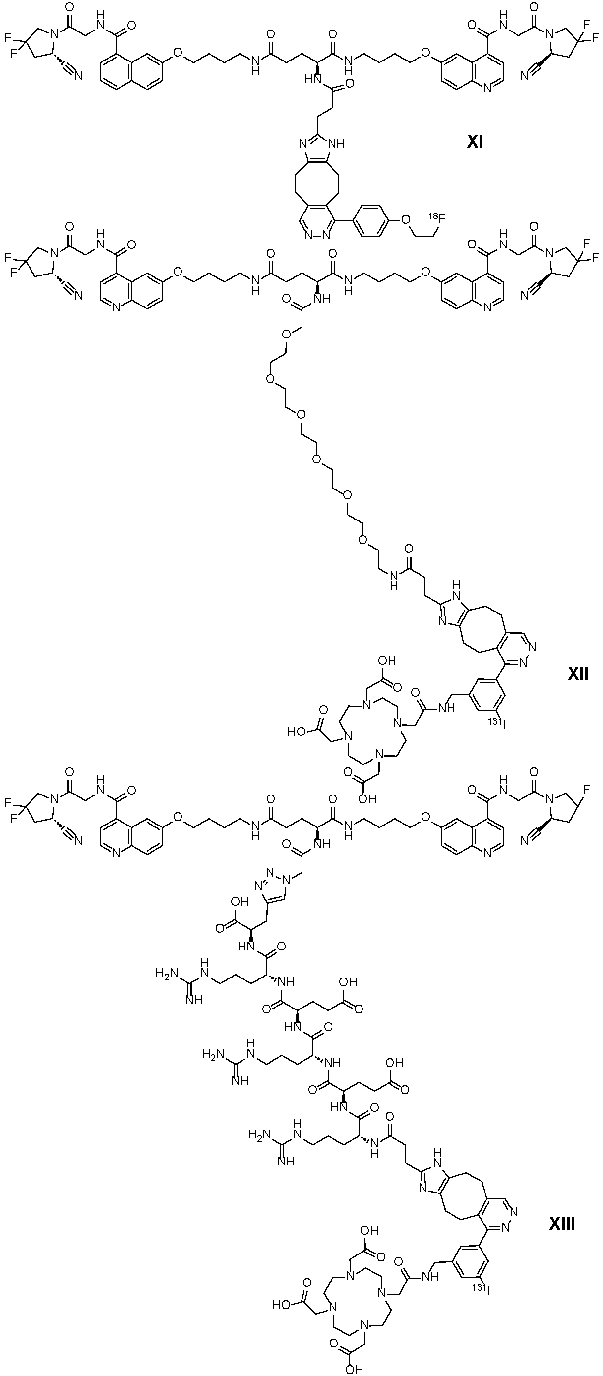

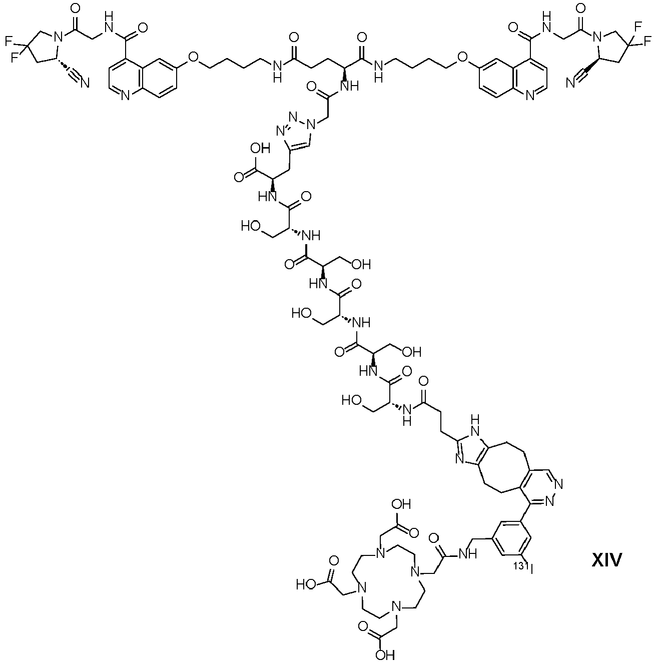

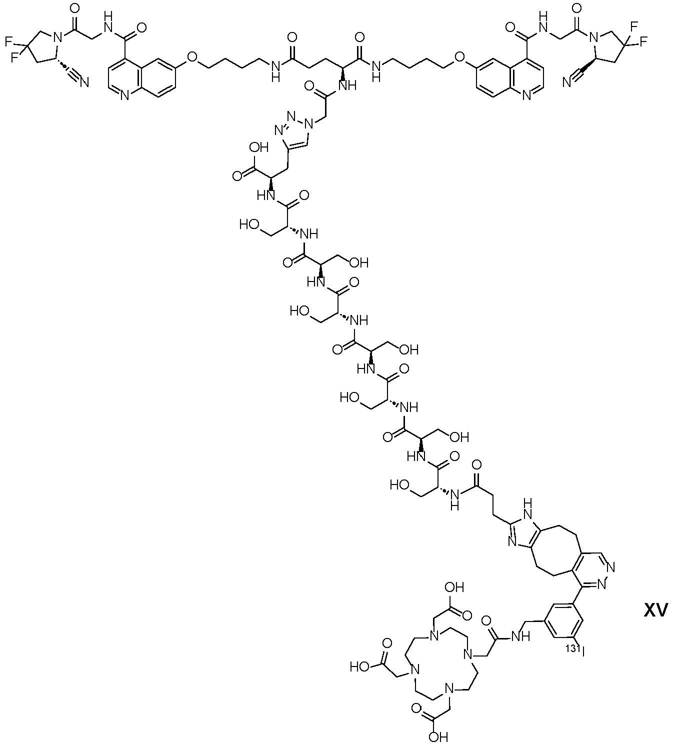

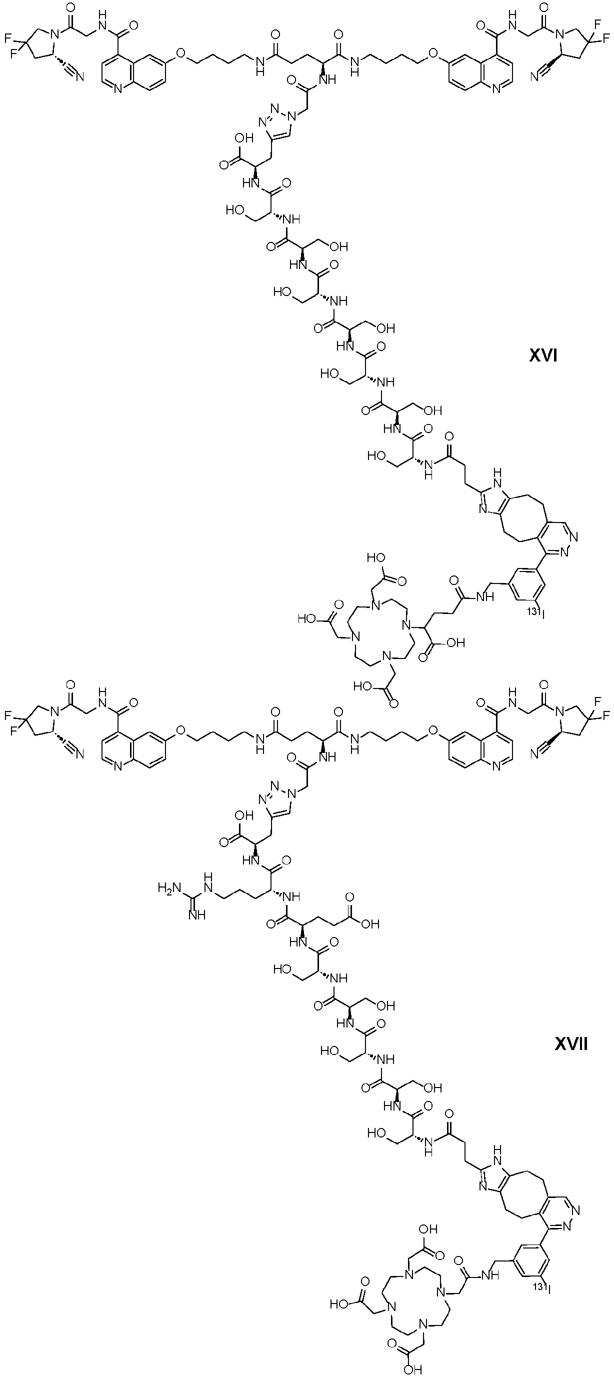

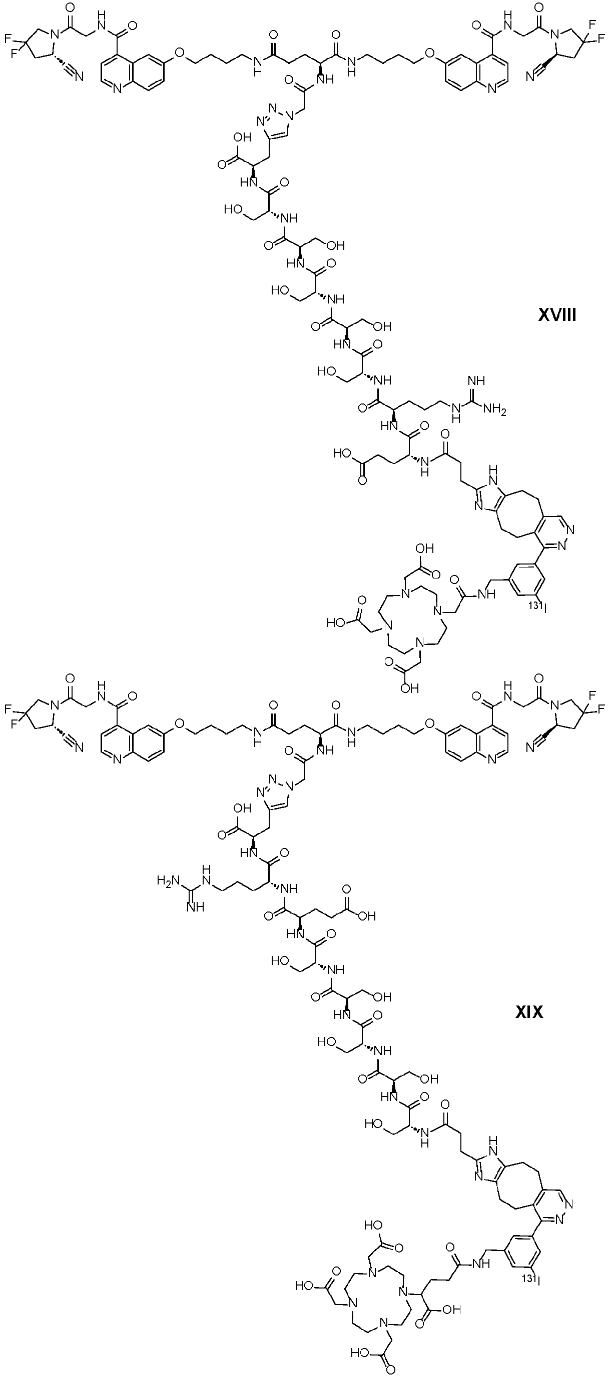

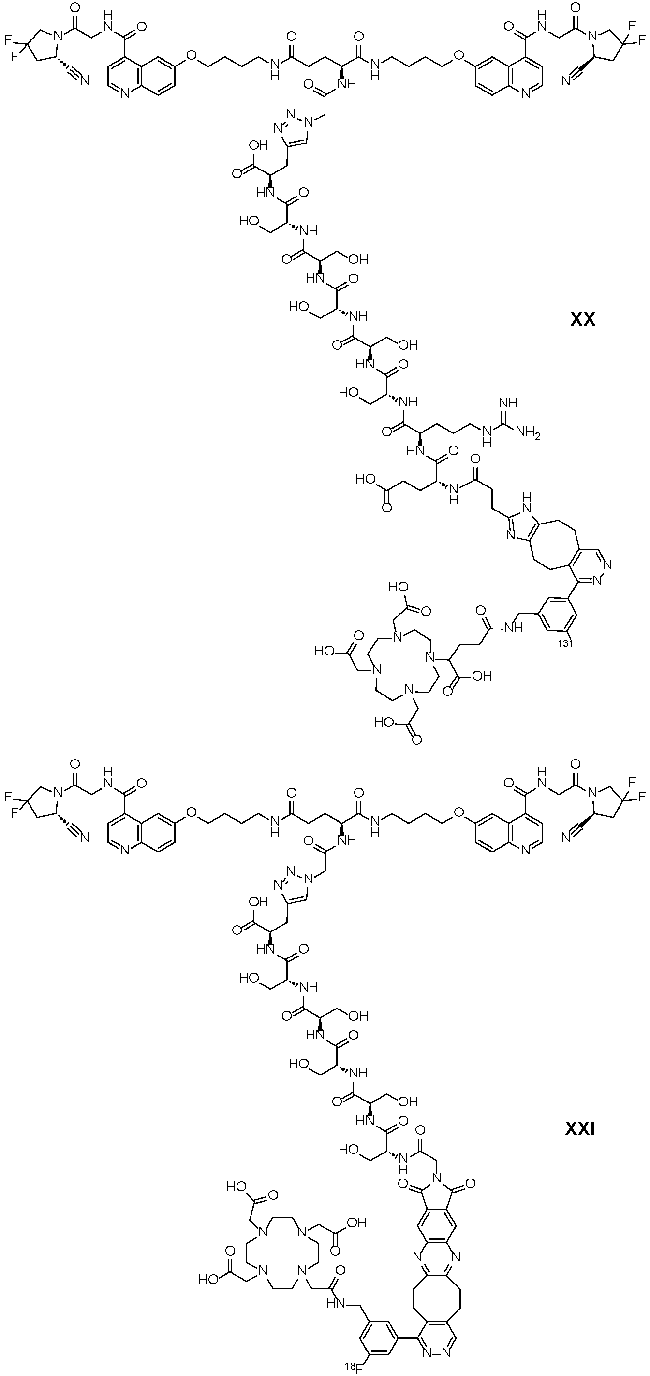

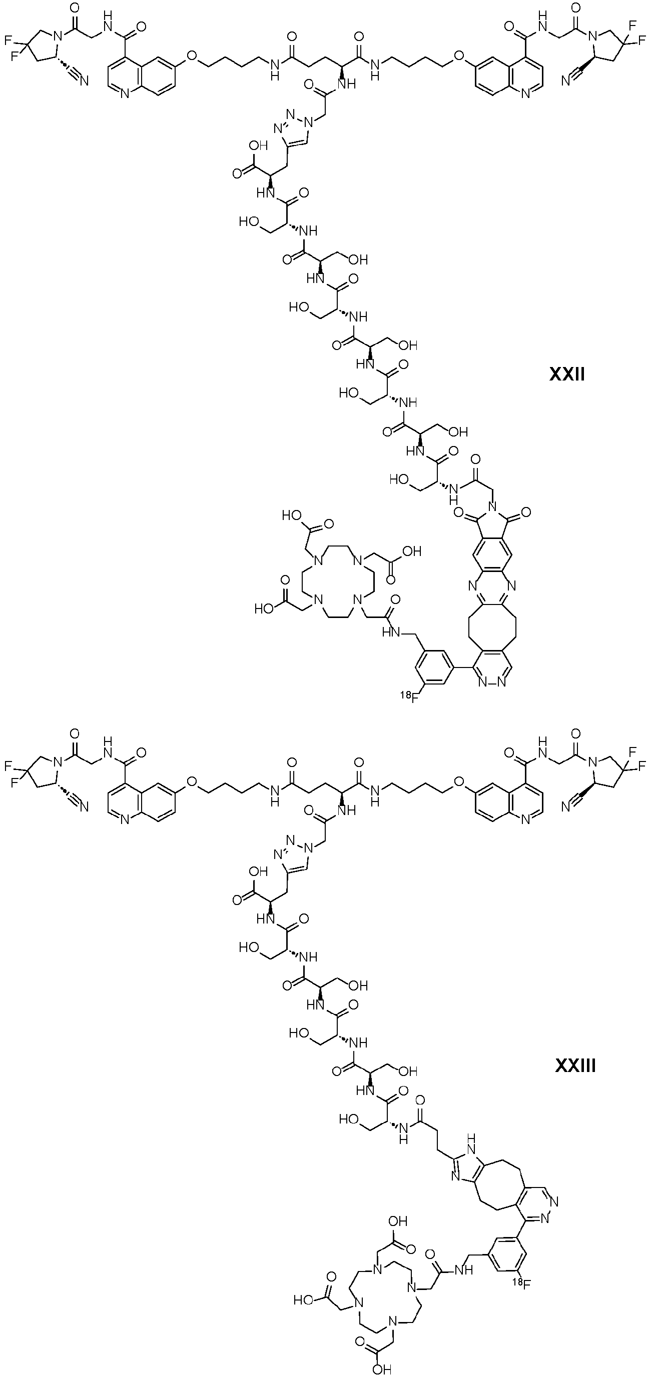

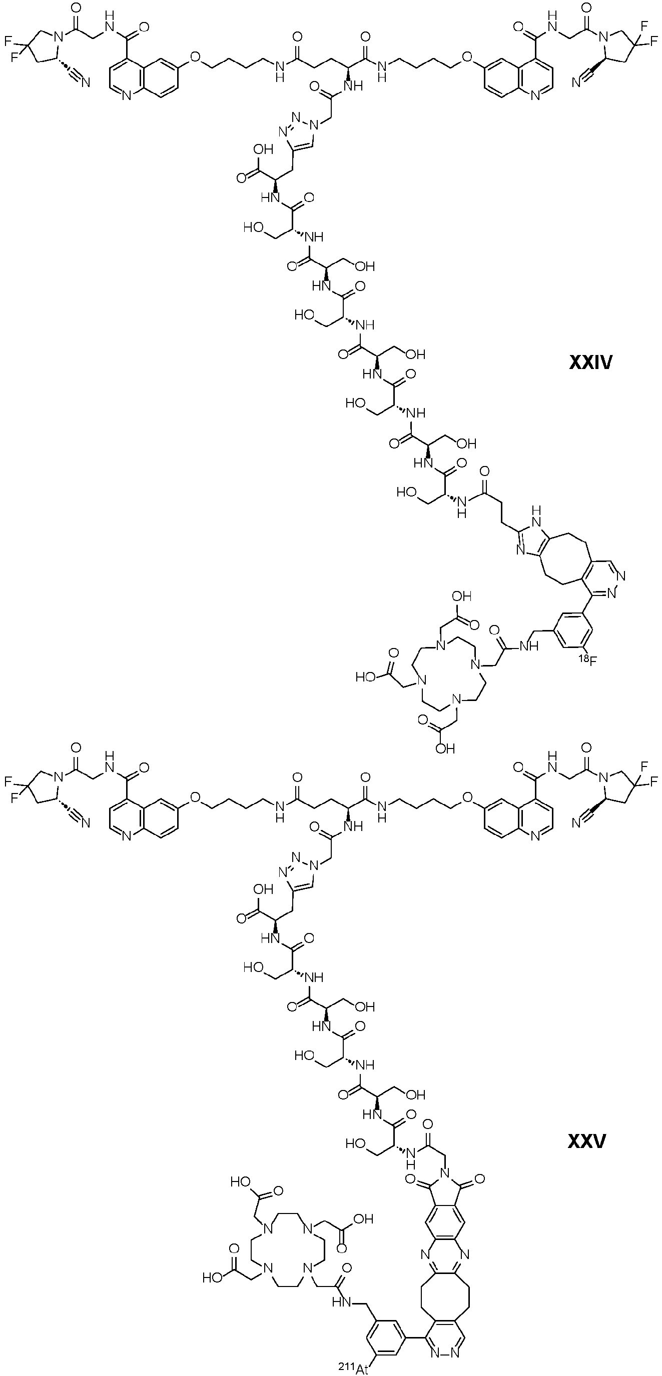

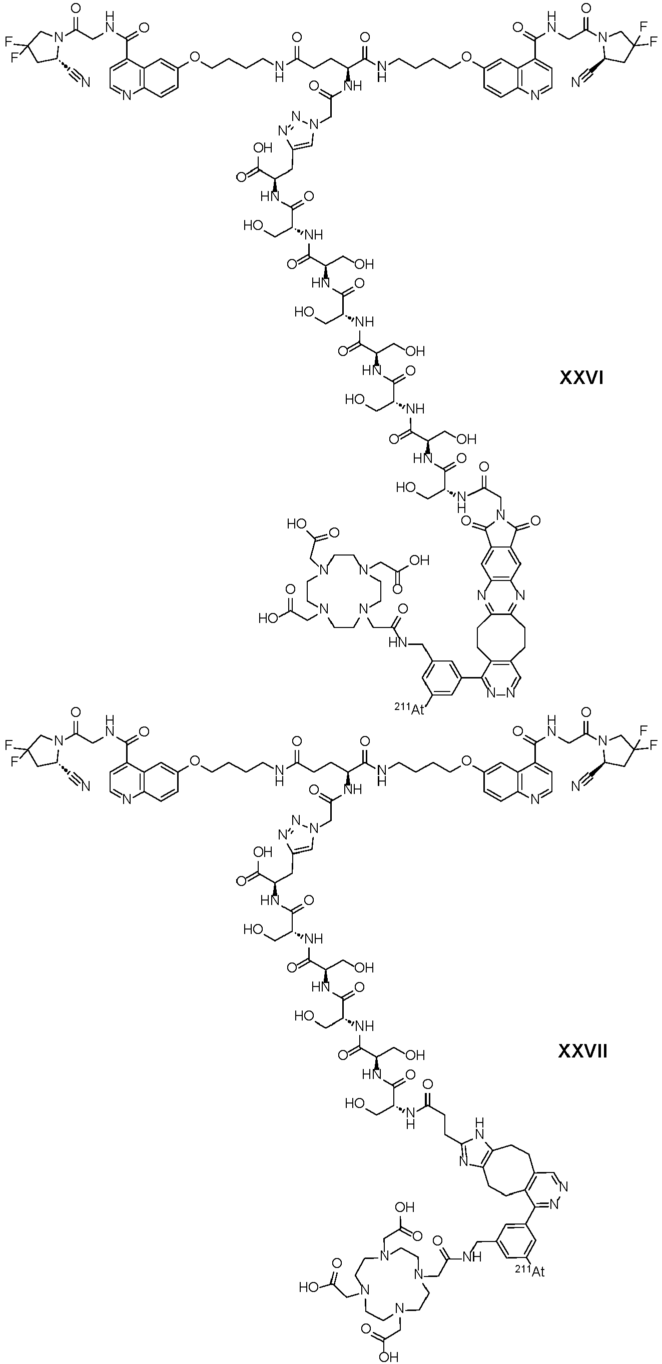

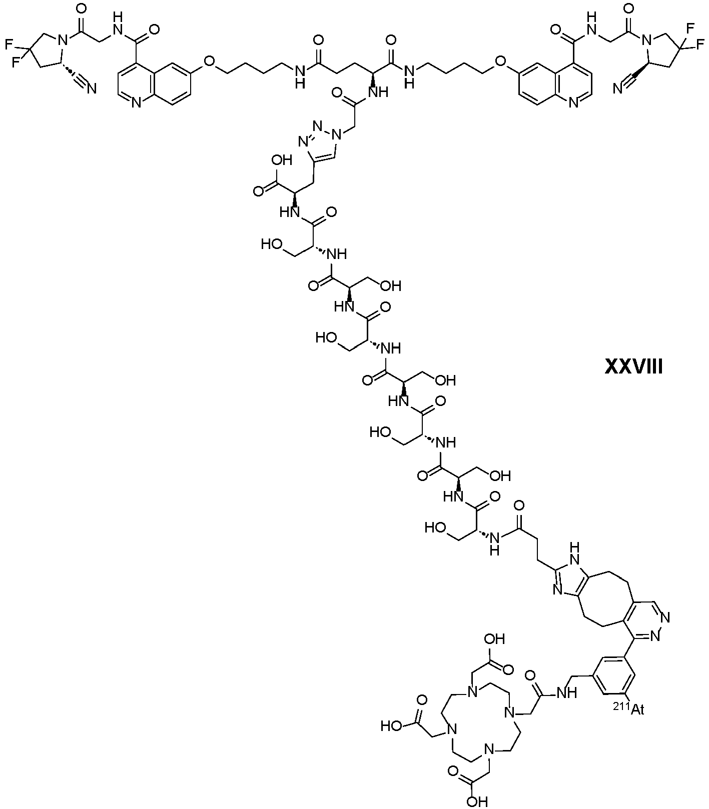

7. FAP inhibitor ligand according to claim 1, 2 or 5 selected from the group consisting of:

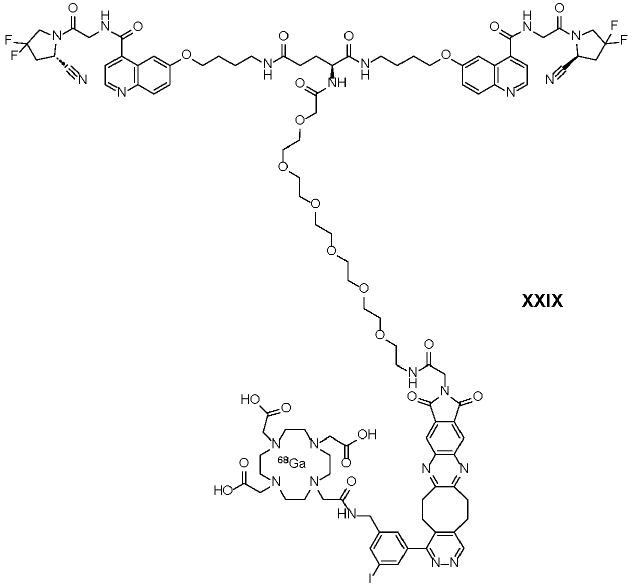

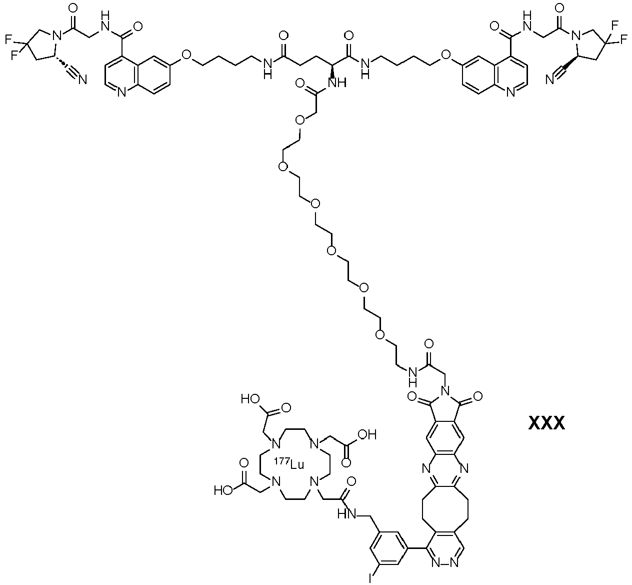

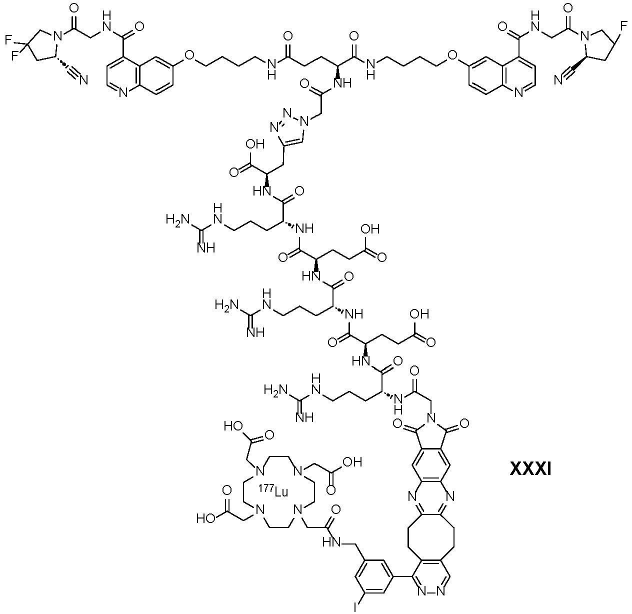

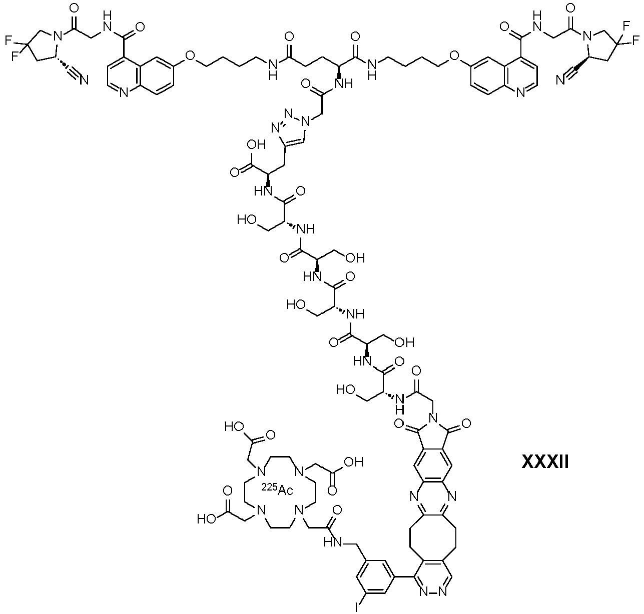

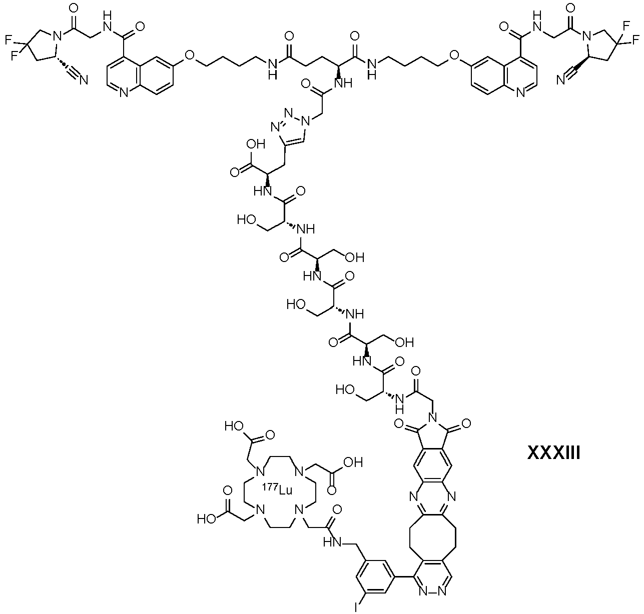

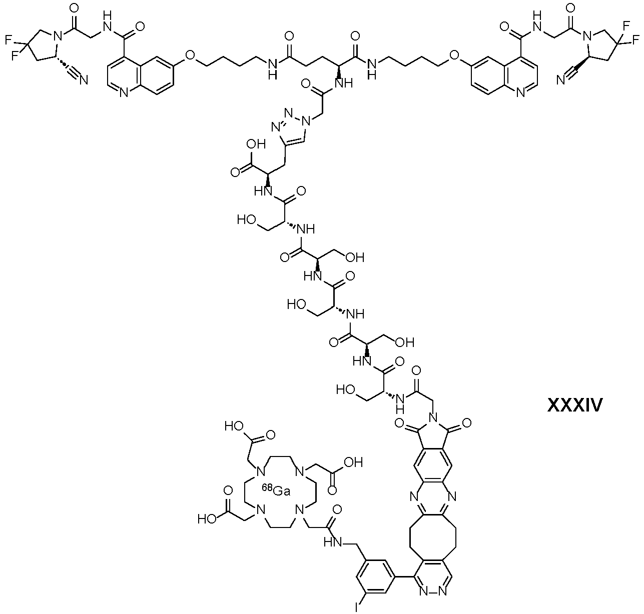

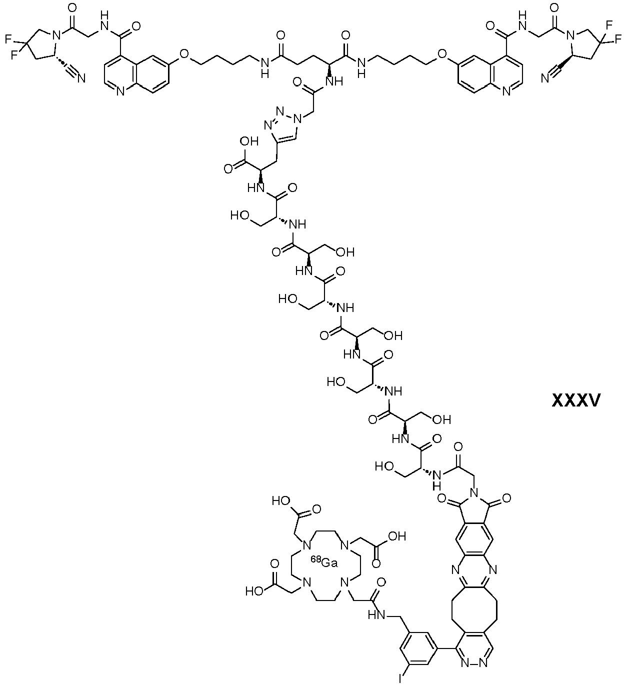

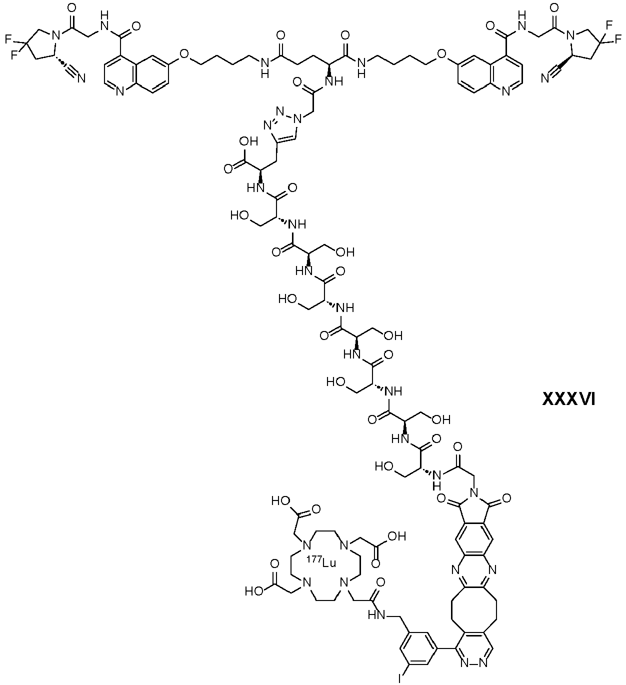

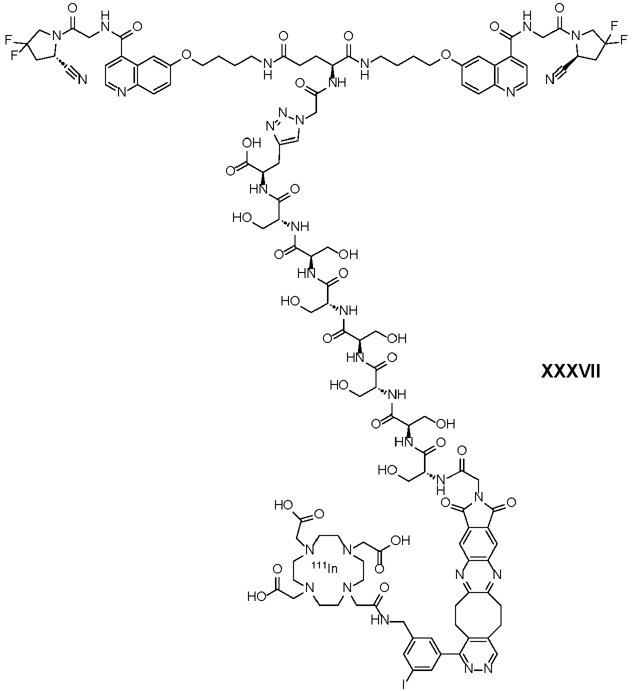

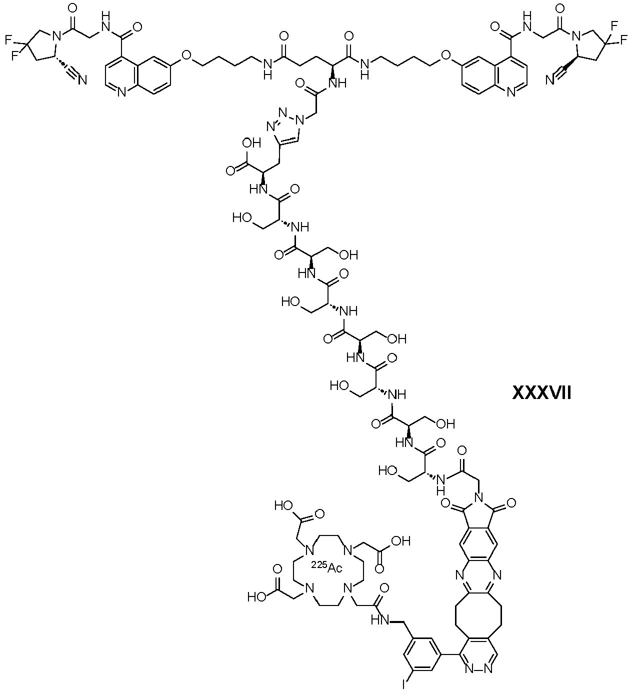

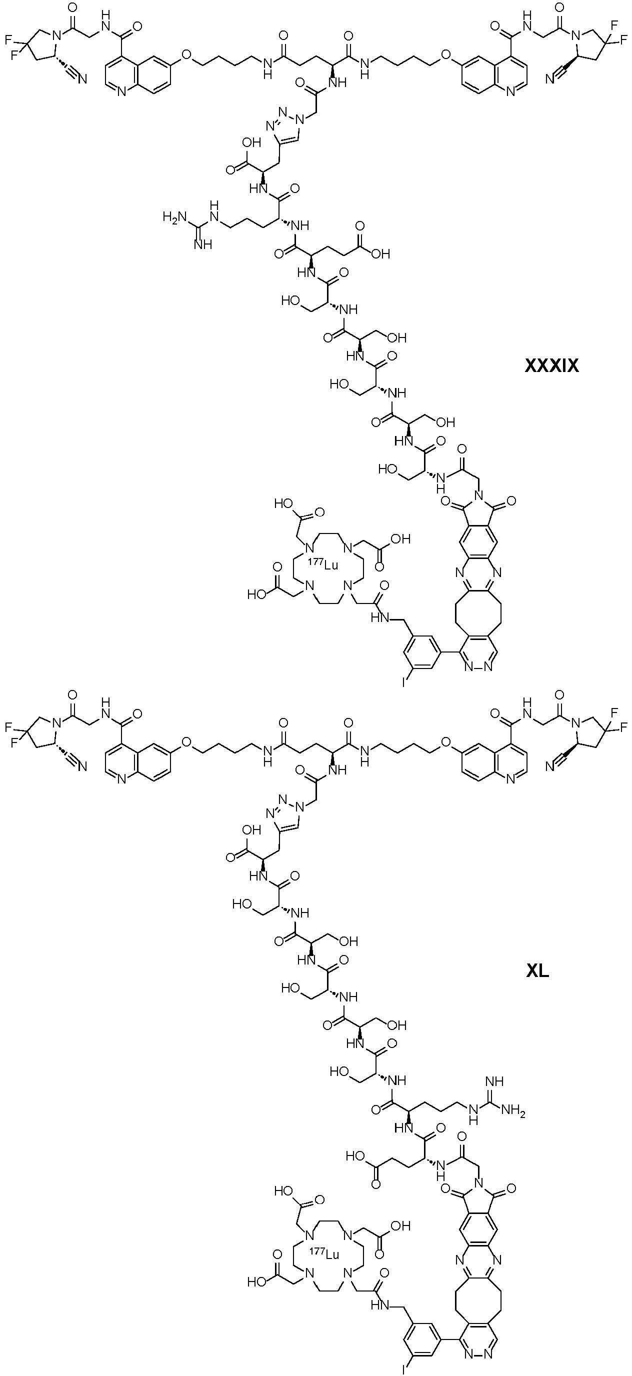

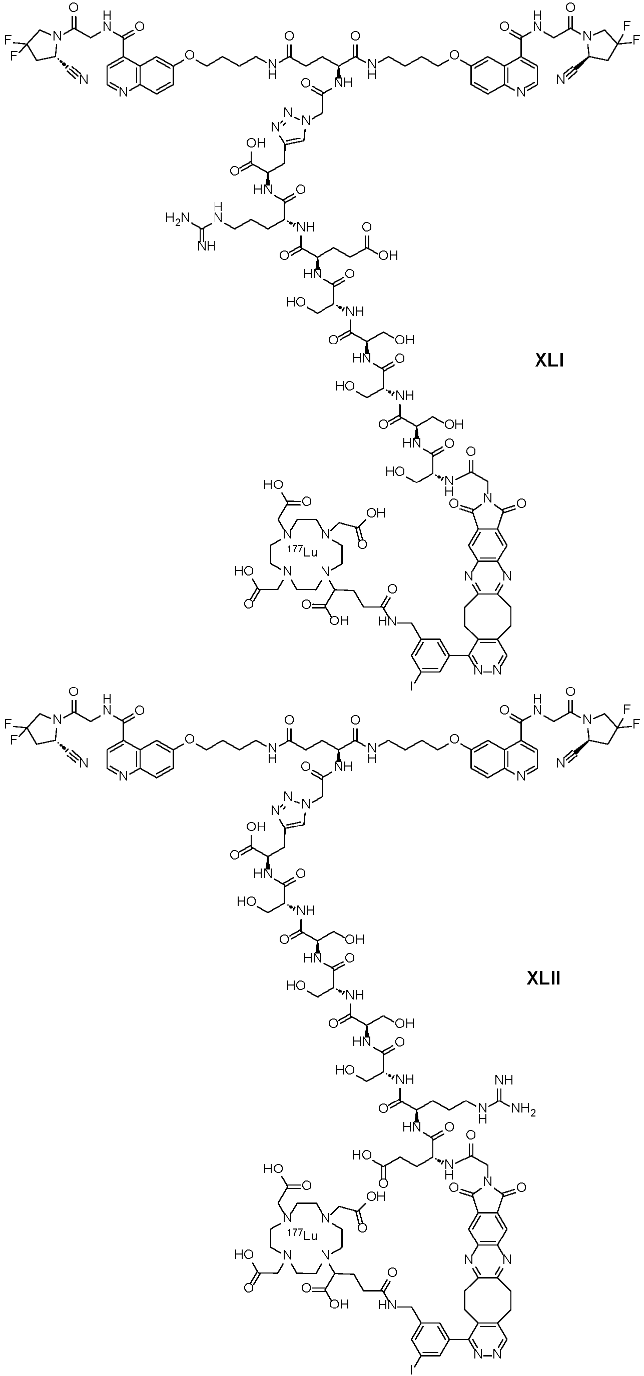

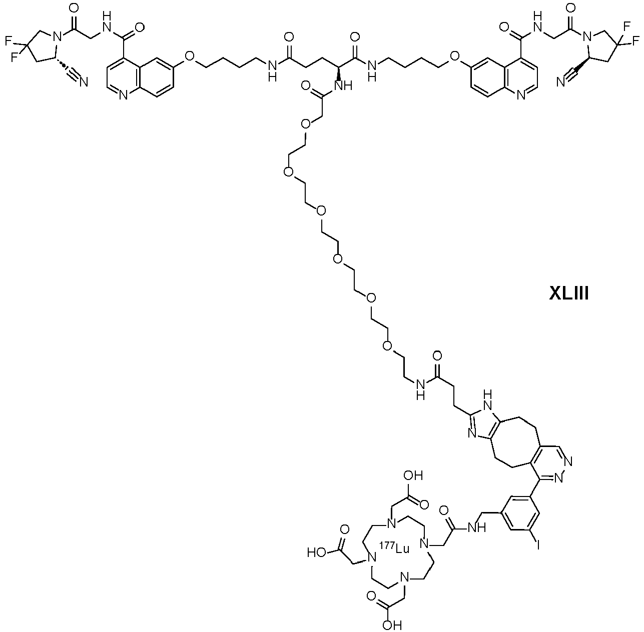

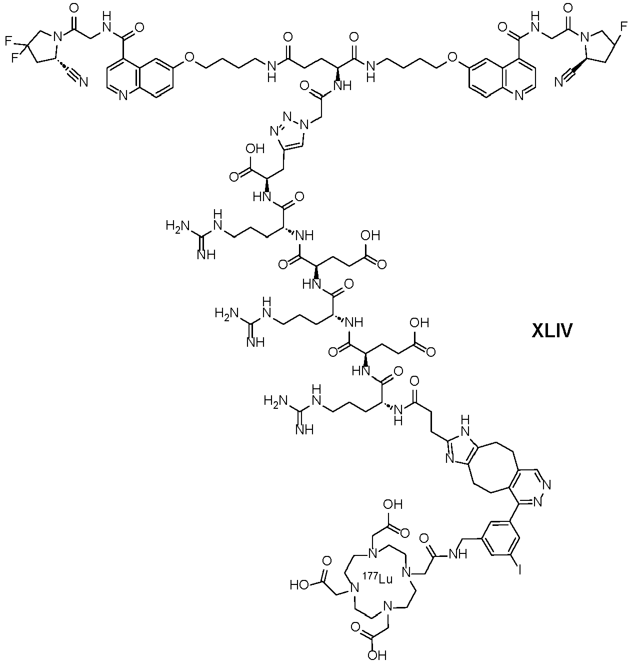

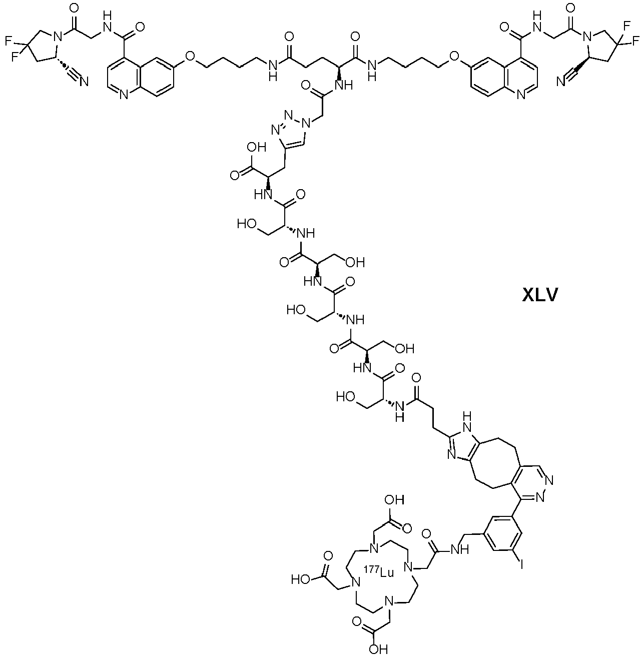

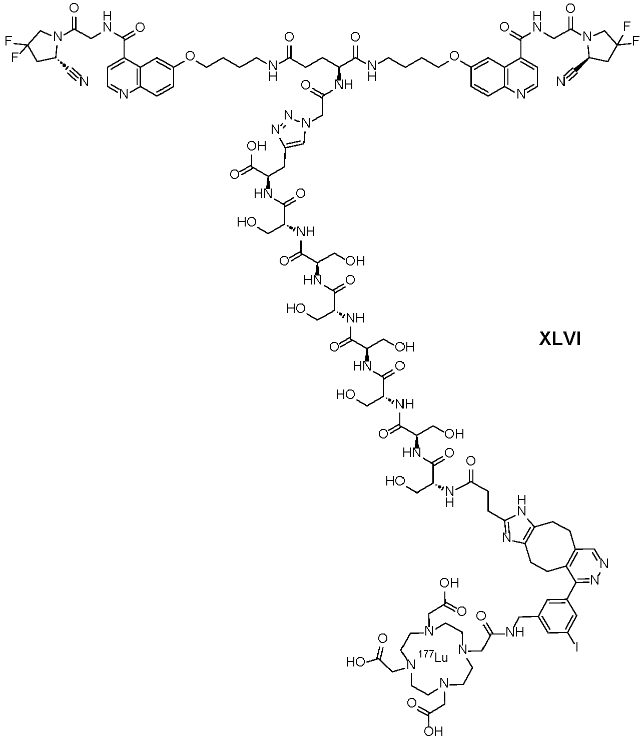

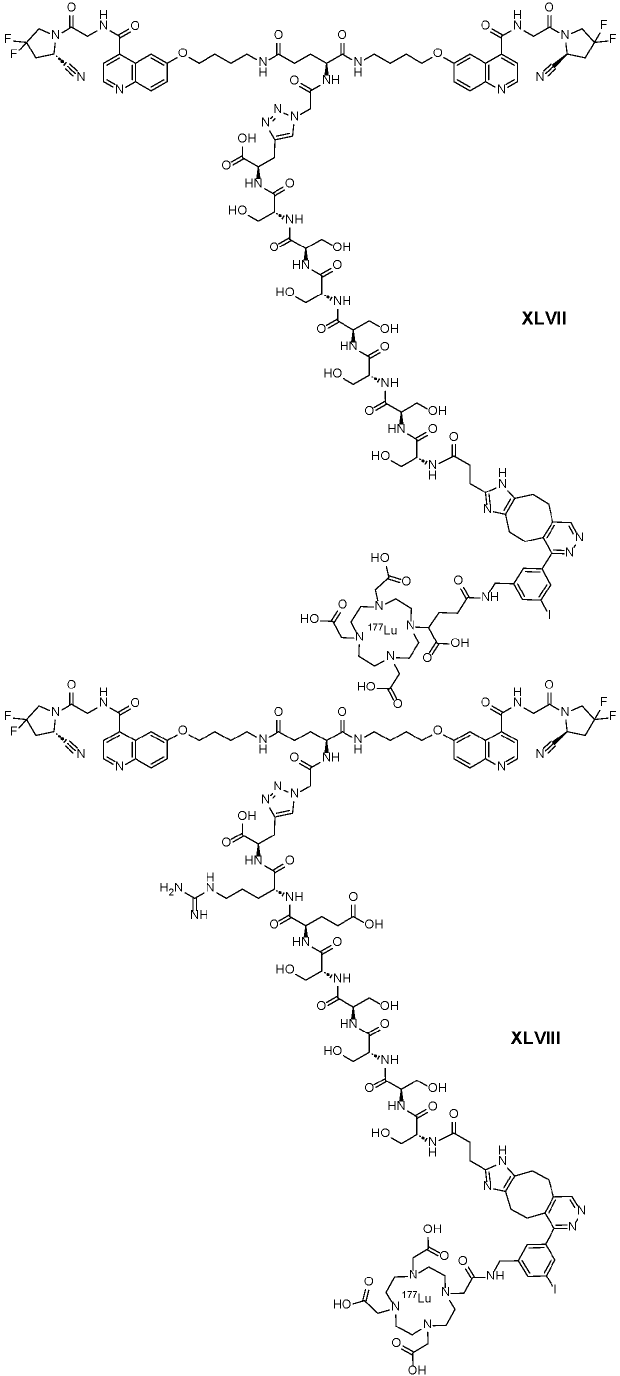

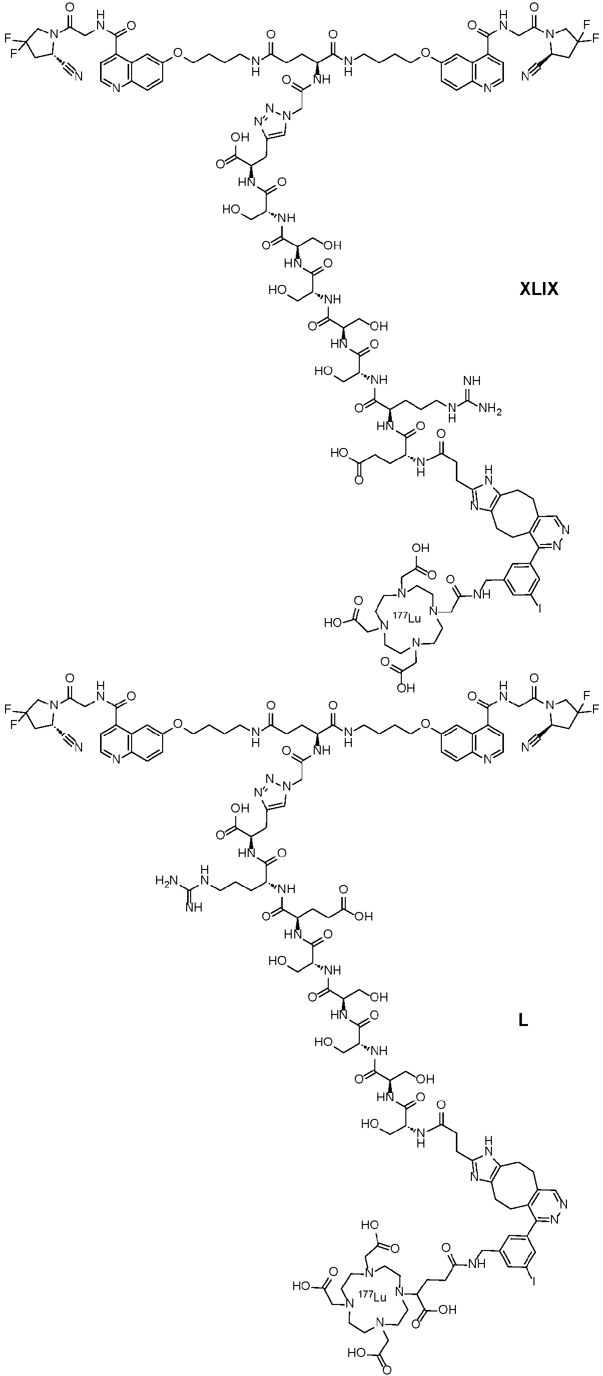

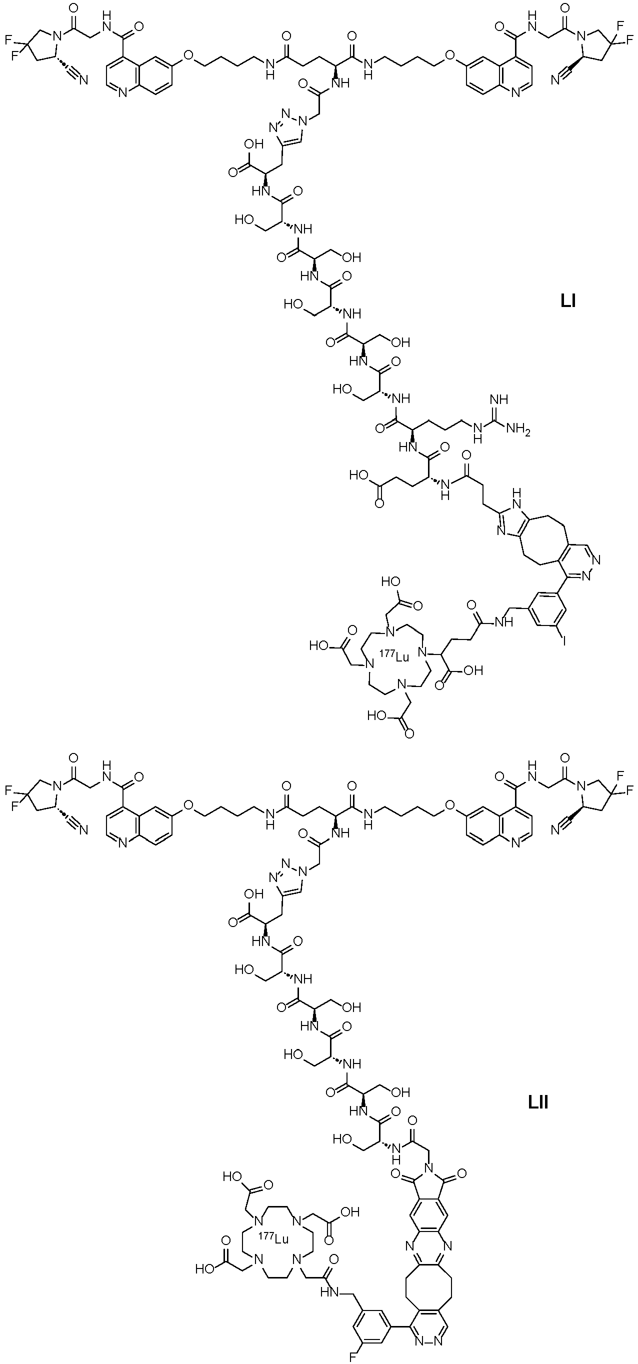

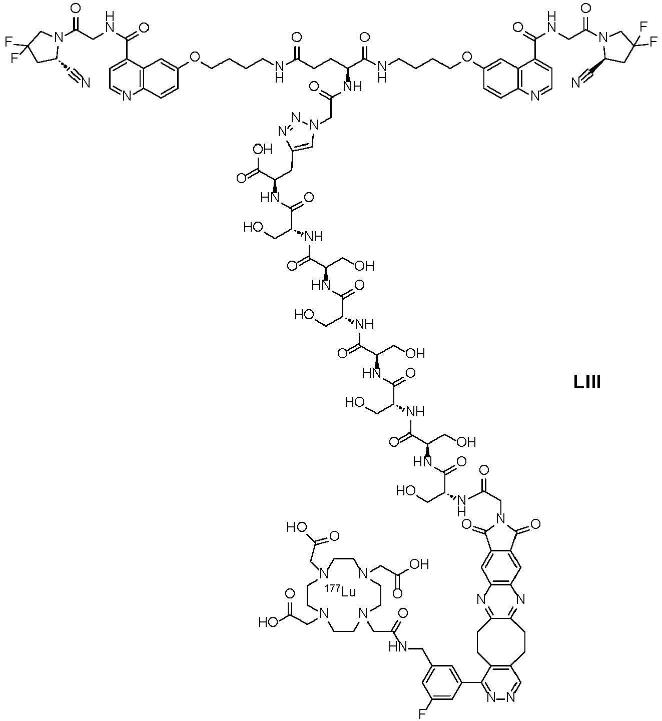

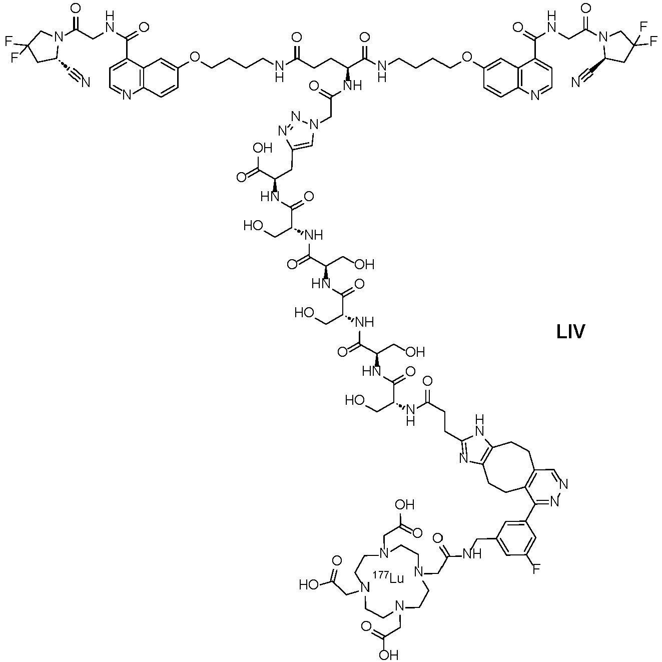

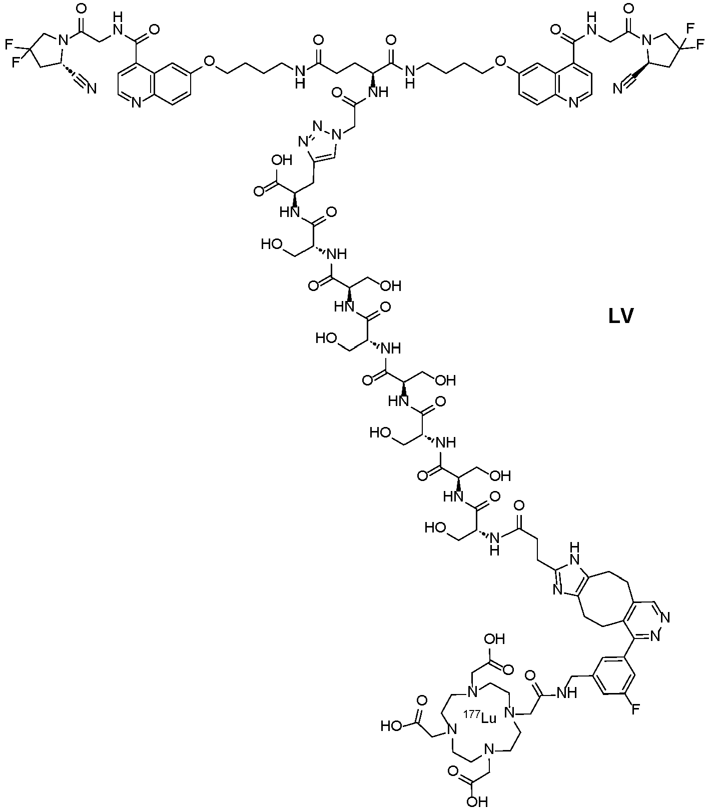

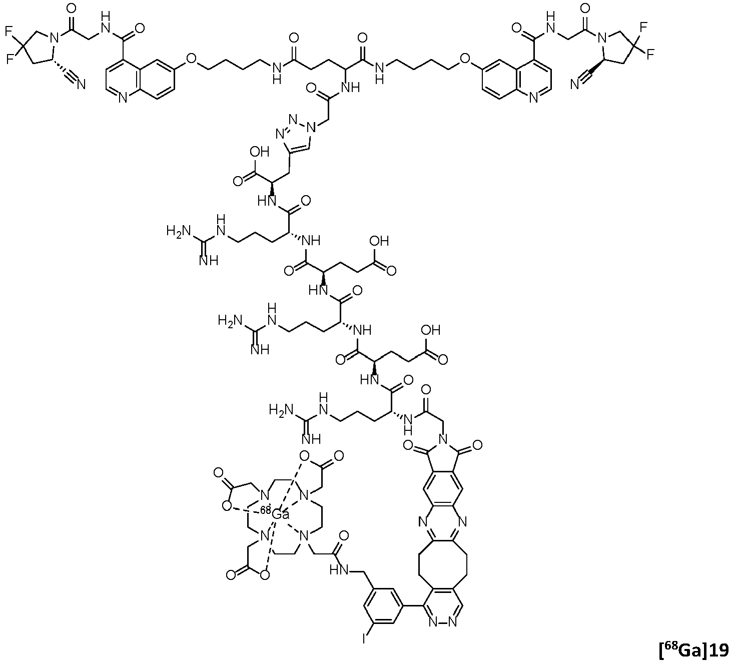

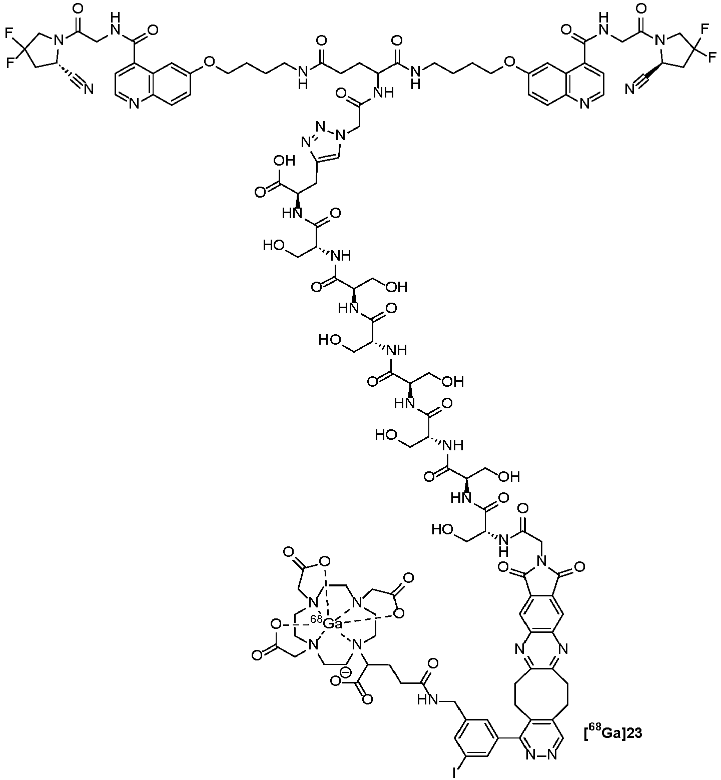

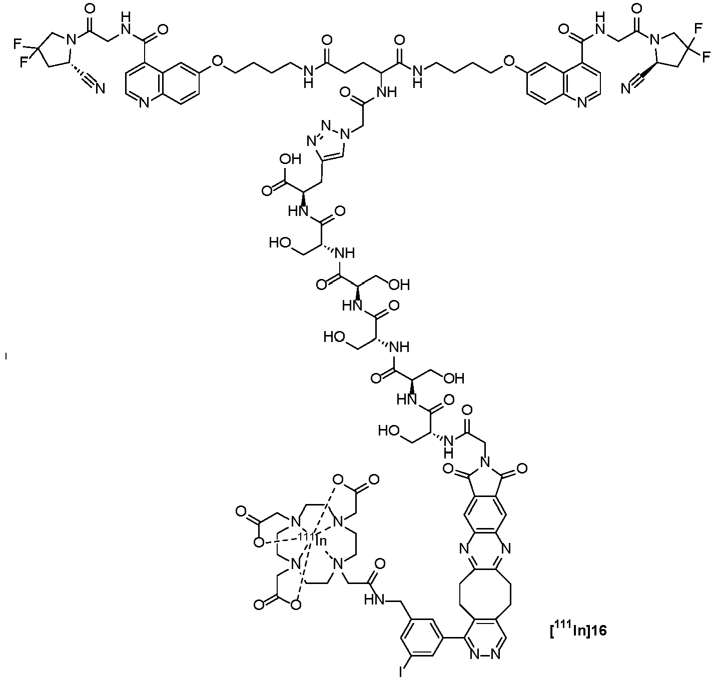

8. A pharmaceutic formulation comprising a FAP inhibitor ligand according to any of claims 1 to 7.

9. FAP inhibitor ligand of formula I: 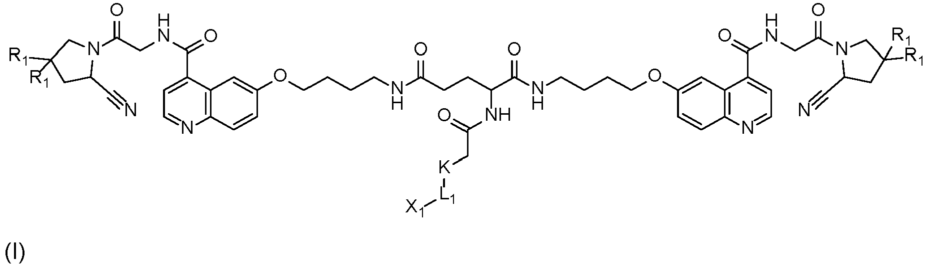 wherein:
R₁ is H or F
K is 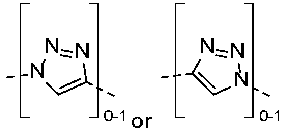
L₁ is selected from:
-(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
wherein n and m are integers independently selected from 0-20,
and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, - COCH(CH₂OH)NH-, -COCH(CH₂CH₂OH)NH-, -COCH(CH(OH)CH₃)NH-, - COCH(CH₂SeH)NH-, -COCH(CH₂C₈H₆N)NH-, -COCH(CH₂C₆H₄OH)NH-; - COCH(CH₂CH₂SeCH₃)NH-,
X₁ is a pyridazine selected from the group consisting of: 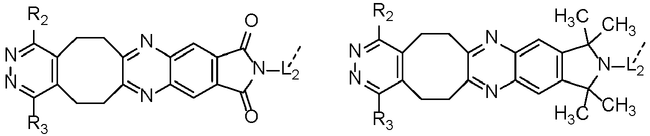 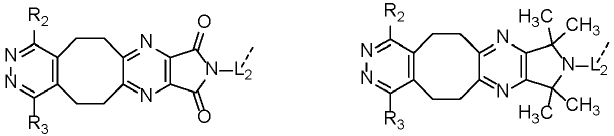 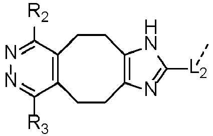
wherein L₂ is -CO(CH₂)ₙ- or -CO(CH₂CH₂O)ₙ(CH₂)ₘ-,
wherein n and m are integers independently selected from 1-10,
R₂ is selected from -H, -Me, and R₃,
R₃ is 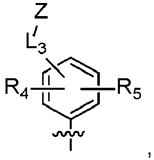 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, - NHCH₂CH₂Y₁, -OCH₂C₆H₄Y₁, -OCH₂CH₂C₆H₄Y₁, -NHCOCH₂CH₂Y₁, -CONHCH₂CH₂Y₁, - NHSO₂CH₂CH₂Y₁, -SO₂NHCH₂CH₂Y₁,
wherein Y₁ is selected from the group constisting of;
¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At,²¹¹At,
and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, - NHSO₂(CH₂)ₙ(OCH₂CH₂)ₘNH-, -CONH(CH₂)ₙ(OCH₂CH₂)ₘNH-, - NHCO(CH₂)ₙ(OCH₂CH₂)ₘNH-, -CH₂(OCH₂CH₂)(CH₂)ₘNH-, -OCH₂(OCH₂CH₂)ₙ(CH₂)ₘNH-, -SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, -NHSO₂CH₂CH₂(OCH₂CH₂)ₙ(CH₂)ₘNHCO-, - CONHCH₂CH₂(OCH₂CH₂)ₙ(CH₂)ₘNHCO-, -NHCOCH₂CH₂(OCH₂CH₂)ₙ(CH₂)ₘNHCO-,
where n and m are independently selected from the group consisting of 0-25;
and wherein Z is a chelator selected from: -H, 1,4,7,10-tetraazacyclododecane-*N,N',N',N"-*tetraacetic acid (DOTA), 2-(4,7,10-tris(2-(tert-butoxy)-2-oxoethyl)-1,4,7,10-tetraazacyclododecan-1-yl)acetic acid (tBu-DOTA), /V,/V'-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N'*-(5-acetyl (hydroxy)aminopentyl-N-(5-(4-(5- aminopentyl)(hydroxy)amino-4-oxobutanoyl)amino)pentyl-*N*-hydroxysuccinamide (DFO), diethylenetriaminepentaacetic acid (DTPA), trans-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
and wherein Z is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
and wherein R₅ is selected from a -H, a halogen, -OH, -OMe, -OEt, -OPr, -NHCOH, - NHCOMe, NHCOEt, -NHCOPr, -CONH₂, -CONHMe, -CONHEt, -CONHPr, NHSOH, - NHSO₂Me, NHSO₂Et, -NHSO₂Pr, -SO₂NH₂, -SO₂NHMe, -SO₂NHEt, -SO₂NHPr, -NH₂, -NHMe, -NHEt, -NHPr;
and wherein said FAP inhibitor ligand of formula (I) comprises at least one radionuclide;
and pharmaceutically acceptable salts thereof, for use as a medicament in therapy, imaging, diagnostics, or theranostics.

10. FAP inhibitor ligand of formula I: 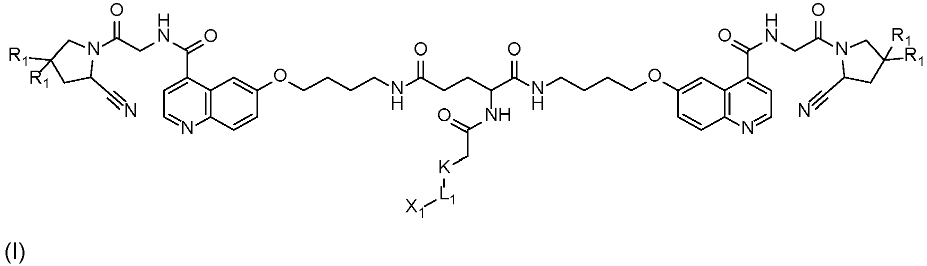 wherein:
R₁ is H or F
K is 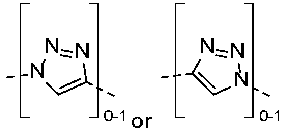
L₁ is selected from:
-(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
wherein n and m are integers independently selected from 0-20,
and A is an amino acid independently selected from the group consisting of: -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, - COCH(CH₂OH)NH-, -COCH(CH₂CH₂OH)NH-, -COCH(CH(OH)CH₃)NH-, - COCH(CH₂SeH)NH-, -COCH(CH₂C₈H₆N)NH-, -COCH(CH₂C₆H₄OH)NH-; - COCH(CH₂CH₂SeCH₃)NH-,
X₁ is a pyridazine selected from the group consisting of: 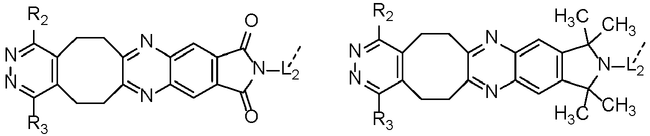 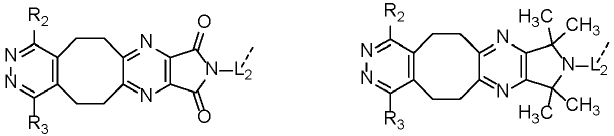 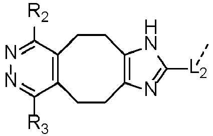
wherein L₂ is -CO(CH₂)ₙ- or -CO(CH₂CH₂O)ₙ(CH₂)ₘ-,
wherein n and m are integers independently selected from 1-10,
and R₂ is selected from -H, -Me, and R₃,
R₃ is 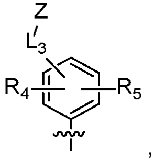 wherein the curly sign indicates the link to the tetrazine; and where R₄ is selected from: -H, -Y₁, -OCH₂CH₂Y₁, -OCH₂CH₂ CH₂Y₁, -SCH₂CH₂Y₁, -NHCH₂CH₂Y₁, - OCH₂C₆H₄Y₁, -OCH₂CH₂C₆H₄Y₁, -NHCOCH₂CH₂Y₁, -CONHCH₂CH₂Y₁, - NHSO₂CH₂CH₂Y₁, or -SO₂NHCH₂CH₂Y₁,
wherein Y₁ is selected from the group constisting of;
¹H, ²H, ³H, ¹¹C, ¹²C, ¹³C, ¹⁴C, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At, ²¹¹At,
and wherein L₃ is selected from: -H, -(CH₂)ₙNH-, -O(CH₂)ₙNH-, -S(CH₂)ₙNH-, - SO₂NH(CH₂)ₙNH-, -NHSO₂(CH₂)ₙNH-, -CONH(CH₂)ₙNH-, -NHCO(CH₂)ₙNH-, - (OCH₂CH₂)ₙ(CH₂)ₘNH-, -(CH₂)ₙ(OCH₂CH₂)ₘNH-, -O(CH₂)ₙ(OCH₂CH₂)ₘNH-, - S(CH₂)ₙ(OCH₂CH₂)ₘNH-, -SO₂NH(CH₂)ₙ(OCH₂CH₂)ₘNH-, - NHSO₂(CH₂)ₙ(OCH₂CH₂)ₘNH-, -CONH(CH₂)ₙ(OCH₂CH₂)ₘNH-, - NHCO(CH₂)ₙ(OCH₂CH₂)ₘNH-, -CH₂(OCH₂CH₂)(CH₂)ₘNH-, -OCH₂(OCH₂CH₂)ₙ(CH₂)ₘNH-, -SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, -NHSO₂CH₂CH₂(OCH₂CH₂)ₙ(CH₂)ₘNHCO-, - CONHCH₂CH₂(OCH₂CH₂)ₙ(CH₂)ₘNHCO-, -NHCOCH₂CH₂(OCH₂CH₂)ₙ(CH₂)ₘNHCO-,
where n and m are independently selected from the group consisting of 0-25;
and wherein Z is a chelator selected from: -H, 1,4,7,10-tetraazacyclododecane-*N,N',N',N"-*tetraacetic acid (DOTA), 2-(4,7,10-tris(2-(tert-butoxy)-2-oxoethyl)-1,4,7,10-tetraazacyclododecan-1-yl)acetic acid (tBu-DOTA), /V,/V'-bis(2-hydroxy-5-(carboxyethyl)benzyl)ethylenediamine *N,N'*-diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N'*-(5-acetyl (hydroxy)aminopentyl-N-(5-(4-(5- aminopentyl)(hydroxy)amino-4-oxobutanoyl)amino)pentyl-*N*-hydroxysuccinamide (DFO), diethylenetriaminepentaacetic acid (DTPA), trans-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA),
and wherein Z is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr,⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁵⁴Gd, ¹⁵⁵Gd, ¹⁵⁶Gd, ¹⁵⁷Gd, ¹⁵⁸Gd, ¹⁶⁰Gd ¹⁵²Tb, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ¹⁶¹Tb, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th;
and wherein R₅ is selected from a -H, a halogen, -OH, -OMe, -OEt, -OPr, -NHCOH, - NHCOMe, NHCOEt, -NHCOPr, -CONH₂, -CONHMe, -CONHEt, -CONHPr, NHSOH, - NHSO₂Me, NHSO₂Et, -NHSO₂Pr, -SO₂NH₂, -SO₂NHMe, -SO₂NHEt, -SO₂NHPr, -NH₂, -NHMe, -NHEt, -NHPr;
and wherein said FAP inhibitor ligand of formula (I) comprises at least one radionuclide;
and pharmaceutically acceptable salts thereof for use in the treatment and/ or in the diagnosis of FAP expressing diseases including cancer, such as breast cancer, lung cancer, colorectal cancer, prostate cancer, ovarian cancer, pancreatic cancer, hepatocellular cancers, sarcomas and non-small cell lung cancer; and fibrosis, arthritis, atherosclerosis, and inflammatory diseases like spondyloarthritis.

11. FAP inhibitor precursor of formula (II): 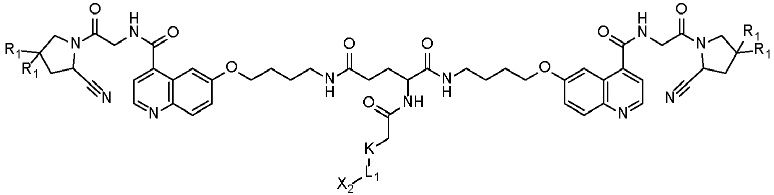 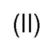 wherein:
R₁ is H or F
K is 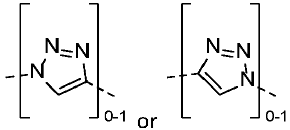
L₁ is selected from:
-(CH₂)ₙNH-, -CH₂(OCH₂CH₂)ₙNH-, -(CH₂)nNHAₘ-, -CH₂(OCH₂CH₂)ₙNHAₘ-, - CH₂C(COOH)NHAₘ-, -CH₂CH₂C(COOH)NHAₘ-
wherein n and m are integers independently selected from 0-20,
and A is an amino acid independently selected from the group consisting of; -COCH₂NH-, -COCH(CH₃)NH-, -COCH(CH₂SH)NH-, -COCH(CH₂COOH)NH-, - COCH(CH₂CH₂COOH)NH-, -COCH(CH₂C₆H₅)NH-, -COCH(CH₂C₃H₃N₂)NH-, - COCH(CH(CH₃)₂)NH-, -COCH((CHCH₃)CH₂CH₃)NH-, -COCH((CH₂)₄NH₂)NH-, - COCH(CH₂CH(CH₃)₂)NH-, -COCH(CH₂CH₂SCH₃)NH-, -CO(CHCH₂CH₂N)-, - COCH(CH₂CONH₂)NH-, -COCH(CH₂CH₂CONH₂)NH-, -COCH((CH₂)₃NH-C(NH)NH₂)NH-, -COCH(CH₂OH)NH-, -COCH(CH₂CH₂OH)NH-, - COCH(CH(OH)CH₃)NH-, -COCH(CH₂SeH)NH-, -COCH(CH₂C₈H₆N)NH-, - COCH(CH₂C₆H₄OH)NH-; -COCH(CH₂CH₂SeCH₃)NH-,
X₂ is a cyclooctene selected from the group consisting of: 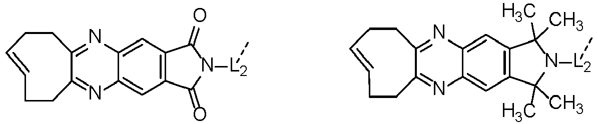 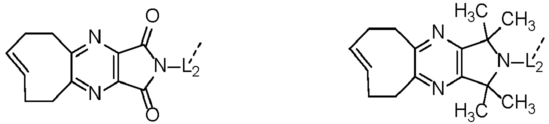 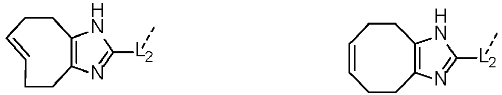 wherein L₂ is -CO(CH₂)ₙ- or -CO(CH₂CH₂O)ₙ(CH₂)ₘ-,
wherein n and m are integers independently selected from 1-10,

12. FAP inhibitor precursor according to claim 11 selected from the group consisting of compounds **LVI-LXIX:**
